# EUROPEAN PATENT APPLICATION

(11) **EP 3 363 801 A1**
(43) Date of publication of application: **22.08.2018**
(21) Application number: 16874865.5
(22) Date of filing: 15.12.2016
(51) Int. Cl.: C07D 487/04, A61K 31/5517, A61P 23/00, A61P 25/20, A61P 25/22, A61P 25/00

(54) **SHORT-ACTING BENZODIAZEPINE DERIVATIVE, PREPARATION METHOD THEREFOR, AND USE THEREOF**

(30) Priority: 17.12.2015 CN 201510952439
(71) Applicant: Sichuan Kelun-Biotech Biopharmaceutical Co., Ltd., Chengdu, Sichuan 611138 (CN)
(72) Inventor: LIU, Gang, Chengdu Sichuan 611138 (CN); YU, Hua, Chengdu Sichuan 611138 (CN); YANG, Dingju, Chengdu Sichuan 611138 (CN); DU, Jing, Chengdu Sichuan 611138 (CN); TANG, Jianchuan, Chengdu Sichuan 611138 (CN); HE, Ting, Chengdu Sichuan 611138 (CN); ZENG, Hong, Chengdu Sichuan 611138 (CN); SONG, Hongmei, Chengdu Sichuan 611138 (CN); SU, Donghai, Chengdu Sichuan 611138 (CN); LIU, Wei, Chengdu Sichuan 611138 (CN); WANG, Lichun, Chengdu Sichuan 611138 (CN); WANG, Jingyi, Chengdu Sichuan 611138 (CN)
(74) Representative: Turner, Craig Robert
(86) International application number: PCT/CN2016/110075
(87) International publication number: WO 2017/101808

(57) **Abstract**

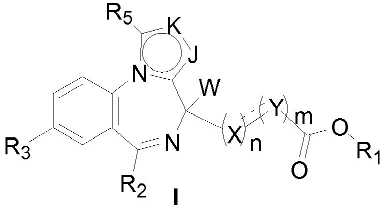

The present invention relates to a benzodiazepine derivative of Formula I as a short-acting anesthetic, a pharmaceutical composition comprising the same, a kit comprising the same, a preparation method thereof, an method of anesthesia using the same and use thereof in the manufacture of an anesthetic medicament.

## Description

### FIELD OF THE INVENTION

The present invention relates to a benzodiazepine derivative as a short-acting anesthetic, a pharmaceutical composition comprising the same, a kit comprising the same, a preparation method thereof, an method of anesthesia using the same and use thereof in the manufacture of an anesthetic medicament.

### BACKGROUND OF THE INVENTION

Benzodiazepine derivatives (benzodiazepines) are GABA_{A} receptor (also known as γ-aminobutyric acid type A receptor) activators. GABA_{A} receptor is a chloride ion channel-gating receptor, and the chloride ion channel consists of two α-subunits and two β-subunits (α2β2). There is a GABA receptor site on the β-subunit. When GABA is bound to the GABA receptor site, the chloride ion channel opens, allowing the inflow of chloride ions, thus leading to the hyperpolarization of nerve cells, so as to produce a suppression effect. On the α subunit, there is benzodiazepine receptor. When benzodiazepine is bound to the benzodiazepine receptor, it is possible to increase the opening frequency of the chloride ion channel through facilitating the binding of GABA with the GABA_{A} receptor (instead of extending the opening duration of the chlorine ion channel or increasing the chlorine ion flow), so that more chlorine ions enter the cell. Thus, benzodiazepine derivatives are capable of enhancing GABA neurotransmission and synaptic function suppressive effect, resulting in anxiolytic, sedative, hypnotic, anticonvulsant, muscle relaxant, and calming effects in clinic.

There are more than 20 benzodiazepine derivatives commonly used in clinic. Although they are similar in structure, they are different in clinical indications. Midazolam was introduced to the market in the early 1980s as the first water-soluble benzodiazepine compound, and was used as an intravenous injection to provide sedation and anesthesia for short-term surgery or intensive care unit. However, midazolam will produce an active metabolite *in vivo,* leading to a long conscious recovery period for patients from the midazolam-induced state of sedation. In addition, as the metabolism of midazolam depends on hepatic cytochrome P4503A4, potential drug-drug interaction problems may arise if midazolam is administered to a patient with impaired liver function.

Therefore, it is desired to develop short-acting central nervous system (CNS) inhibitors with a shorter onset time, a shorter duration of action, and a shorter recovery time. Such inhibitors are expected to have lower incidences of adverse cardiovascular suppressive effects, and can be used for intravenous administration in the following clinical settings: presurgical sedation, antianxiety and anti-amnesia during surgery; conscious sedation during short-term diagnostic, surgical or endoscopic procedures; induction and maintenance of general anesthesia prior to and/or at the same time of administration of other anesthetics and analgesics; ICU sedation; etc.

### SUMMARY OF THE INVENTION

In one aspect, the present invention provides a compound of Formula I, 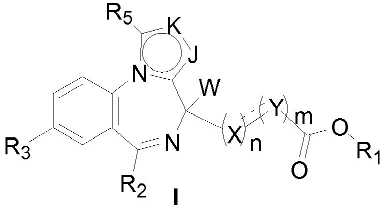 wherein,
R₁ is selected from the group consisting of hydrogen, C₁₋₆ alkyl, C₂₋₆ alkenyl, C₂₋₆ alkynyl, 3-10 membered cycloalkyl, 3-10 membered heterocyclyl, 6-14 membered aryl and 5-14 membered heteroaryl, wherein the C₁₋₆ alkyl, C₂₋₆ alkenyl, C₂₋₆ alkynyl, 3-10 membered cycloalkyl, 3-10 membered heterocyclyl, 6-14 membered aryl and 5-14 membered heteroaryl are each optionally substituted with one or more (e.g., 1, 2, 3 or 4) substituents independently selected from the group consisting of halogen, hydroxy, R₆R₇N-, cyano, nitro, C₁₋₆ alkyl, C₁₋₆ alkoxy, RsS-, RsS(O)-, R₈S(O)₂-, 3-10 membered cycloalkyl, 3-10 membered heterocyclyl, 6-14 membered aryl and 5-14 membered heteroaryl;
R₂ is selected from the group consisting of 3-10 membered cycloalkyl, 3-10 membered heterocyclyl, 6-14 membered aryl and 5-14 membered heteroaryl, wherein the 3-10 membered cycloalkyl, 3-10 membered heterocyclyl, 6-14 membered aryl and 5-14 membered heteroaryl are each optionally substituted with one or more (e.g., 1, 2, 3 or 4) substituents independently selected from the group consisting of halogen, hydroxy, cyano, nitro, C₁₋₆ alkyl, C₂₋₆ alkenyl, C₂₋₆ alkynyl, RsC(O)-, RsS-, RsS(O)-, R₈S(O)₂-, R₆R₇N-, C₁₋₆ alkoxy, 3-10 membered cycloalkyl, 3-10 membered heterocyclyl, 6-14 membered aryl and 5-14 membered heteroaryl;
R₃ is selected from the group consisting of hydrogen, halogen, cyano, nitro, C₁₋₆ alkyl, C₂₋₆ alkenyl and C₂₋₆ alkynyl, wherein the C₁₋₆ alkyl, C₂₋₆ alkenyl, and C₂₋₆ alkynyl are each optionally substituted with one or more (e.g., 1, 2, 3 or 4) substituents independently selected from halogen;
K, J are each independently selected from the group consisting of N and CR₄;
R₄ at each occurance is independently selected from the group consisting of hydrogen, halogen, hydroxy, R₆R₇N-, cyano, carboxy, nitro, C₁₋₆ alkyl, C₁₋₆ alkoxy, C₂₋₆ alkenyl, C₂₋₆ alkynyl, 3-10 membered cycloalkyl, 3-10 membered heterocyclyl, 6-14 membered aryl and 5-14 membered heteroaryl, wherein the C₁₋₆ alkyl, C₁₋₆ alkoxy, C₂₋₆ alkenyl, C₂₋₆ alkynyl, 3-10 membered cycloalkyl, 3-10 membered heterocyclyl, 6-14 membered aryl and 5-14 membered heteroaryl are each optionally substituted with one or more (e.g., 1, 2, 3 or 4) substituents independently selected from the group consisting of halogen, hydroxy, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl;
R₅ is independently selected from the group consisting of hydrogen, halogen, hydroxy, R₆R₇N-, cyano, C₁₋₆ alkyl, C₁₋₆ alkoxy, C₂₋₆ alkenyl, C₂₋₆ alkynyl, 3-10 membered cycloalkyl, 3-10 membered heterocyclyl, 6-14 membered aryl, 5-14 membered heteroaryl, RsC(O)-, RsS-, R₈S(O)- and R₈S(O)₂-, wherein the C₁₋₆ alkyl, C₁₋₆ alkoxy, C₂₋₆ alkenyl, C₂₋₆ alkynyl, 3-10 membered cycloalkyl, 3-10 membered heterocyclyl, 6-14 membered aryl, 5-14 membered heteroaryl, RsC(O)-, RsS-, R₈S(O)- and R₈S(O)₂- are each optionally substituted with one or more (e.g., 1, 2, 3 or 4) substituents independently selected from the group consisting of deuterium, halogen, hydroxy, cyano, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl;
R₆, R₇ at each occurance are each independently selected from the group consisting of hydrogen, C₁₋₆ alkyl, C₂₋₆ alkenyl and C₂₋₆ alkynyl;
R₈ at each occurance is independently selected from the group consisting of hydroxy, R₆R₇N-, C₁₋₆ alkyl, C₂₋₆ alkenyl, C₂₋₆ alkynyl, 3-10 membered cycloalkyl, 3-10 membered heterocyclyl, 6-14 membered aryl and 5-14 membered heteroaryl;
X, Y are each independently -(R₉R₁₀)C-, and the bond between X and Y is a single bond, a double bond or a triple bond;
one or both of R₉ and R₁₀ are absent, or R₉ and R₁₀ at each occurrence are independently selected from the group consisting of hydrogen, halogen, hydroxy, R₆R₇N-, cyano, carboxy, nitro, C₁₋₆ alkoxy, C₁₋₆ alkyl, C₂₋₆ alkenyl, C₂₋₆ alkynyl, 3-10 membered cycloalkyl, 3-10 membered heterocyclyl, 6-14 membered aryl and 5-14 membered heteroaryl, wherein the C₁₋₆ alkoxy, C₁₋₆ alkyl, C₂₋₆ alkenyl, C₂₋₆ alkynyl, 3-10 membered cycloalkyl, 3-10 membered heterocyclyl, 6-14 membered aryl and 5-14 membered heteroaryl are each optionally substituted with one or more (e.g., 1, 2, 3 or 4) substituents independently selected from the group consisting of halogen, hydroxy, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl;
m, n are each independently selected from the group consisting of 0, 1 and 2, and m + n ≥ 1; and
W is selected from the group consisting of hydrogen, halogen, hydroxy, cyano, C₁₋₆ alkoxy, C₁₋₆ alkyl, C₂₋₆ alkenyl and C₂₋₆ alkynyl, wherein the C₁₋₆ alkoxy, C₁₋₆ alkyl, C₂₋₆ alkenyl and C₂₋₆ alkynyl are each optionally substituted with one or more (e.g., 1, 2, 3 or 4) substituents independently selected from the group consisting of halogen, hydroxy, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl group;
wherein the above-mentioned hydrogen and the hydrogen contained in the above-mentioned substituents are independently selected from the group consisting of protium, deuterium and tritium,
or a pharmaceutically acceptable salt, stereoisomer, tautomer, polymorph, solvate, metabolite or prodrug thereof.

In another aspect, the present invention provides a pharmaceutical composition comprising an effective amount of the compound of the present invention and one or more pharmaceutically acceptable carriers.

In another aspect, the present invention provides a kit comprising the compound of the present invention or the pharmaceutical composition of the present invention.

In another aspect, the present invention provides a method for preparing the compound of the present invention, wherein
when J is N, the method is carried out according to Scheme I, 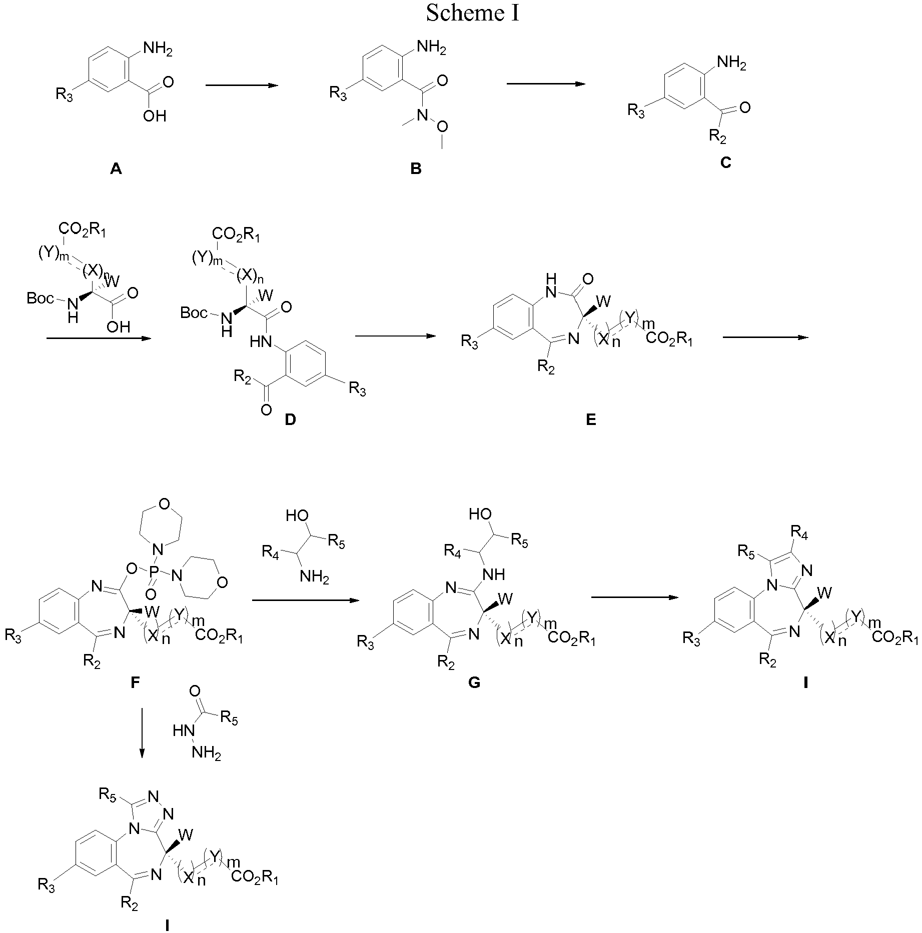 wherein R₁-R₅, n, m, W are as defined above; and the bond between X and Y is a single bond or a double bond;
or
when K is CR₄ and J is N, the method is carried out according to Scheme II: 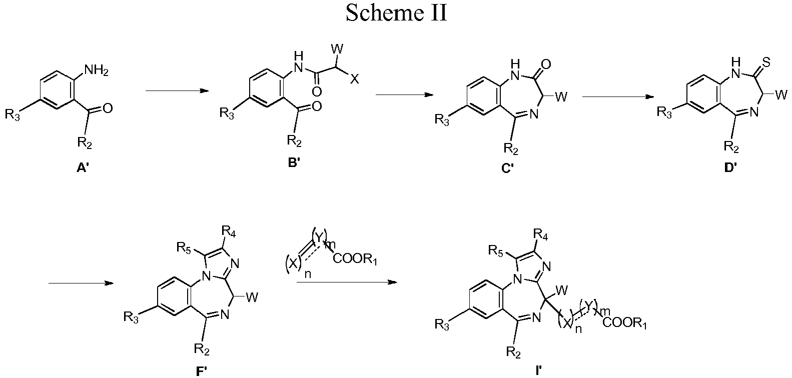 wherein R₁-R₄, W are as defined above; the bond between X and Y is a single bond or a double bond; m and n are each 1; and R₅ is C₁₋₆ alkyl optionally substituted with one or more (e.g., 1, 2, 3 or 4) substituents independently selected from the group consisting of deuterium, halogen, hydroxy, cyano, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl, preferably, R₅ is selected from the group consisting of methyl, ethyl, isopropyl, trifluoromethyl, difluoromethyl, hydroxymethyl, methoxymethyl, N,N-dimethylaminomethyl, N,N-dimethylaminoethyl, N,N-diethylaminomethyl, and deuteromethyl;
or
when K is N and J is CR₄, the method is carried out according to Scheme III: 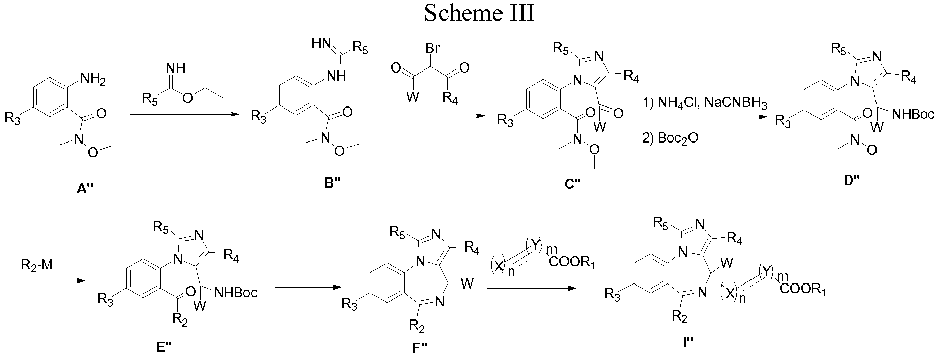 wherein R₁-R₅, W are as defined as above; the bond between X and Y is a single bond or a double bond; and m and n are each 1.

In another aspect, the present invention provides a method of anesthesia, comprising administering an effective amount of the compound or the pharmaceutical composition of the present invention.

In another aspect, the present invention provides the compound of the present invention or the pharmaceutical composition of the present invention, for use as an anesthetic medicament.

In another aspect, the present invention provides use of the compound of the present invention or the pharmaceutical composition of the present invention in the manufacture of an anesthetic medicament.

As compared with the prior art, the benzodiazepine compounds of the present invention not only maintain high affinity and selectivity to the GABA_{A} receptor, but also have the following advantages through structural modification on the benzodiazepine skeleton and the carboxylate ester group: predictable and fast onset time for achieving sedation, short duration of effective action, short recovery time, reduced adverse suppressive effects on the cardiovascular and respiratory systems, and reduced side effects on the patient's nervous system, including lethargy, dizziness and other problems.

### DETAILED DESCRIPTION OF THE INVENTION

### Definition

Unless otherwise defined below, all technical and scientific terms used herein are intended to have the same meanings as those commonly understood by a person skilled in the art. References to techniques employed herein are intended to refer to the techniques as commonly understood in the art, including variations on those techniques or substitutions of equivalent techniques which would be apparent to a person skilled in the art. While it is believed that most of the following terms will be readily understood by a person skilled in the art, the following definitions are put forth to better illustrate the present invention.

The terms "include", "comprise", "have", "contain", or "involve", as well as other variations used herein are inclusive or open-ended, and do not exclude additional, unrecited elements or method steps.

As used herein, the term "alkyl" refers to a linear or branched, saturated aliphatic hydrocarbon residue. In some embodiments, an alkyl group has 1 to 6, e.g., 1 to 4 carbon atoms. For example, as used herein, the term "C₁₋₆ alkyl" refers to a linear or branched alkyl group containing 1 to 6 carbon atoms, such as methyl, ethyl, n-propyl, isopropyl, n-butyl, isobutyl, *sec*-butyl, *tert*-butyl, *n*-pentyl or *n*-hexyl, optionally substituted with one or more (e.g., 1, 2 or 3) suitable substituents, such as CF₃, CH₂OH, CD₃, etc.

As used herein, the term "alkenyl" refers to a linear or branched, unsaturated aliphatic hydrocarbon residue containing at least one carbon-carbon double bond, and an alkenyl may contain 2 to 10, e.g., 2 to 6 carbon atoms. For example, as used herein, the term "C₂₋₆ alkenyl" refers to a linear or branched alkenyl group containing 2 to 6 carbon atoms, such as ethenyl, 1-propenyl, 2-propenyl (allyl), isopropenyl, 2-methyl-1-propenyl, 1-butenyl or 2-butenyl, optionally substituted with one or more (e.g., 1, 2, 3 or 4) suitable substituents.

As used herein, the term "alkynyl" refers to a linear or branched, unsaturated aliphatic hydrocarbon residue containing at least one carbon-carbon triple bond, and an alkynyl may contain 2 to 10, e.g., 2 to 6 carbon atoms. For example, as used herein, the term "C₂₋₆ alkynyl" refers to a linear or branched alkynyl group containing 2 to 6 carbon atoms, such as ethynyl, 1-propynyl, 2-propynyl, 1-butynyl or 2-butynyl, optionally substituted with one or more (e.g., 1, 2, 3 or 4) suitable substituents.

As used herein, the term "alkoxy" refers to a linear, branched or cyclic, saturated monovalent hydrocarbon residue represented by a formula of -O-alkyl, wherein the term "alkyl" is as defined above or refers to a "cycloalkyl" as defined below, such as methoxy, ethoxy, n-propoxy, isopropoxy, cyclopropyloxy, n-butoxy, isobutoxy, tert-butoxy, *sec*-butoxy, cyclobutoxy, pentoxy, *iso*-pentoxy or n-hexyloxy group, or isomers thereof.

As used herein, the term "cycloalkyl" refers to a saturated or unsaturated, non-aromatic, monocyclic or polycyclic (such as bicyclic) hydrocarbon ring (e.g. a monocyclic hydrocarbon ring, such as cyclopropyl, cyclobutyl, cyclopentyl, cyclohexyl, cycloheptyl, cyclooctyl, or cyclononyl; or a bicyclic hydrocarbon ring, including spiro, fused or bridged ring systems (such as bicyclo[1.1.1]pentyl, bicyclo[2.2.1]heptyl, bicyclo[3.2.1]octyl, bicyclo[5.2.0]nonyl, decahydronaphthyl, etc.), optionally substituted with one or more (e.g., 1, 2, 3 or 4) suitable substituents. A cycloalkyl group has 3 to 15, e.g., 3 to 10 carbon atoms. For example, the term "3-10 membered cycloalkyl" refers to a saturated or unsaturated, non-aromatic, monocyclic or polycyclic (such as bicyclic) hydrocarbon ring containing 3 to 10 ring-forming carbon atoms, (e.g., cyclopropyl, cyclobutyl, cyclopentyl, cyclohexyl or bicyclo[1.1.1]pentyl), optionally substituted with one or more (e.g., 1, 2, 3 or 4) suitable substituents, such as methyl-substituted cyclopropyl.

As used herein, the term "heterocyclyl" refers to a saturated or unsaturated, monovalent, monocyclic or bicyclic residue having 2, 3, 4, 5, 6, 7, 8 or 9 carbon atoms and one or more (e.g., 1, 2, 3 or 4) heteroatom-containing groups selected from the group consisting of C(=O), O, S, S(=O), S(=O)₂ and NRₐ wherein Rₐ represents a hydrogen atom, C₁₋₆ alkyl, or C₁₋₆ haloalkyl group, in the ring. A heterocycloalkyl may be linked to the rest of a molecule through any one of the carbon atoms or a nitrogen atom (if present). In particular, a 3-10 membered heterocyclyl refers to a group having 3 to 10 carbon atoms and one or more (e.g., 1, 2, 3, or 4) heteroatom-containing groups as mentioned above in the ring, including, but are not limited to, oxiranyl, aziridinyl, azetidinyl, oxetanyl, tetrahydrofuranyl, dioxolinyl, pyrrolidinyl, pyrrolidinonyl, imidazolidinyl, pyrazolidinyl, pyrrolinyl, tetrahydropyranyl, piperidinyl, morpholinyl, dithianyl, thiomorpholinyl, piperazinyl or trithianyl.

As used herein, the term "aryl" refers to an all-carbon, monocyclic or fused-ring polycyclic, aromatic group having a conjugated π-electron system. For example, as used herein, the term "6-14 membered aryl" refers to an aromatic group containing 6 to 14 carbon atoms, such as phenyl or naphthyl. An aryl is optionally substituted with one or more (for example 1, 2, 3 or 4) suitable substituents.

As used herein, the term "heteroaryl" refers to a monovalent, mono-, bi- or tri-cyclic, aromatic ring system having 5, 6, 8, 9, 10, 11, 12, 13 or 14 ring atoms, in particular including 1 or 2 or 3 or 4 or 5 or 6 or 9 or 10 carbon atoms, and at least one heteroatoms (e.g., oxygen, nitrogen or sulfur) that can be the same or different, and, in each case it may be benzo-fused. In particular, a heteroaryl is selected from the group consisting of thienyl, furyl, pyrrolyl, oxazolyl, thiazolyl, imidazolyl, pyrazolyl, isoxazolyl, isothiazolyl, oxadiazolyl, triazolyl, thiadiazolyl, and benzo derivatives thereof; or pyridyl, pyridazinyl, pyrimidinyl, pyrazinyl, triazinyl, and benzo derivatives thereof.

As used herein, the term "halo" or "halogen" refers to F, Cl, Br or I.

The term "substituted" means that one or more (e.g., 1, 2, 3 or 4) hydrogen atoms on the designated atom are replaced with specified groups, provided that the designated atom's normal valency under the existing circumstances is not exceeded, and that the substitution results in a stable compound. Combinations of substituents and/or variables are permissible only if such combinations result in stable compounds.

The term "optionally substituted" means that a compound is unsbstituted or substituted with a specified group, radical or moiety.

When a bond of a substituent goes through the bond linking two ring-forming atoms, such a substituent may be linked to any of the ring-forming atoms that can be substituted.

The compound of the present invention may also contain one or more (e.g., 1, 2, 3, or 4) isotopes. For example, in the compound of the present invention, hydrogen or H may be in any isotopic form, including ¹H, ²H (D or deuterium) and ³H (T or tritium); carbon or C may be in any isotopic form, including ¹²C, ¹³C and ¹⁴C; and oxygen or O may be in any isotopic form, including ¹⁶O and ¹⁸O.

The term "stereoisomer" refers to isomers formed due to the presence of at least one asymmetric center. For a compound having one or more (e.g., 1, 2, 3 or 4) asymmetric centers, racemates, racemic mixtures, single enantiomers, diastereomeric mixtures and individual diastereomers can be formed. Certain molecules may also have geometric isomers (cis/trans). Similarly, the compound of the present invention may exist as a mixture of two or more forms having different structures in rapid equilibrium (often referred to as tautomers). Representative examples of tautomers include ketone-enol tautomers, phenol-ketone tautomers, nitroso-oxime tautomers, imine-enamine tautomers, etc. It is understood that the present application cover all isomers in any percentage (e.g., 60%, 65%, 70%, 75%, 80%, 85%, 90%, 95%, 96%, 97%, 98%, 99%) or mixtures thereof.

The term "tautomer" refers to structural isomers of different energies that are interconversable due to a low energy barrier. For example, proton tautomers (also known as proton transfer tautomers) are interconversable through proton migration, such as keto-enol tautomers and imine-enamine tautomers. A specific example of proton tautomers is imidazole in which protons can migrate between the two ring-forming nitrogen groups. Valence tautomers are interconversable through rearrangement of bonding electrons.

As used herein, the term "effective amount" refers to an amount of compound that can induce an anesthetic effect to some extent after administration.

The present invention encompasses all the possible crystalline forms or polymorphs of the compound of the present invention, and the compound of the present invention may be in the form of a single polymorph or a mixture of more than one polymorph in any ratio.

It should also be understood that certain compounds of the present invention can be used in the free form thereof, or can be present as a pharmaceutically acceptable derivative as appropriate. In accordance with the present invention, pharmaceutically acceptable derivatives include, but not limited to, pharmaceutically acceptable salts, solvates, metabolites or prodrugs. After these derivatives are administered to a patient in need, the compound of the present invention or a metabolite or residue thereof will be released directly or indirectly.

A pharmaceutically acceptable salt of the compound of the present invention includes an acid addition salt and a base addition salt.

A suitable acid addition salt is formed from an acid which forms a non-toxic salt. Examples of acid addition salts include acetate, adipate, aspartate, benzoate, bicarbonate/carbonate, bisulfate/sulfate, borate, camsylate, citrate, cyclamate, edisylate, ethanesulfonate, formate, fumarate, gluceptate, gluconate, glucuronate, hexafluorophosphate, hydrobromide/bromide, hydroiodide/iodide, isethionate, lactate, malate, maleate, mesylate, methylsulfate, naphthylate, 2-napsylate, nicotinate, nitrate, orotate, palmitate, pamoate, phosphate/hydrogen phosphate/dihydrogen phosphate, pyroglutamate, saccharate, stearate, succinate, tannate, tartrate, trifluoroacetate and xinafoate salts.

A suitable base addition salt is formed from a base which forms a non-toxic salt. Examples of base addition salts include aluminum, arginine, benzathine, calcium, choline, diethylamine, diolamine, glycine, lysine, magnesium, meglumine, olamine, potassium, sodium, tromethamine and zinc salts.

For a review on suitable salts, see "Handbook of Pharmaceutical Salts: Properties, Selection, and Use" by Stahl and Wermuth (Wiley-VCH, 2002). Methods for preparing pharmaceutically acceptable salts of the compound of the present invention are known to a person skilled in the art.

The compound of the present invention may exist in the form of a hydrate or a solvate, wherein the compound of the present invention contains a polar solvent, in particular water, methanol or ethanol, for example, as a structural element of the crystal lattice of the compound. Polar solvents, in particular water, may exist in a stoichiometric or non-stoichiometric amount.

The present invention further encompasses a metabolite of the compound of the present invention, i.e., a compound generated *in vivo* upon administration of a drug.

Prodrugs of the compound of the present invention can be formed through replacing suitable functional groups in the compound of Formula I with those known in the art (for example, the "pro-moieties" described in "Design of Prodrugs" by H. Bundgaard (Elsevier, 1985)).

### Compounds

One object of the present invention is to provide novel benzodiazepine compounds, which have the following advantages as compared with the prior arts: predictable and fast onset time of sedation, short duration of effective action, short recovery time, lower incidences of adverse cardiovascular and respiratory suppressive effects, and reduced side effects on the patient's nervous system, including lethargy, dizziness and other problems.

In particular, the present invention relates to a compound of Formula I, 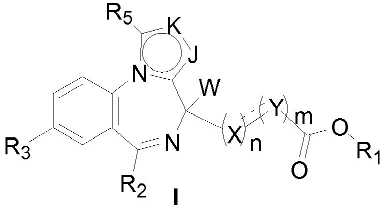 wherein,
R₁ is selected from the group consisting of hydrogen, C₁₋₆ alkyl, C₂₋₆ alkenyl, C₂₋₆ alkynyl, 3-10 membered cycloalkyl, 3-10 membered heterocyclyl, 6-14 membered aryl and 5-14 membered heteroaryl, wherein the C₁₋₆ alkyl, C₂₋₆ alkenyl, C₂₋₆ alkynyl, 3-10 membered cycloalkyl, 3-10 membered heterocyclyl, 6-14 membered aryl and 5-14 membered heteroaryl are each optionally substituted with one or more (e.g., 1, 2, 3 or 4) substituents independently selected from the group consisting of halogen, hydroxy, R₆R₇N-, cyano, nitro, C₁₋₆ alkyl, C₁₋₆ alkoxy, RsS-, RsS(O)-, R₈S(O)₂-, 3-10 membered cycloalkyl, 3-10 membered heterocyclyl, 6-14 membered aryl and 5-14 membered heteroaryl;
R₂ is selected from the group consisting of 3-10 membered cycloalkyl, 3-10 membered heterocyclyl, 6-14 membered aryl and 5-14 membered heteroaryl, wherein the 3-10 membered cycloalkyl, 3-10 membered heterocyclyl, 6-14 membered aryl and 5-14 membered heteroaryl are each optionally substituted with one or more (e.g., 1, 2, 3 or 4) substituents independently selected from the group consisting of halogen, hydroxy, cyano, nitro, C₁₋₆ alkyl, C₂₋₆ alkenyl, C₂₋₆ alkynyl, R₈C(O)-, R₈S-, R₈S(O)-, R₈S(O)₂-, R₆R₇N-, C₁₋₆ alkoxy, 3-10 membered cycloalkyl, 3-10 membered heterocyclyl, 6-14 membered aryl and 5-14 membered heteroaryl;
R₃ is selected from the group consisting of hydrogen, halogen, cyano, nitro, C₁₋₆ alkyl, C₂₋₆ alkenyl and C₂₋₆ alkynyl, wherein the C₁₋₆ alkyl, C₂₋₆ alkenyl, and C₂₋₆ alkynyl are each optionally substituted with one or more (e.g., 1, 2, 3 or 4) substituents independently selected from halogen;
K, J are each independently selected from the group consisting of N and CR₄;
R₄ at each occurance is independently selected from the group consisting of hydrogen, halogen, hydroxy, R₆R₇N-, cyano, carboxy, nitro, C₁₋₆ alkyl, C₁₋₆ alkoxy, C₂₋₆ alkenyl, C₂₋₆ alkynyl, 3-10 membered cycloalkyl, 3-10 membered heterocyclyl, 6-14 membered aryl and 5-14 membered heteroaryl, wherein the C₁₋₆ alkyl, C₁₋₆ alkoxy, C₂₋₆ alkenyl, C₂₋₆ alkynyl, 3-10 membered cycloalkyl, 3-10 membered heterocyclyl, 6-14 membered aryl and 5-14 membered heteroaryl are each optionally substituted with one or more (e.g., 1, 2, 3 or 4) substituents independently selected from the group consisting of halogen, hydroxy, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl;
R₅ is independently selected from the group consisting of hydrogen, halogen, hydroxy, R₆R₇N-, cyano, C₁₋₆ alkyl, C₁₋₆ alkoxy, C₂₋₆ alkenyl, C₂₋₆ alkynyl, 3-10 membered cycloalkyl, 3-10 membered heterocyclyl, 6-14 membered aryl, 5-14 membered heteroaryl, RsC(O)-, RsS-, R₈S(O)- and R₈S(O)₂-, wherein the C₁₋₆ alkyl, C₁₋₆ alkoxy, C₂₋₆ alkenyl, C₂₋₆ alkynyl, 3-10 membered cycloalkyl, 3-10 membered heterocyclyl, 6-14 membered aryl, 5-14 membered heteroaryl, RsC(O)-, RsS-, R₈S(O)- and R₈S(O)₂- are each optionally substituted with one or more (e.g., 1, 2, 3 or 4) substituents independently selected from the group consisting of deuterium, halogen, hydroxy, cyano, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl;
R₆, R₇ at each occurance are each independently selected from the group consisting of hydrogen, C₁₋₆ alkyl, C₂₋₆ alkenyl and C₂₋₆ alkynyl;
R₈ at each occurance is independently selected from the group consisting of hydroxy, R₆R₇N-, C₁₋₆ alkyl, C₂₋₆ alkenyl, C₂₋₆ alkynyl, 3-10 membered cycloalkyl, 3-10 membered heterocyclyl, 6-14 membered aryl and 5-14 membered heteroaryl;
X, Y are each independently -(R₉R₁₀)C-, and the bond between X and Y is a single bond, a double bond or a triple bond;
one or both of R₉ and R₁₀ are absent, or R₉ and R₁₀ at each occurrence are independently selected from the group consisting of hydrogen, halogen, hydroxy, R₆R₇N-, cyano, carboxy, nitro, C₁₋₆ alkoxy, C₁₋₆ alkyl, C₂₋₆ alkenyl, C₂₋₆ alkynyl, 3-10 membered cycloalkyl, 3-10 membered heterocyclyl, 6-14 membered aryl and 5-14 membered heteroaryl, wherein the C₁₋₆ alkoxy, C₁₋₆ alkyl, C₂₋₆ alkenyl, C₂₋₆ alkynyl, 3-10 membered cycloalkyl, 3-10 membered heterocyclyl, 6-14 membered aryl and 5-14 membered heteroaryl are each optionally substituted with one or more (e.g., 1, 2, 3 or 4) substituents independently selected from the group consisting of halogen, hydroxy, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl;
m, n are each independently selected from the group consisting of 0, 1 and 2, and m + n ≥ 1; and
W is selected from the group consisting of hydrogen, halogen, hydroxy, cyano, C₁₋₆ alkoxy, C₁₋₆ alkyl, C₂₋₆ alkenyl and C₂₋₆ alkynyl, wherein the C₁₋₆ alkoxy, C₁₋₆ alkyl, C₂₋₆ alkenyl and C₂₋₆ alkynyl are each optionally substituted with one or more (e.g., 1, 2, 3 or 4) substituents independently selected from the group consisting of halogen, hydroxy, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl group;
wherein the above-mentioned hydrogen and the hydrogen contained in the above-mentioned substituents are independently selected from the group consisting of protium, deuterium and tritium,
or a pharmaceutically acceptable salt, stereoisomer, tautomer, polymorph, solvate, metabolite or prodrug thereof.

According to an embodiment of the present invention, R₁ is C₁₋₆ alkyl, e.g., methyl or ethyl.

According to an embodiment of the present invention, R₂ is selected from the group consisting of 6-14 membered aryl and 5-14 membered heteroaryl optionally substituted with one or more (e.g., 1, 2, 3 or 4) halogen, e.g., pyridyl and phenyl optionally substituted with one or more (e.g., 1, 2, 3 or 4) halogen. Preferably, R₂ is selected from the group consisting of pyridyl and phenyl optionally substituted with one or more (e.g., 1, 2, 3 or 4) fluorines, e.g., 2-pyridyl, 2-fluorophenyl and phenyl.

According to an embodiment of the present invention, R₃ is halogen, such as chlorine or bromine.

According to an embodiment of the present invention, K, J are not CR₄ at the same time.

According to an embodiment of the present invention, K, J are each independently selected from the group consisting of N, CH, C-CH₃, C-CH₂N(CH₃)₂, and C-CH₂N(CH₂CH₃)₂.

According to an embodiment of the present invention, R₄ at each occurrence is independently selected from the group consisting of hydrogen and C₁₋₆ alkyl optionally substituted with R₆R₇N-. For example, R₄ at each occurrence is independently selected from the group consisting of hydrogen, methyl, N,N-dimethylaminomethyl and N,N-diethylaminomethyl.

According to an embodiment of the present invention, R₅ is independently selected from the group consisting of hydrogen, C₁₋₆ alkyl, C₁₋₆ alkoxy, C₂₋₆ alkenyl, C₂₋₆ alkynyl, 3-10 membered cycloalkyl, and 3-10 membered heterocyclyl, wherein the C₁₋₆ alkyl, C₁₋₆ alkoxy, C₂₋₆ alkenyl, C₂₋₆ alkynyl, 3-10 membered cycloalkyl, or 3-10 membered heterocyclyl is each optionally substituted with one or more (e.g., 1, 2, 3 or 4) substituents independently selected from the group consisting of deuterium, halogen, hydroxy, C₁₋₆ alkyl, R₆R₇N-, and C₁₋₆ alkoxy. Preferably, R₅ is independently selected from the group consisting of hydrogen, methyl, ethyl, isopropyl, cyclopropyl, methylcyclopropyl, cyclobutyl, oxetanyl, trifluoromethyl, difluoromethyl, hydroxymethyl, methoxymethyl, N,N-dimethylaminomethyl, N,N-dimethylaminoethyl, N,N-diethylaminomethyl, deuteromethyl, ethenyl, ethynyl and methoxy.

According to an embodiment of the present invention, R₆, R₇ at each occurance are each independently selected from the group consisting of hydrogen and C₁₋₆ alkyl. For example, R₆, R₇ at each occurance are each independently selected from the group consisting of hydrogen, methyl and ethyl.

According to an embodiment of the present invention, one of R₉ and R₁₀ is absent, or R₉ and R₁₀ at each occurrence are independently selected from the group consisting of hydrogen and C₁₋₆ alkyl. For example, one of R₉ and R₁₀ is absent, or R₉ and R₁₀ at each occurrence are independently selected from the group consisting of hydrogen and methyl.

According to an embodiment of the present invention, the bond between X and Y is a single bond or a double bond.

According to an embodiment of the present invention, when the bond between X and Y is a single bond, X, Y are each independently selected from the group consisting of CH2 and CH₃CH; when the bond between X and Y is a double bond, X, Y are each independently selected from the group consisting of CH and C-CH₃.

According to an embodiment of the present invention, W is hydrogen.

According to an embodiment of the present invention, when K is N and J is CR₄, R₂ is phenyl or pyridyl, preferably phenyl or 2-pyridyl.

According to an embodiment of the present invention, when K is N and J is N, R₂ is phenyl or pyridyl, preferably phenyl or 2-pyridyl; or
According to an embodiment of the present invention, when K is N and J is N, R₂ is phenyl optionally substituted with one or more (e.g., 1, 2, 3 or 4) halogen, such as 2-fluorophenyl; and R₅ is independently selected from the group consisting of hydrogen, 3-10 membered cycloalkyl and C₁₋₆ alkyl, wherein the 3-10 membered cycloalkyl is optionally substituted with one or more (e.g., 1, 2, 3 or 4) substituents independently selected from the group consisting of halogen, hydroxy, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl, and the C₁₋₆ alkyl is optionally substituted with one or more (e.g., 1, 2, 3 or 4) substituents independently selected from the group consisting of halogen, hydroxy, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl, preferably, R₅ is independently selected from the group consisting of hydrogen, 3-10 membered cycloalkyl, and C₁₋₆ alkyl optionally substituted with one or more (e.g., 1, 2, 3 or 4) C₁₋₆ alkoxy, more preferably, R₅ is independently selected from the group consisting of hydrogen, cyclopropyl, and methoxymethyl.

According to an embodiment of the present invention, when K is CR₄ and J is N, R₂ is phenyl optionally substituted with one or more (e.g., 1, 2, 3 or 4) halogen, preferably 2-fluorophenyl; and R₅ independently selected from the group consisting of 3-10 membered cycloalkyl and C₁₋₆ alkyl, wherein the 3-10 membered cycloalkyl is optionally substituted with one or more (e.g., 1, 2, 3 or 4) substituents independently selected from the group consisting of halogen, hydroxy, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl, and the C₁-C₆ alkyl is optionally substituted with one or more (e.g., 1, 2, 3 or 4) substituents independently selected from the group consisting of halogen, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl,, preferably, R₅ is independently selected from the group consisting of 3-10 membered cycloalkyl, and C₁₋₆ alkyl optionally substituted with one or more (e.g., 1, 2, 3 or 4) C₁₋₆ alkoxy, more preferably, R₅ is independently selected from the group consisting of cyclopropyl and methoxymethyl.

According to an embodiment of the present invention, when K is CR₄ and J is N, R₂ is phenyl or pyridyl, preferably phenyl or 2-pyridyl; and the compound of the present invention is not:
methyl (S)-3-(8-chloro-6-(pyridin-2-yl)-4H-benzo[f]imidazo[1,2-a][1,4]diazepin-4-yl) propanoate, methyl (S)-3-(8-chloro-1-methyl-6-(pyridin-2-yl)-4H-benzo[f]imidazo[1,2-a][1,4] diazepin-4-yl)propanoate, methyl (S)-3-(8-bromo-1-methyl-6-(pyridin-2-yl)-4H-benzo[f] imidazo[1,2-a][1,4]diazepin-4-yl)propanoate, methyl (S)-3-(8-bromo-2-methyl-6-(pyridin-2-yl)-4H-benzo[f]imidazo[1,2-a][1,4]diazepin-4-yl) propanoate, methyl (S)-3-(8-chloro-2-methyl-6-(pyridin-2-yl)-4H-benzo[f]imidazo[1,2-a][1,4]diazepin-4-yl) propanoate, methyl (S)-3-(8-chloro-2-(hydroxymethyl)-6-(pyridin-2-yl)-4H-benzo[f]imidazo [1,2-a][1,4]diazepin-4-yl)propanoate, methyl (S)-3-(8-chloro-1-(hydroxymethyl)-6-(pyridin-2-yl)-4H-benzo[f]imidazo[1,2-a][1,4]diazepin-4-yl)propanoate, methyl (S)-3-(8-chloro-1,2-dimethyl-6-(pyridin-2-yl)-4H-benzo[f]imidazo[1,2-a][1,4]diazepin-4-yl) propanoate, or methyl (S)-3-(8-chloro-1-methyl-2-(hydroxymethyl)-6-(pyridin-2-yl)-4H-benzo[f]imidazo[1,2-a][1,4]diazepin-4-yl)propanoate.

According to an embodiment of the present invention, the above-mentioned hydrogen and the hydrogen contained in the above-mentioned substituents are independently selected from the group consisting of protium, deuterium and tritium, preferably, selected from the group consisting of protium or deuterium.

The present invention encompasses compounds of Formula I obtained through any combination of the above-mentioned preferred definitions of substituents.

According to an embodiment of the present invention, the compound of the present invention is selected from the group consisting of:
methyl 3-(8-bromo-1-methyl-6-(pyridin-2-yl)-4H-benzo[f]imidazo[1,2-a][1,4]diazepin-4-yl)acrylate (compound 1),
methyl 3-(8-bromo-6-(pyridin-2-yl)-4H-benzo[f]imidazo[1,2-a][1,4]diazepin-4-yl)propanoate (compound 2),
methyl 3-(8-chloro-6-phenyl-4H-benzo[f][1,2,4]triazolo[4,3-a][1,4]diazepin-4-yl)propanoate (compound 3),
methyl 3-(8-bromo-1-methyl-6-(pyridin-2-yl)-4H-benzo[f][1,2,4]triazolo[4,3-a][1,4]diazepin-4-yl)propanoate (compound 4),
methyl 3-(8-bromo-1-cyclopropyl-6-(pyridin-2-yl)-4H-benzo[f]imidazo[1,2-a][1,4]diazepin-4-yl)propanoate (compound 5),
methyl 3-(8-bromo-1-deuteromethyl-6-(pyridin-2-yl)-4H-benzo[f]imidazo[1,2-a][1,4]diazepin-4-yl)propanoate (compound 6),
methyl 3-(8-chloro-6-(2-fluorophenyl)-4H-benzo[f][1,2,4]triazolo[4,3-a][1,4]diazepin-4-yl)propanoate (compound 7),
methyl 3-(8-bromo-1-cyclopropyl-6-(pyridin-2-yl)-4H-benzo[f][1,2,4]triazolo[4,3-a][1,4]diazepin-4-yl)propanoate (compound 8),
methyl 3-(8-bromo-6-(pyridin-2-yl)-4H-benzo[f][1,2,4]triazolo[4,3-a][1,4]diazepin-4-yl)propanoate (compound 9),
methyl 3-(8-bromo-1-(methoxymethyl)-6-(pyridin-2-yl)-4H-benzo[f]imidazo[1,2-a][1,4]diazepin-4-yl)propanoate (compound 10),
methyl 3-(8-bromo-1-deuteromethyl-6-(pyridin-2-yl)-4H-benzo[f][1,2,4]triazolo[4,3-a][1,4]diazepin-4-yl)propanoate (compound 11),
methyl 3-(8-chloro-1-cyclopropyl-6-(2-fluorophenyl)-4H-benzo[f][1,2,4]triazolo[4,3-a][1,4]diazepin-4-yl)propanoate (compound 12),
methyl 3-(8-bromo-1-ethenyl-6-(pyridin-2-yl)-4H-benzo[f]imidazo[1,2-a][1,4]diazepin-4-yl)propanoate (compound 13),
methyl 3-(8-chloro-1-cyclopropyl-6-(pyridin-2-yl)-4H-benzo[f][1,2,4]triazolo[4,3-a][1,4]diazepin-4-yl)propanoate (compound 14),
methyl 3-(8-bromo-1-ethyl-6-(pyridin-2-yl)-4H-benzo[f]imidazo[1,2-a][1,4]diazepin-4-yl)propanoate (compound 15),
methyl 3-(8-bromo-1-(methoxymethyl)-6-(pyridin-2-yl)-4H-benzo[f][1,2,4]triazolo[4,3-a][1,4]diazepin-4-yl)propanoate (compound 16),
methyl 3-(8-bromo-1-isopropyl-6-(pyridin-2-yl)-4H-benzo[f]imidazo[1,2-a][1,4]diazepin-4-yl)propanoate (compound 17),
methyl 3-(8-chloro-6-(pyridin-2-yl)-4H-benzo[f][1,2,4]triazolo[4,3-a][1,4]diazepin-4-yl)propanoate (compound 18),
methyl 3-(8-chloro-1-cyclopropyl-6-(pyridin-2-yl)-4H-benzo[f]imidazo[1,2-a][1,4]diazepin-4-yl)propanoate (compound 19),
methyl 3-(8-bromo-1-cyclobutyl-6-(pyridin-2-yl)-4H-benzo[f]imidazo[1,2-a][1,4]diazepin-4-yl)propanoate (compound 20),
methyl 3-(8-bromo-1-methyl-6-(pyridin-2-yl)-4H-benzo[f]imidazo[1,2-a][1,4]diazepin-4-yl)butyrate (compound 21),
methyl 3-(8-bromo-1-methyl-6-(pyridin-2-yl)-4H-benzo[f]imidazo[1,2-a][1,4]diazepin-4-yl)-2-methylpropanoate (compound 22),
methyl 3-(8-bromo-1-cyclopropyl-2-methyl-6-(pyridin-2-yl)-4H-benzo[f]imidazo[1,2-a][1,4]diazepin-4-yl)propanoate (compound 23),
methyl 3-(8-bromo-1-(hydroxymethyl)-6-(pyridin-2-yl)-4H-benzo[f]imidazo[1,2-a][1,4]diazepin-4-yl)propanoate (compound 24),
methyl 3-(8-chloro-6-(2-fluorophenyl)-1-(methoxymethyl)-4H-benzo[f][1,2,4]triazolo[4,3a][1,4]diazepin-4-yl)propanoate (compound 25),
methyl 3-(8-chloro-6-(2-fluorophenyl)-1-(methoxymethyl)-4H-benzo[f]imidazo[1,2-a][1,4]diazepin-4-yl)propanoate (compound 26),
methyl 3-(8-chloro-1-cyclobutyl-6-(pyridin-2-yl)-4H-benzo[f]imidazo[1,2-a][1,4]diazepin-4-yl)propanoate (compound 27),
methyl 3-(8-chloro-1-ethyl-6-(pyridin-2-yl)-4H-benzo[f][1,2,4]triazolo[4,3-a][1,4]diazepin-4-yl)propanoate (compound 28),
methyl 3-(8-chloro-1-isopropyl-6-(pyridin-2-yl)-4H-benzo[f][1,2,4]triazolo[4,3-a][1,4]diazepin-4-yl)propanoate (compound 29),
ethyl 3-(8-chloro-1-methyl-6-(pyridin-2-yl)-4H-benzo[f]imidazo[1,2-a][1,4]diazepin-4-yl)propanoate (compound 30),
methyl 3-(8-chloro-1-cyclopropyl-2-methyl-6-(pyridin-2-yl)-4H-benzo[f]imidazo[1,2-a][1,4]diazepin-4-yl)propanoate (compound 31),
methyl 3-(8-chloro-1-(methoxymethyl)-6-(pyridin-2-yl)-4H-benzo[f][1,2,4]triazolo[4,3-a][1,4]diazepin-4-yl)propanoate (compound 32),
methyl 3-(8-chloro-1-(methoxymethyl)-6-(pyridin-2-yl)-4H-benzo[f]imidazo[1,2-a][1,4]diazepin-4-yl)propanoate (compound 33),
methyl 3-(8-chloro-6-(2-fluorophenyl)-1-cyclopropyl-4H-benzo[f]imidazo[1,2-a][1,4]diazepin-4-yl)propanoate (compound 34),
methyl 3-(8-bromo-1-(difluoromethyl)-6-(pyridin-2-yl)-4H-benzo[f]imidazo[1,2-a][1,4]diazepin-4-yl)propanoate (compound 35),
methyl 3-(8-bromo-6-(pyridin-2-yl)-1-(trifluoromethyl)-4H-benzo[f]imidazo[1,2-a][1,4]diazepin-4-yl)propanoate (compound 36),
methyl 3-(8-bromo-1-((N,N-dimethylamino)methyl)-6-(pyridin-2-yl)-4H-benzo[f]imidazo[1,2-a][1,4]diazepin-4-yl)propanoate (compound 37),
methyl 3-(8-bromo-1-ethynyl-6-(pyridin-2-yl)-4H-benzo[f]imidazo[1,2-a][1,4]diazepin-4-yl)propanoate (compound 38),
methyl 3-((8-bromo-1-methoxy-6-(pyridin-2-yl)-4H-benzo[f]imidazo[1,2-a][1,4]diazepin-4-yl)propanoate (compound 39),
methyl 3-(8-bromo-1-(2-(N,N-dimethylamino)ethyl)-6-(pyridin-2-yl)-4H-benzo[f]imidazo[1,2-a][1,4]diazepin-4-yl)propanoate (compound 40),
methyl 3-(8-bromo-2-((N,N-dimethylamino)methyl)-6-(pyridin-2-yl)-4H-benzo[f]imidazo[1,2-a][1,4]diazepin-4-yl)propanoate (compound 41),
methyl 3-(8-bromo-1-methyl-6-(pyridin-2-yl)-4H-benzo[f]imidazo[1,5-a][1,4]diazepin-4-yl)propanoate (compound 42),
methyl 3-(8-bromo-1-(1-methylcyclopropyl)-6-(pyridin-2-yl)-4H-benzo[f]imidazo[1,2-a][1,4]diazepin-4-yl)propanoate (compound 43),
methyl 3-(8-bromo-1-(oxetan-3-yl)-6-(pyridin-2-yl)-4H-benzo[f]imidazo[1,2-a][1,4]diazepin-4-yl)propanoate (compound 44),
methyl 3-(8-chloro-1-methyl-6-(pyridin-2-yl)-4H-benzo[f]imidazo[1,2-a][1,4]diazepin-4-yl)butyrate (compound 45),
methyl 3-(8-bromo-2-((N,N-diethylamino)methyl)-6-(pyridin-2-yl)-4H-benzo[f]imidazo[1,2-a][1,4]diazepin-4-yl)propionate (compound 46), and
methyl 3-(8-chloro-1-methyl-6-(pyridin-2-yl)-4H-benzo[f]imidazo[1,2-a][1,4]diazepin-4-yl)-2-methylpropionate (compound 47).

According to a preferred embodiment of the present invention, the compound of the present invention is selected from the group consisting of: 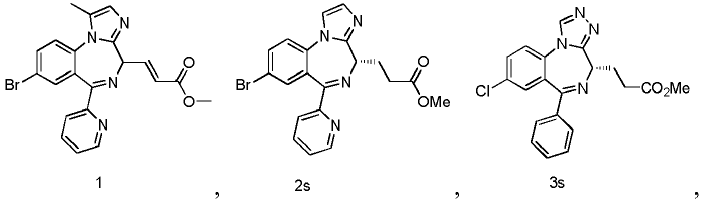 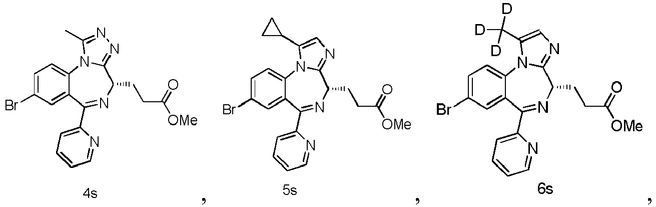 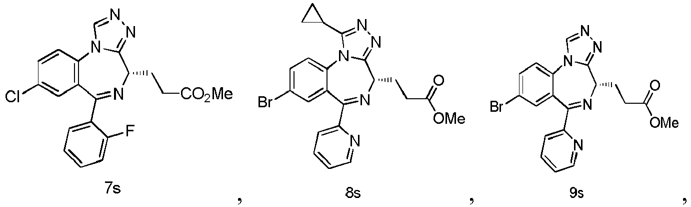 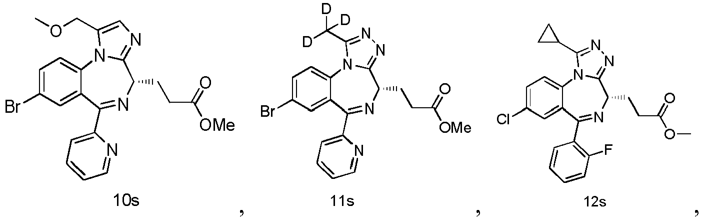 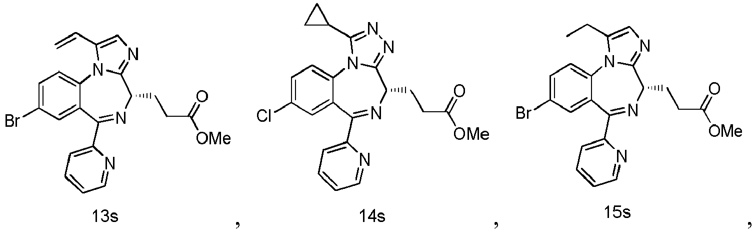 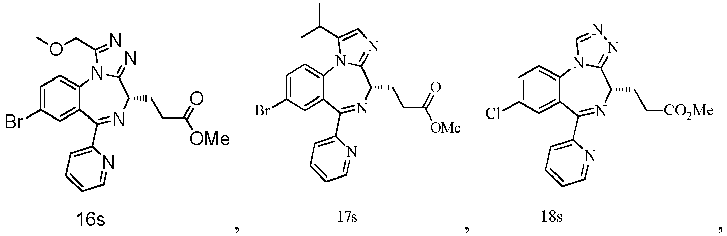 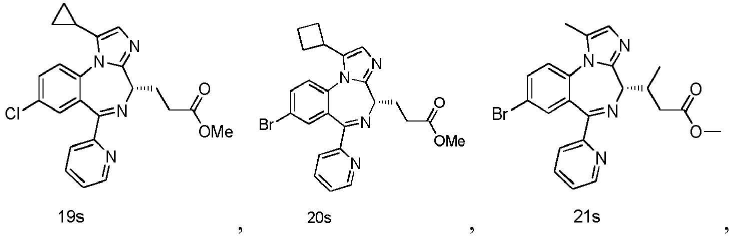 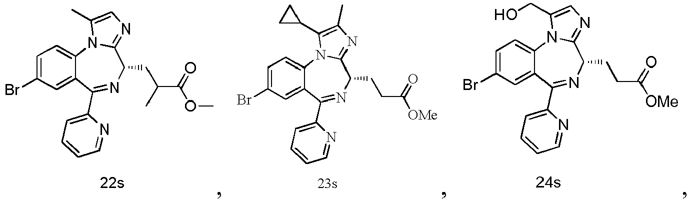 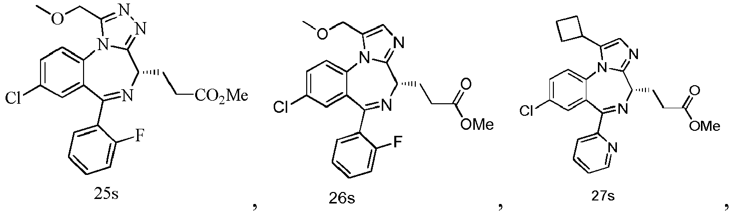 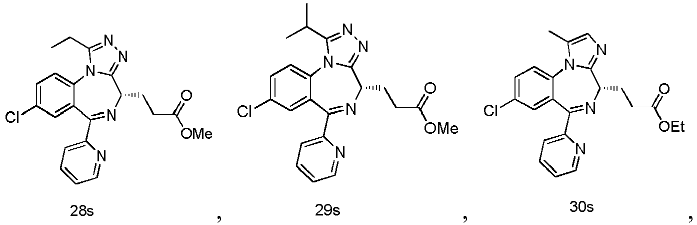 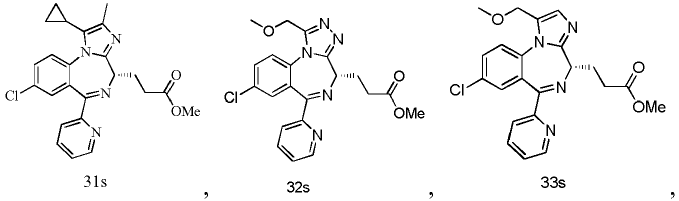 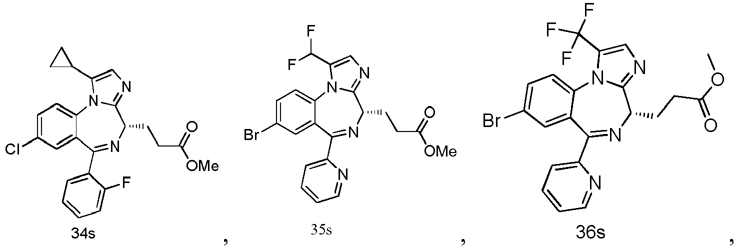 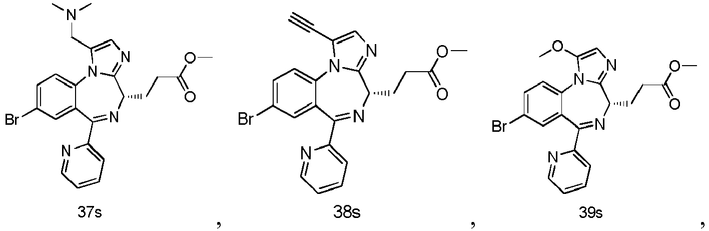 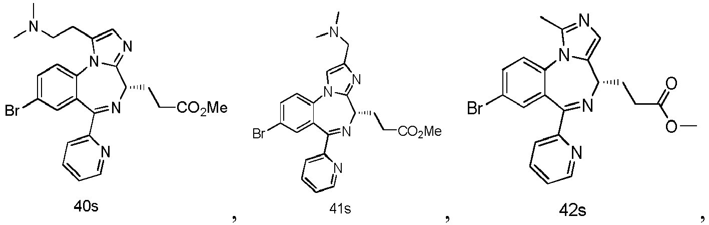 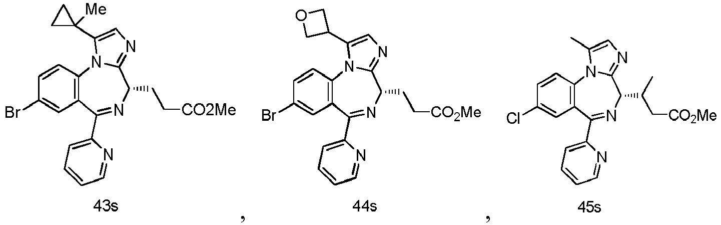 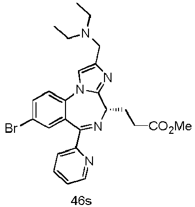 and

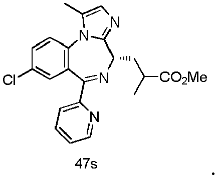

### Pharmaceutical composition and kit

Another object of the present invention is to provide a pharmaceutical composition comprising an effective amount of the compound of the present invention and one or more pharmaceutically acceptable carriers.

The term "pharmaceutically acceptable carrier" used in the present invention refers to a diluent, adjuvant, excipient, or vehicle administered together with the therapeutic agent, which, according sound medical judgment, is suitable for contacting the tissues of human and/or other animals without undue toxicity, irritation, allergic reaction or other problems or complications beyond a reasonable benefit / risk ratio.

The pharmaceutically acceptable carrier which can be employed in the pharmaceutical composition of the present invention may includes, but is not limited to, sterile liquids, such as water and oils, including those of petroleum, animal, vegetable or synthetic origin, such as peanut oil, soybean oil, mineral oil, sesame oil and the like. Water is an exemplary carrier when the pharmaceutical composition is administered intravenously. Physiological salines as well as aqueous dextrose and glycerol solutions can also be employed as liquid carriers, particularly for injectable solutions. Suitable pharmaceutical excipients include starch, glucose, lactose, sucrose, gelatin, maltose, chalk, silica gel, sodium stearate, glycerol monostearate, talc, sodium chloride, dried skim milk, glycerol, propylene glycol, water, ethanol and the like. The composition, if desired, can also contain minor amounts of wetting agents, emulsifying agents, or pH buffering agents.

The pharmaceutical compositions of the present invention may be administrated via suitable routes. Preferably, the pharmaceutical compositions of the present invention are administrated via intravenous, intraarterial, subcutaneous, intraperitoneal, intramuscular, or transdermal routes.

The content or amount of the compound of the present invention in a pharmaceutical composition may be from about 0.01 mg to about 1000 mg, appropriately 0.1-500 mg, preferably 0.5-300 mg, more preferably 1-150 mg, particularly preferably 1-50 mg, e.g., 2 mg, 4 mg, 6 mg, 8 mg, 10 mg, 25 mg and so on.

Another object of the present invention is to provide a kit comprising the compound of the present invention or the pharmaceutical composition of the present invention.

### Method of Preparation

Another object of the present invention is to provide a method for preparing the compound of the present invention.

When J is N, the compound of Formula I of the present invention can be prepared according to Scheme I: 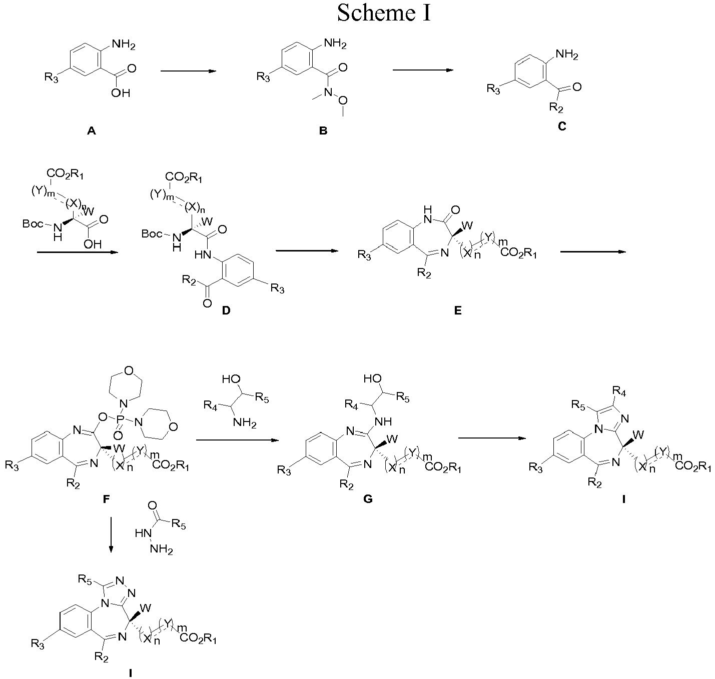 wherein R₁-R₅, n, m, W are defined as above; and the bond between X and Y is a single bond or a double bond.

The method according to Scheme I comprises the following steps:
a. Compound A is allowed to react with N,O-dimethylhydroxylamine hydrochloride to obtain amide intermediate B.
   Preferably, the reaction is carried out in the presence of a base and a condensing agent. The base includes, for example, but is not limited to DIPEA, N- methylmorpholine, or TEA. The condensing agent includes, for example, but is not limited to HATU, DCC, EDCI, PyBOP, BOP-Cl or T3P.
   Preferably, the reaction is carried ou in a suitable solvent. The suitable solvent includes, for example, but is not limited to THF, DMF or DCM.
b. The amide intermediate B is allowed to react with a lithium reagent and a bromo-substituted aryl or heteroaryl compound to obtain a ketone intermediate C.
   Preferably, the reaction is carried out in a suitable solvent. The suitable solvent includes, for example, but is not limited to THF or diethyl ether.
   The lithium reagent includes, for example, but is not limited to n-butyllithium.
   The bromo-substituted aryl or heteroaryl compound includes, for example, but is not limited to bromobenzene, 1-bromo-2-fluorobenzene or 2-bromopyridine.
c. A condensation reaction is carried out between the ketone intermediate C and an N-protected chiral amino acid or a derivative thereof to obtain intermediate D.
   Preferably, the reaction is carried out in the presence of a base and a condensing agent. The base includes, for example, but is not limited to DIPEA, N-methylmorpholine or TEA. The condensing agent includes, for example, but is not limited to HATU, DCC, EDCI, PyBOP, BOP-Cl or T3P.
   Preferably, the reaction is carried out in a suitable solvent. The suitable solvent includes, for example, but is not limited to THF, DMF or DCM.
   Preferably, the reaction is carried out in an ice-water bath or at room temperature.
   The chiral amino acid or derivative thereof includes, for example, but is not limited to 5-methyl L-glutamate or 5-ethyl L-glutamate.
d. The intermediate D is deprotected to obtain benzodiazepine intermediate E.
   Preferably, the reaction is carried out under acidic conditions. The acid used in the acidic condictions includes, for example, but is not limited to trifluoroacetic acid or hydrochloric acid.
f. The benzodiazepine intermediate E is allowed to react with dimorpholino phosphinic chloride to obtain reactive intermediate F.
   Preferably, the reaction is carried out in the presence of a strong base. The strong base includes, for example, but is not limited to, sodium hydride or *t*-BuOK.
   Preferably, the reaction is carried out in a suitable solvent. The suitable solvent includes, for example, but is not limited to THF or diethyl ether.
g. A substitution reaction is carried out between the reactive intermediate F and an amino alcohol to obtain intermediate G.
   The amino alcohol includes, for example, but is not limited to 2-amino-1-cyclopropylethanol, 1-amino-3-methoxypropan-2-ol, 2-amino-1-cyclopropylpropan-1-ol, 2-amino-2-cyclopropylethanol or 1-amino-2,3,3,3-tetradeuteropropan-2-ol.
h. The intermediate G is oxidized through an oxidation reaction to achieve ring closure and obtain the target product I.
   Preferably, the reaction is carried out in a suitable solvent. The suitable solvent includes, for example, but is not limited to CH₃CN, DMF or DCM.
   The oxidation reaction can be achieved by, for example, Swern oxidation.
   Alternatively, the oxidation reaction can be achieved using an oxidizing agent, for example, but not limited to, PCC, PDC, TEMPO or Dess-Martin periodinane.
   Alternatively, the target product I can be obtained as follows.
h'. A substitution reaction is carried out between the reactive intermediate F and a hydrazide, followed by cyclization via condensation, to obtain the target product I.

The hydrazide includes, for example, but is not limited to cyclopropane cyclopropane carbohydrazide, 2-methylpropionyl hydrazide, propionyl hydrazide, 2-methoxyacethydrazide or acethydrazide-D₃.

When K is CR₄ and J is N, the compound of Formula I of the present invention can be prepared according to the Scheme II: 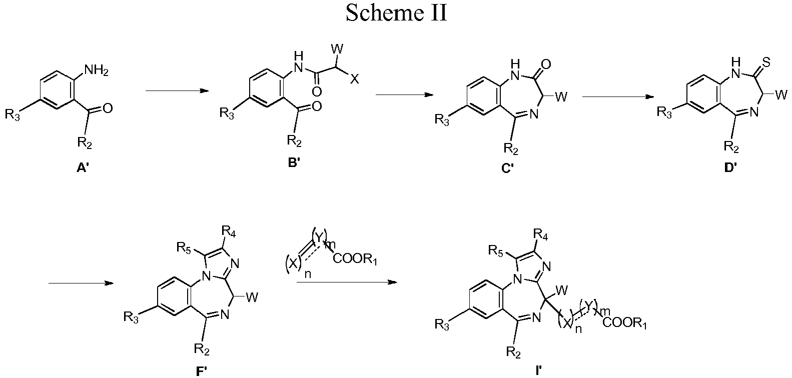 wherein R₁-R₄, W are defined as above; the bond between X and Y is a single bond or a double bond; m and n are each 1; and R₅ is C₁₋₆ alkyl optionally substituted with one or more (e.g., 1, 2, 3 or 4) substituents independently selected from the group consisting of deuterium, halogen, hydroxy, cyano, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl, preferably, R₅ is selected from the group consisting of methyl, ethyl, isopropyl, trifluoromethyl, difluoromethyl, hydroxymethyl, methoxymethyl, N,N-dimethylaminomethyl, N,N-dimethylaminoethyl, N,N-diethylaminomethyl, and deuteromethyl.

The method according to Scheme II comprises the following steps:
a. Intermediate A' is allowed to react with a halogen-substituted acetyl halide to obtain intermediate B'.
   Examples of the halogen-substituted acetyl halide include, but are not limited to, chloroacetyl chloride or bromoacetyl bromide.
b. The intermediate B' undergoes cyclization to obtain lactam intermediate C'.
   Preferably, the reaction is carried out in an alcohol or ether solution of excess ammonia. Examples of the alcohol include, but are not limited to, methanol, ethanol or isopropanol. Examples of the ether include, but are not limited to, tetrahydrofuran or dioxane.
   Preferably, the reaction is carried out under heating conditions. Examples of heating conditions include, but not limited to, at a temperature of 30 °C to 80 °C.
c. A sulfur-oxygen exchange reaction is carried out on the lactam intermediate C in the presence of a thiating agent to obtain thiolactam intermediate D'.
   Preferably, the reaction is carried out under heating conditions. Examples of the heating conditions include, but are not limited to, at a temperature of heating at a temperature of 80 °C to 150 °C.
   Examples of the thiating agent include, but are not limited to, phosphorus pentasulfide or Lawesson's reagent.
d. A substitution reaction is carried out between the thiolactam intermediate D' and an amino-containing alkyne, followed by a cyclization reaction to obtain intermediate F'.
   Examples of the amino-containing alkyne include, but are not limited to, prop-2-yn-1-amine, but-2-yn-1-amine, 4-methoxybut-2-yn-1-amine, N,N-dimethylpent-2-yn-1,5-diamine or 3-cyclopropylprop-2-yn-1-amine.
   Preferably, the cyclization reaction is carried out in the presence of a catalyst. Examples of the catalyst include, but are not limited to, Hg(OAc)₂ or HgSO₄.
e. A Michael addition reaction is carried out between the intermediate F' and an enoate ester or an alkynoate ester to obtain racemic target molecule I', followed by a chiral separation to obtain enantiomerically pure target molecule I'.

Examples of the enoate ester include, but are not limited to, methyl acrylate, ethyl methylbut-2-enoate or methyl methacrylate.

Examples of the alkynoate ester include, but are not limited to, methyl propiolate or methyl methylbut-2-ynoate.

Preferably, the Michael addition reaction is carried out in the presence of a base. Examples of the base include, but are not limited to, sodium hydride, LDA or *t*-BuOK.

Preferably, the Michael addition reaction is carried out in a suitable solvent. Examples of the suitable solvent include, but are not limited to, THF or 2-methyltetrahydrofuran.

When K is N and J is CR₄, the compound of Formula I of the present invention can be prepared according to Scheme III: 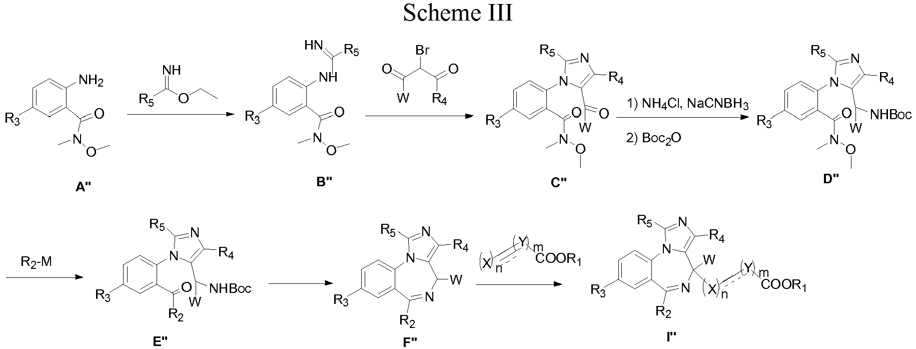 wherein R₁-R₅, W are defined above; the bond between X and Y is a single bond or a double bond; and m and n are each 1.

The method according to scheme III comprises the following steps:
a. Intermediate A" is allowed to react with substituted ethyl imidate to obtain intermediate B".
   Examples of the substituted ethyl imidate include, but are not limited to, ethyl acetimidate, propionimidic acid ethyl ester, 2-methoxy-acetimidic acid ethyl ester or cyclopropanecarboximidic acid ethyl ester.
b. A condensation reaction is carried out between the intermediate B" and 2-bromomalonaldehyde to obtain intermediate C".
   Preferably, the reaction is carried out under heating conditions. Examples of the heating conditions include, but are not limited to, at a temperature of 50 °C to 150 °C.
c. A two-step reaction is carried out to convert the intermediate C" to intermediate D", wherein the first step converts the aldehyde group in the intermediate C" to aminomethyl using reductive amination, and the second step protects the amino group in the aminomethyl with Boc₂O to obtain a *tert*-butyl carbamate.
d. The intermediate D" is allowed to react with a lithium reagent and a bromo-substituted aryl or heteroaryl compound to obtain ketone intermediate E".
   Preferably, the reaction is carried out in a suitable solvent. Examples of the suitable solvent include, but are not limited to THF or diethyl ether.
   Examples of the lithium reagent include, but are not limited to, *n*-butyllithium.
   Examples of the bromo-substituted aryl or heteroaryl compound include, but are not limited to, bromobenzene, 1-bromo-2-fluorobenzene or 2-bromopyridine.
e. The intermediate E" is deprotected, followed by cyclization under the same reaction conditions to obtain intermediate F".
   Preferably, the reaction is carried out under acidic conditions. Examples of the acid used in the acid conditions include, but are not limited to, trifluoroacetic acid or hydrochloric acid.
f. a Michael addition reaction is carried out between the intermediate F" and an enoate ester or or an alkynoate ester to obtain racemic target molecule I', followed by a chiral separation to obtain enantiomerically pure target molecule I'.

Examples of the enoate ester include, but are not limited to, methyl acrylate, ethyl methylbut-2-enoate or methyl methacrylate.

Examples of the alkynoate ester include, but are not limited to, methyl propiolate or methyl methylbut-2-ynoate.

Preferably, the Michael addition reaction is carried out in the presence of a base. Examples of the base include, but are not limited to, sodium hydride, LDA or *t*-BuOK.

Preferably, the Michael addition reaction is carried out in a suitable solvent. Examples of the suitable solvent include, but are not limited to, THF or 2-methyltetrahydrofuran.

### Treatment Method and Use

Another object of the present invention is to provide a method of anesthesia, comprising administering, preferably intravenously, intraarterially, subcutaneously, intraperitoneally, intramuscularly or transdermally, an effective amount of the compound of the present invention or the pharmaceutical composition of the present invention. The method of anesthesia is preferably used in the following clinical settings: presurgical sedation, antianxiety and anti-amnesia during surgery; conscious sedation during short-term diagnostic, surgical or endoscopic procedures; induction and maintenance of general anesthesia prior to and/or at the same time of administration of other anesthetics and analgesics; ICU sedation; etc.

Another object of the present invention is to provide the compound of present invention, for use as an anesthetic medicament. The anesthetic medicament is preferably used for intravenous administration in the following clinical settings: presurgical sedation, antianxiety and anti-amnesia during surgery; conscious sedation during short-term diagnostic, surgical or endoscopic procedures; induction and maintenance of general anesthesia prior to and/or at the same time of administration of other anesthetics and analgesics; ICU sedation; etc.

Another object of the present invention is to provide use of the compound of the present invention or the pharmaceutical composition of the present invention in the manufacture of an anesthetic medicament. The anesthetic medicament is preferably used for intravenous administration in the following clinical settings: presurgical sedation, antianxiety and anti-amnesia during surgery; conscious sedation during short-term diagnostic, surgical or endoscopic procedures; induction and maintenance of general anesthesia prior to and/or at the same time of administration of other anesthetics and analgesics; ICU sedation; etc.

The dose regimen can be adjusted to achieve an optimal response. For example, the drug can be administered in a single bolus or in several sub-doses over time, or the dose can be proportionally decreased or increased according to practical requirement of treatment. It should be noted that the dose may vary depending on the type and severity of the condition to be alleviated, and may include single- or multi-doses. It will be further understood that for any particular subject, the specific dose regimen should be adjusted according to the requirement of the subject, and the professional judgment of the skilled physician who is in charge of administering or monitoring the administration of the pharmaceutical composition.

The amount of the compound of the present invention to be administered will depend on the subject to be treated, the rate of administration, the form of the compound, and the judgment of the prescribing physician. In general, the effective dose is about 0.0001 to about 50 mg per kg body weight per day, for example about 0.01 to about 10 mg/kg/day (in single-or multi-doses). For a person of 70 kg, the effective dose will be about 0.007 mg/day to about 3500 mg/day in total, for example, about 0.7 mg/day to about 700 mg/day in total. In some instances, dose levels below the lower limit of the aforementioned range may be adequate; while in other cases, a higher dose can be employed without causing any disadvantageous side effects, with the proviso that the higher dose is divided into several sub-doses to be administered throughout a day.

### EXAMPLES

The present invention has been further described in detail with reference to the following examples for apparency of the purpose and technical solution of the present invention. It should be understood that these examples are merely provided for illustration of the present invention and are not intended to limit the scope of the invention. In addition, the specific experimental procedures not mentioned in the examples below are carried out according to conventional experimental procedures.

The abbreviations as used herein have the following meanings:

| **Abbreviation** | **Meaning** |
|---|---|
| BBr₃ | boron tribromide |
| BOP-Cl | bis(2-oxo-3-oxazolidinyl)phosphonic chloride |
| DCM | dichloromethane |
| Dess- Martin periodinane | Dess-Martin periodinane 1,1,1-triacetoxy-1,1-dihydro-1,2-benziodoxol-3-(1H)-one |
| DCC | dicyclo hexylcarbodiimide |
| DIPEA | N,N-diisopropylethylamine |
| DMAP | 4-dimethylaminopyridine |
| EDCI | 1-ethyl-3-(3-dimethylaminopropyl)carbodiimide hydrochloride |
| MeOH | methanol |
| THF | tetrahydrofuran |
| EtOH | ethanol |
| DMF | N,N-dimethylformamide |
| DMF-DMA | *N,N*-dimethylformamide dimethylacetal |
| HATU | 2-(7-azobenzotriazole)-N,N,N',N'-tetramethyluronium hexafluorophosphate |
| HgSO₄ | mercury sulfate |
| NMP | N-methylpyrrolidinone |
| NaN₃ | sodium azide |
| LAH | lithium aluminum hydride |
| TMSCN | trimethylsilyl cyanide |
| LCMS | liquid chromatography-mass spectrometry |
| LDA | lithium diisopropylamide |
| P₂S₅ | phosphorus pentasulfide |
| PCC | pyridinium chlorochromate |
| PDC | pyridinium dichromate |
| PyBOP | (benzotriazol-1-yloxy)tripyrrolidinophosphonium hexafluorophosphate |
| T3P | propylphosphonic anhydride |
| TEMPO | (2,2,6,6-tetramethylpiperidin-1-yl)oxyl |
| Boc₂O | di-*tert*-butyl dicarbonate |
| TEA | triethylamine |
| *t*-BuOK | potassium *tert*-butoxide |
| Pd/C | palladium on carbon |
| DAST | diethylaminosulfur trifluoride |
| TLC | thin layer chromatographyl |
| s | singlet |
| d | doublet |
| t | triplet |
| q | quartet |
| dd | double doublet |
| m | multiplet |
| br | broad |

The structures of compounds were identified by NMR spectroscopy (¹HNMR) or mass spectrometry (MS). The reactions were monitored using thin-layer chromatography (TLC) or LCMS. The developing solvent systems as used include: dichloromethane-methanol system, *n*-hexane-ethyl acetate system, and petroleum ether-ethyl acetate system.

Microwave reactions were conducted using the BiotageInitiator+ (400 W, RT ∼ 300 °C) microwave reactor.

Silica gel of 200 to 300 mesh (Qingdao Ocean) was generally used as the stationary phase in column chromatography. Eluent systems included: dichloromethane-methanol system and *n*-hexane-ethyl acetate system, and the volume ratio of solvents in the systems varied depending on the polarity of compounds.

In the following examples, the reaction temperature was generally room temperature (20 °C ∼ 30 °C), unless otherwise specified.

The reagents used in this application were purchased from Acros Organics, Aldrich Chemical Company or Topbiochem, etc.

### Example 1. Preparation of methyl 3-(8-bromo-1-methyl-6-(pyridin-2-yl)-4H-benzo[f]imidazo[1,2-a][1,4]diazepin-4-yl)acrylate (compound 1)

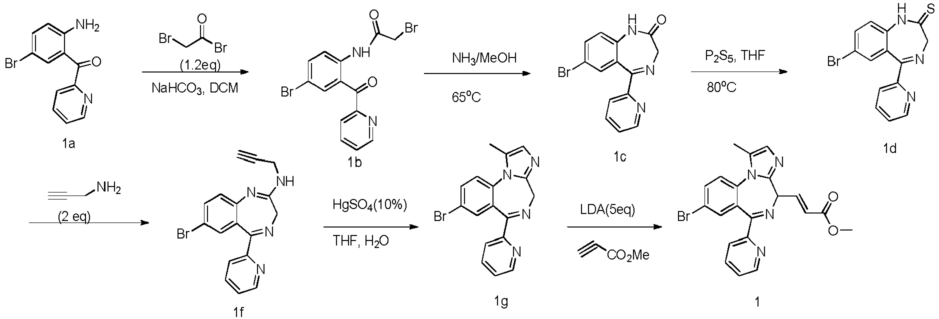

### Step 1. Preparation of 2-bromo-N-(4-bromo-2-(pyridin-2-ylcarbonyl)phenyl)acetamide (compound 1b)

2-(2-Amino-5-bromobenzoyl)pyridine (compound 1a, 60 g, 0.22 mol) was dissolved in DCM (3 L), and NaHCO₃ (36.9 g, 0.44 mol) was added. The mixture was cooled to 0 °C, and 2-bromoacetyl bromide (52.4 g, 0.26 mol) was added dropwise slowly. The reaction mixture was stirred at 0 °C for 2 h until TLC indicated that the reaction was completed. The reaction mixture was concentrated to obtain 2-bromo-N-(4-bromo-2-(pyridin-2-ylcarbonyl)phenyl)acetamide (compound 1b, 88 g, yield.: 100.0%).

### Step 2. Preparation of 7-bromo-5-(pyridin-2-yl)-1H-benzo[e][1,4]diazepin-2(3H)-one (compound 1c)

2-Bromo-N-(4-bromo-2-(pyridin-2-ylcarbonyl)phenyl)acetamide (compound 1b, 88 g, 0.22 mol) was dissolved in MeOH (1.5 L). The mixture was cooled to 0 °C, and ammonia was introduced slowly. The reaction mixture was stirred at 80°C for 4 h until TLC indicated that the reaction was completed. The reaction mixture was concentrated to remove most of MeOH, and was filtered. The resulting solid was washed with DCM and MeOH and dried to obtain 7-bromo-5-(pyridin-2-yl)-1H-benzo[e][1,4]diazepin-2(3H)-one (compound 1c, 40 g, yield. 58.0%).

### Step 3. Preparation of 7-bromo-5-(pyridin-2-yl)-1H-benzo[e][1,4]diazepin- 2(3H)-thione (compound 1d)

7-Bromo-5-(pyridin-2-yl)-1H-benzo[e][1,4]diazepin-2(3H)-one (compound 1c, 10 g, 0.03 mol) was dissolved in THF (300 L), and then P₂S₅ (7.2 g, 0.04 mol) was added. The reaction mixture was stirred at 80 °C for 4 h until TLC indicated that the reaction was completed. The mixture was cooled to 0 °C, and was adjusted to pH ~8 using a saturated NaHCO₃ aqueous solution. The water phase was extracted with ethyl acetate. The organic phase was dried, and concentrated to obtain 7-bromo-5-(pyridin-2-yl)-1H-benzo[e][1,4]diazepin-2(3H)-thione (compound 1d, 8 g, yield. 80.3%).

### Step 4. Preparation of 7-bromo-N-(prop-2-yn-1-yl)-5-(pyridin-2-yl)-3H-benzo[e][1,4]diazepin-2-amine (compound 1f)

7-Bromo-5-(pyridin-2-yl)-1H-benzo[e][1,4]diazepin-2(3H)-thione (compound 1d, 2 g, 6.02 mmol) was dissolved in DMF (20 mL), and prop-2-yn-1-amine (1.66 g, 30.1 mmol) was added. The mixture was heated to 60 °C, and was allowed to react for 2 h. The mixture was cooled, poured into ice water, and extracted with ethyl acetate. The organic layer was washed with water, dried and concentrated to obtain 7-bromo-N-(prop-2-yn-1-yl)-5-(pyridin-2-yl)-3H-benzo[e][1,4]diazepin-2-amine (compound 1f, 2 g, yield: 94%).
MS m/z (ESI): 353 [M+H]⁺.

### Step 5. Preparation of 8-bromo-1-methyl-6-(pyridin-2-yl)-4H-benzo[f]imidazo[1,2-a][1,4]diazepine (compound 1g)

7-Bromo-N-(prop-2-yn-1-yl)-5-(pyridin-2-yl)-3H-benzo[e][1,4]diazepin-2-amine (compound 1f, 1 g, 2.83 mmol) and HgSO₄ (84.25 mg, 0.283 mmol) were dissolved in THF (10 mL) and water (3.5 mL), and conc. HCl was added to adjust pH to ∼ 4. The mixture was stirred under 60 °C for 1 h until LCMS indicated that the reaction was completed. The reaction mixture was concentrated, and the residue was purified through TLC to obtain 8-bromo-1-methyl-6-(pyridin-2-yl)-4H-benzo[f]imidazo[1,2-a][1,4]diazepine (compound 1 g, 300 mg, yield: 30.0%).
MS m/z (ESI): 353 [M+H]⁺.

### Step 6. Preparation of methyl 3-(8-bromo-1-methyl-6-(pyridin-2-yl)-4H-benzo[f]imidazo[1,2-a][1,4]diazepin-4-yl)acrylate (compound 1)

8-Bromo-1-methyl-6-(pyridin-2-yl)-4H-benzo[f]imidazo[1,2-a][1,4]diazepine (compound 1g, 300 mg, 0.85 mmol) was dissolved in dried THF (2 mL). The mixture was cooled to -65 °C, and LDA (2.13 mL, 4.25 mmol) was added dropwise. The mixture was stirred for 30 min, and then methyl propiolate (72 mg, 1.7 mmol) was added. The reaction mixture was stirred for 1 h until LCMS indicated that the reaction was completed. The reaction mixture was concentrated, and the residue was purified through preparative TLC to obtain methyl 3-(8-bromo-1-methyl-6-(pyridin-2-yl)-4H-benzo[f]imidazo[1,2-a][1,4]diazepin-4-yl)acrylate (compound 1, 16 mg, yield: 4.3%).
MS m/z (ESI): 437[M+H]⁺.
¹HNMR (400 MHz, CDCl₃) δ: 8.81(s, 1H), 8.20 (s, 1H), 7.74 (s, 1H), 7.65-7.63(m, 1H), 7.45-7.43 (m, 1H), 7.38-7.34 (m, 1H), 7.16-7.14 (m, 1H), 6.99-6.90 (m, 3H), 6.73 (s, 1H), 3.81 (s, 3H), 2.13 (s, 3H).

### Example 2. Preparation of (S)-methyl 3-(8-bromo-6-(pyridin-2-yl)-4H-benzo[f]imidazo[1,2-a][1,4]diazepin-4-yl)propanoate (compound 2s)

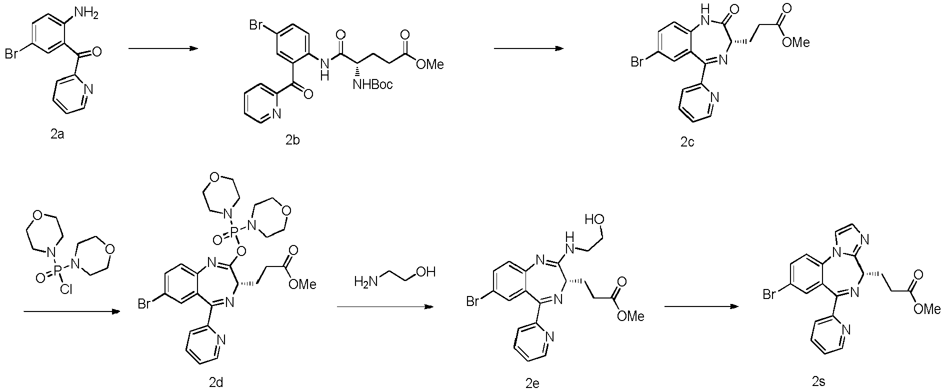

### Step 1. Preparation of (S)-methyl 5-((4-bromo-2-nicotinoylphenyl)amino)-4-((tert-butoxycarbonyl)amino)-5-oxopentanoate (compound 2b)

HATU (45.6 g, 0.12 mol), N-methylmorpholine (20.2 g, 0.2 mol) and 5-methyl N-Boc-L-glutamate (16.1 g, 0.1 mol) were sequentially added to DMF (100 mL) in an ice bath. The resulted mixture was allowed to react for 30 min in an ice bath, and then (2-amino-5-bromophenyl)(pyridin-2-yl)methanone (compound 2a, 27.7 g, 0.1 mol) was added. Water was added to the reaction system after TLC indicated that the reaction was completed, and the mixture was extracted with ethyl acetate (20 mL × 4). The ethyl acetate layer was evaporated to dry, and the residue was purified through silica gel column chromatography to obtain (S)-methyl 5-((4-bromo-2-nicotinoylphenyl)amino)-4-((*tert*-butoxycarbonyl)amino)-5-oxopentanoate (compound 2b, 36 g, yield: 69%).

### Step 2. Preparation of (S)-methyl 3-(7-bromo-2-oxo-5-(pyridin-2-yl)-2,3-dihydro-1H-benzo[e][1,4]diazepin-3-yl)propanoate (compound 2c)

(*S*)-Methyl 5-((4-bromo-2-nicotinoylphenyl)amino)-4-((*tert*-butoxycarbonyl)amino)-5-oxopentanoate (compound 2b, 10 g, 19 mmol) was dissolved in DCM (30 mL), and trifluoroacetic acid (10 mL) was added in an ice bath. After LCMS indicated that the reaction was completed, the solvent was removed by evaporation. The residue was dissolved in methanol (50 mL), and was adjusted to pH ∼10 with Na₂CO₃. The mixture was stirred at room temperature overnight before EtOH was removed by evaporation. The mixture was extracted with ethyl acetate (30 mL × 4). The ethyl acetate layer was dried over Na₂SO₄, and concentrated. The residue was purified through silica gel column chromatography to obtain (S)-methyl 3-(7-bromo-2-oxo-5-(pyridin-2-yl)-2,3-dihydro-1H-benzo[e][1,4]diazepin-3-yl)propanoate (compound 2c, 4.1 g, yield: 53%).

### Step 3. Preparation of (S)-methyl 3-(7-bromo-2-((2-hydroxyethyl)amino)-5-(pyridin-2-yl)-3H-benzo[e][1,4]diazepin-3-yl)propanoate (compound 2e)

(*S*)-Methyl 3-(7-bromo-2-oxo-5-(pyridin-2-yl)-2,3-dihydro-1H-benzo[e][1,4]diazepin-3-yl)propanoate (compound 2c, 1 g, 2.49 mmol) was dissolved in dried THF (10 mL). The solution was cooled to 0 °C, NaH (149.2 mg, 3.73 mmol) was added, and the reaction mixture was stirred for 30 min. Morpholinyl chlorophosphate (1.27 g, 4.98 mmol) was added, and the reaction mixture was stirred for 1 h until LCMS indicated that the reaction was completed. 2-Aminoethanol (609 mg, 9.96 mmol) was added, and the reaction mixture was stirred for 3 h until LCMS indicated that the reaction was completed. The reaction mixture was poured into ice water, and extracted with ethyl acetate. The organic layer was washed with water for three times, dried and concentrated. The residue was purified through preparative TLC to obtain (S)-methyl 3-(7-bromo-2-((2-hydroxyethyl)amino)-5-(pyridin-2-yl)-3H-benzo[e][1,4]diazepin-3-yl)prop anoate (compound 2e, 300 mg, yield: 27.3%).
MS m/z (ESI): 445 [M+H]⁺.

### Step 4. Preparation of (S)-methyl 3-(8-bromo-6-(pyridin-2-yl)-4H-benzo[f]imidazo[1,2-a][1,4]diazepin-4-yl)propanoate (compound 2s)

(*S*)-Methyl 3-(7-bromo-2-((2-hydroxyethyl)amino)-5-(pyridin-2-yl)-3H-benzo[e][1,4]diazepin-3-yl)propanoate (compound 2e, 300 mg, 0.68 mmol) was dissolved in CH₃CN (2 mL). PDC (508.4 mg, 1.38 mmol) and a little silica gel were added, and the mixture was stirred for 2 h until LCMS indicated that the reaction was completed. The reaction mixture was concentrated, and the residue was purified through preparative TLC to obtain (*S*)-methyl 3-(8-bromo-6-(pyridin-2-yl)-4H-benzo[f]imidazo[1,2-a][1,4]diazepin-4-yl)propanoate (compound 2s, 102 mg, yield: 35%).
MS m/z (ESI): 425 [M+H]⁺.
¹HNMR (400 MHz, DMSO-*d6*) δ: 8.53(d, *J=* 4.1Hz, 1H), 8.10-8.08 (m, 1H), 7.99-7.93 (m, 2H), 7.86 (s, 1H), 7.73-7.71 (m, 1H), 7.61-7.60 (m, 1H), 7.53-7.50 (m, 1H), 7.11 (s, 1H),4.20-4.17 (m, 1H), 3.64 (s, 3H), 2.78-2.57 (m, 4H).

### Example 3. Preparation of (S)-methyl 3-(8-chloro-6-phenyl-4H-benzo[f][1,2,4]triazolo[4,3-a][1,4]diazepin-4-yl)propanoate (compound 3s)

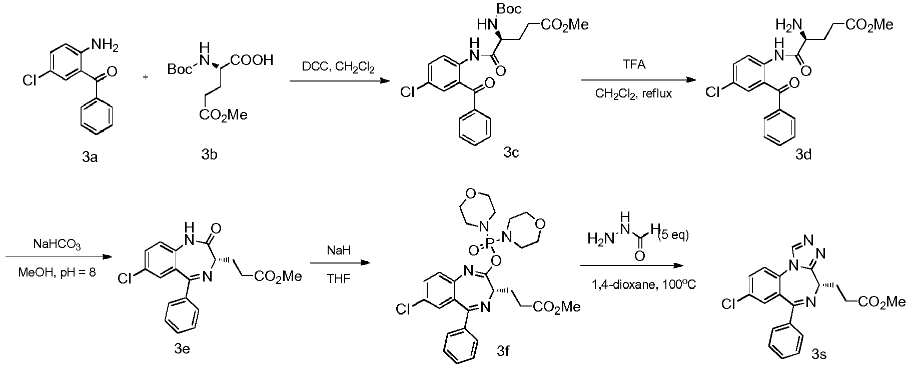

### Step 1. Preparation of (S)-methyl 5-((2-benzoyl-4-chlorophenyl)amino)-4-((tert-butoxycarbonyl)amino)-5-oxopentanoate (compound 3c)

(2-Amino-5-chlorophenyl)(benzyl)methanone (compound 3a, 5 g, 0.022 mol) and 5-methyl N-*tert*-butoxycarbonyl-L-glutamate (compound 3b, 6.32 g, 0.024 mol) were dissolved in DCM (50 mL). The mixture was cooled to 0 °C, and DCC (4.99 g, 0.024 mmol) was added. The reaction mixture was stirred for 24 h until LCMS indicated that the reaction was completed. The reaction mixture was poured into ice water, and extracted with ethyl acetate. The organic layer was washed with water, dried and concentrated. The residue was purified through silica gel column chromatography to obtain (S)-methyl 5-((2-benzoyl-4-chlorophenyl)amino)-4-((*tert*-butoxycarbonyl)amino)-5-oxopentanoate (compound 3c, 7 g, yield: 67.3%).

### Step 2. (S)-methyl 4-amino-5-((2-benzoyl-4-chlorophenyl)amino)-5-oxopentanoate (compound 3d)

(*S*)-Methyl 5-((2-benzoyl-4-chlorophenyl)amino)-4-((*tert*-butoxycarbonyl)amino)-5-oxopentanoate (compound 3c, 7 g, 14.8 mmol) was dissolved in DCM (70 mL), and TFA (70 mL) was added. The reaction mixture was heated to 40 °C and stirred for 2 hrs until LCMS indicated that the reaction was completed. The reaction mixture was concentrated, and the residue was used directly in the next step without further purification (7 g, crude).

### Step 3. Preparation of (S)-methyl 3-(7-chloro-2-oxo-5-phenyl-2,3-dihydro-1H-benzo[e][1,4]diazepin-3-yl)propanoate (compound 3e)

(*S*)-Methyl 4-amino-5-((2-benzoyl-4-chlorophenyl)amino)-5-oxopentanoate (compound 3d, 7 g) was dissolved in MeOH (250 mL), and NaHCO₃ aqueous solution was added to adjust pH to ∼10. The mixture was stirred for 24 h until LCMS indicated that the reaction was completed. The reaction mixture was filtered. The filtrate was poured into ice water, and extracted with ethyl acetate. The organic layer was washed with water, dried and concentrated. The residue was purified through silica gel column chromatography to obtain (S)-methyl 3-(7-chloro-2-oxo-5-phenyl-2,3-dihydro-1H-benzo[e][1,4]diazepin-3-yl)propanoate (compound 3e, 3g, yield: 57.7%).
MS m/z (ESI): 357 [M+H]⁺.

### Step 4. Preparation of (S)-methyl 3-(7-chloro-2-((dimorpholinophosphoryl)oxy)-5-phenyl-3H-benzo[e][1,4]diazepin-3-yl)propanoate (compound 3f)

(*S*)-Methyl 3-(7-chloro-2-oxo-5-phenyl-2,3-dihydro-1H-benzo[e][1,4]diazepin-3-yl)propanoate (compound 3e, 1 g, 2.81 mmol) was dissolved in dried THF (10 mL). The mixture was cooled to 0 °C, and NaH (168.8 mg, 4.22 mmol) was added. The reaction mixture was stirred for 30 min, and morpholinyl chlorophosphate (1.44 g, 5.62 mmol) was added. The reaction mixture was stirred for 1 h until LCMS indicated that the reaction was completed. The reaction mixture was poured into ice water and extracted with ethyl acetate. The organic layer was washed with water, dried and concentrated. The residue was purified through preparative TLC to obtain (*S*)-methyl 3-(7-chloro-2-((dimorpholinophosphoryl)oxy)-5-phenyl-3H-benzo[e] [1,4]diazepin-3-yl)propa noate (compound 3f, 300 mg, yield: 18.8%).

### Step 5. Preparation of (S)-methyl 3-(8-chloro-6-phenyl-4H-benzo[f][1,2,4]triazolo[4,3-a] [1,4]diazepin-4-yl)propanoate (compound 3s)

(*S*)-Methyl 3-(7-chloro-2-((dimorpholinophosphoryl)oxy)-5-phenyl-3H-benzo[e][1,4]diazepin-3-yl)propanoate (compound 3f, 300 mg) was dissolved in 1,4-dioxane (2 mL), and formohydrazide (156.8 mg, 2.61 mmol) was added. The reaction mixture was heated to 100 °C, and stirred for 14 h until LCMS indicated that the reaction was completed. The reaction mixture was concentrated, and the residue was purified through preparative HPLC to obtain (*S*)-methyl 3-(8-chloro-6-phenyl-4H-benzo[f][1,2,4]triazolo[4,3-a][1,4]diazepin-4-yl)propanoate (compound 3s).
MS m/z (ESI): 381 [M+H]+.
¹HNMR (400 MHz, CDCl₃) δ: 8.57 (s, 1H), 7.67-7.65 (m, 1H), 7.52-7.46 (m, 5H), 7.41-7.37 (m, 2H), 4.27 (m, 1H), 3.68 (s, 3H), 2.92-2.88 (m, 4H).

### Example 4. Preparation of (S)-methyl 3-(8-bromo-1-methyl-6-(pyridin-2-yl)-4H-benzo[f][1,2,4]triazolo[4,3-a][1,4]diazepin-4-yl)propanoate (compound 4s)

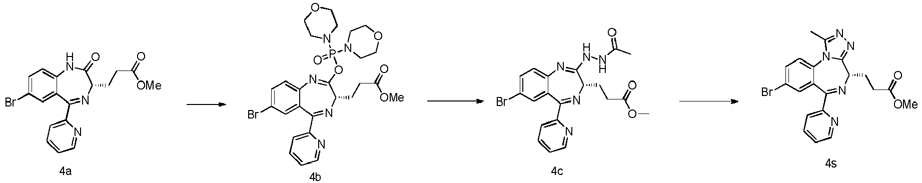

### Step 1. Preparation of (S)-Methyl 3-(7-bromo-2-((dimorpholinophosphoryl)oxy)-5-(pyridin-2-yl)-3H-benzo[e][1,4]diazepin-3-yl)propanoate (compound 4b)

(*S*)-Methyl 3-(7-bromo-2-oxo-5-(pyridin-2-yl)-2,3-dihydro-1H-benzo[e][1,4]diazepin-3-yl)propanoate (compound 4a, 1 g, 2.49 mmol) was dissolved in dried THF (10 mL). The reaction mixture was cooled to 0 °C, and NaH (149.2 mg, 3.73 mmol) was added. The reaction mixture was stirred for 30 min. Morpholinyl chlorophosphate (1.27 g, 4.98 mmol) was added, and the reaction mixture was stirred for 1 h until LCMS indicated that the reaction was completed. Water was added, and the mixture was extracted with ethyl acetate. The organic layer was dried and concentrated. The residue was used directly in the next step without further purification.

### Step 2. Preparation of (S)-methyl 3-(8-bromo-1-methyl-6-(pyridin-2-yl)-4H-benzo[f][1,2,4]triazolo[4,3-a][1,4]diazepin-4-yl)propanoate (compound 4s)

(*S*)-Methyl 3-(7-bromo-2-((dimorpholinophosphoryl)oxy)-5-(pyridin-2-yl)-3H-benzo[e][1,4]diazepin-3-yl)propanoate (compound 4b) obtained in Step 1 was dissolved in dioxane, and acethydrazide (736 mg, 9.96 mmol) was added. The reaction mixture was allowed to react for 2 h at room temperature, then heated to 100 °C, and stirred for 5 hrs. The reaction mixture was poured into ice water, and extracted with ethyl acetate. The organic layer was washed with water, dried and concentrated. The residue was purified through preparative TLC to obtain (*S*)-methyl 3-(8-bromo-1-methyl-6-(pyridin-2-yl)-4H-benzo[f][1,2,4]triazolo[4,3-a][1,4]diazepin-4-yl)propanoate (compound 4s, 200 mg, yield: 18.2%).
MS m/z (ESI): 440 [M+H]⁺.
¹HNMR (400 MHz, DMSO-*d6*) δ: 8.58-8.54 (m, 1H), 8.09 (d, *J* = 8.0 Hz, 1H), 7.98-7.93 (m, 2H), 7.78 (d, *J=* 8.8 Hz, 1H), 7.65 (d, *J=* 2 Hz, 1H), 7.53-7.49 (m, 1H), 4.31-4.28 (m, 1H), 3.62 (s, 3H), 2.76-2.50 (m, 7H).

### Example 5. Preparation of (S)-methyl 3-(8-bromo-1-cyclopropyl-6-(pyridin-2-yl)-4H-benzo[f]imidazo[1,2-a][1,4]diazepin-4-yl)propanoate (compound 5s)

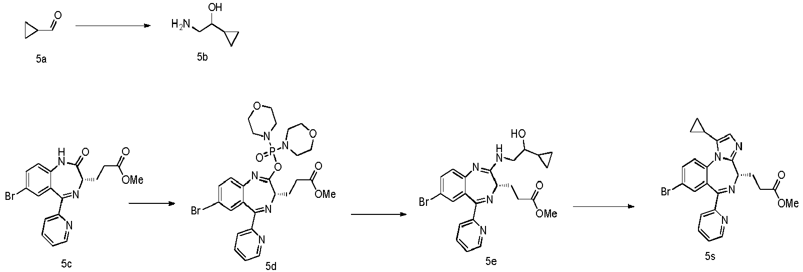

### Step 1. Preparation of 2-amino-1-cyclopropylethanol (compound 5b)

To a 500 mL three-necked flask, TMSCN (25.8 g, 0.26 moL), DCM (300 mL) and ZnI₂ (10 mg) were added. The reaction mixture was cooled to 0 °C under stirring, and cyclopropylformaldehyde (15.2 g, 0.217 mol) was added dropwise. The reaction mixture was stirred at room temperature for 3 h, and then was concentrated to dryness. The residue was dissolved in THF (300 mL), and then LAH (9.88 g, 0.26 mol) was added portionwise in an ice bath. The reaction mixture was allowed to react at room temperature for 3 h. Na₂SO₄·10H₂O (30 g) was added portionwise in an ice bath, and the the reaction mixture was stirred overnight. The reaction mixture was filtered and concentrated to obtain 2-amino-1-cyclopropylethanol (6.5 g, yield: 30%).

### Step 2. Preparation of methyl 3-((3S)-7-bromo-2-((2-cyclopropyl-2-hydroxyethyl)amino)-5-(pyridin-2-yl)-3H-benzo[e][1,4]diazepin-3-yl)propanoate (compound 5e)

(*S*)-Methyl 3-(7-bromo-2-oxo-5-(pyridin-2-yl)-2,3-dihydro-1H-benzo[e][1,4]diazepin-3-yl)propanoate (compound 5c, 1 g, 2.49 mmol) was dissolved in dried THF (10 mL). The reaction mixture was cooled to 0 °C, and NaH (149.2 mg, 3.73 mmol) was added. The reaction mixture was stirred for 30 min. Morpholinyl chlorophosphate (1.27 g, 4.98 mmol) was added, and the reaction mixture was stirred for 1 h until LCMS indicated that the reaction was completed. 2-Amino-1-cyclopropylethanol (609 mg, 9.96 mmol) was added, and the reaction mixture was stirred overnight. The reaction mixture was poured into ice water, and extracted with ethyl acetate. The organic layer was washed with water for three times, dried and concentrated. The residue was purified through preparative TLC to obtain methyl 3-((3*S*)-7-bromo-2-((2-cyclopropyl-2-hydroxyethyl)amino)-5-(pyridin-2-yl)-3H-benzo[e][1,4]diazepin-3-yl)propanoate (compound 5e, 400 mg, yield: 33.3%).
MS m/z (ESI): 485 [M+H]⁺.

### Step 3. Preparation of (S)-methyl 3-(8-bromo-1-cyclopropyl-6-(pyridin-2-yl)-4H-benzo[f]imidazo[1,2-a][1,4]diazepin-4-yl)propanoate (compound 5s)

Methyl 3-((3*S*)-7-Bromo-2-((2-cyclopropyl-2-hydroxyethyl)amino)-5-(pyridin-2-yl)-3H-benzo[e][1,4]diazepin-3-yl)propanoate (compound 5e, 400 mg, 0.82 mmol) was dissolved in CH₃CN (5 mL), and PDC (615.6 mg, 1.64mmol) and a little silica gel were added. The mixture was heated to 38 °C, and stirred for 1 h until LCMS indicated that the reaction was completed. The reaction mixture was concentrated, and the residue was purified through preparative TLC to obtain (*S*)-methyl 3-(8-bromo-1-cyclopropyl-6-(pyridin-2-yl)-4H-benzo[f]imidazo[1,2-a][1,4]diazepin-4-yl)propanoate (compound 5s, 50 mg, yield: 13.1%).
MS m/z (ESI): 465 [M+H]⁺.
¹H NMR (400 MHz, DMSO-*d6*) δ: 8.53(d, *J=* 4 Hz, 1H), 8.09-8.07 (*d, J=* 8.0 Hz, 1H), 7.97-7.85 (m, 3H), 7.61(s, 1H), 7.51-7.48 (m, 1H), 6.76 (s, 1H), 4.05-4.08 (m, 1H), 3.61 (s, 3H), 2.71-2.49 (m, 4H), 1.02-1.00 (m, 1H), 0.81-0.78(m, 1H), 0.72-0.69 (m, 1H), 0.55-0.53(m, 1H).

### Example 6. Preparation of (S)-methyl 3-(8-bromo-1-deuteromethyl-6-(pyridin-2-yl)-4H-benzo[f]imidazo[1,2-a][1,4]diazepin-4-yl)propanoate (compound 6s)

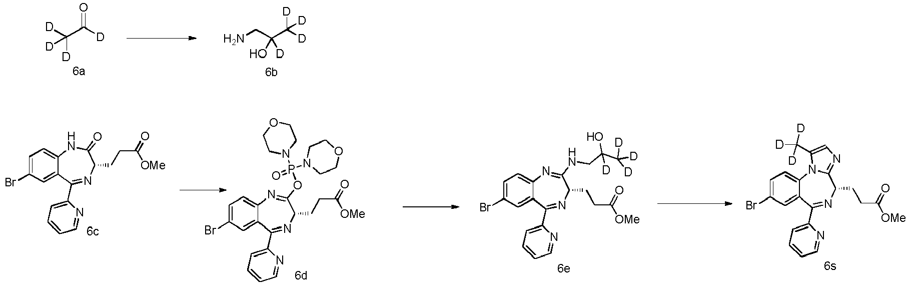

### Step 1. Preparation of 1-amino-2,3,3,3-tetradeuteropropan-2-ol (compound 6b)

To a 500 mL three-necked flask, TMSCN (12.62 g, 0.127 moL), DCM (100 mL) and ZnI₂ (10 mg) were added. The reaction mixture was cooled to at 0 °C under stirring, and deuteroacetaldehyde (compound 6a, 5.0 g, 0.106 moL) was added dropwise. The reaction mixture was stirred at room temperature for 3 h, and then concentrated to dryness. The residue was dissolved in THF (200 mL), and then LAH (4.85 g, 0.127 mol) was added portionwise in an ice bath. The reaction mixture was allowed to react at room temperatue for 3 h. Na₂SO₄·10H₂O (10 g) was added portionwise in an ice bath. The reaction mixture was stirred overnight. The reaction mixture was filtered and concentrated to obtain 1-amino-2,3,3,3-tetradeuteropropan-2-ol (compound 6b, 1.9 g, yield: 22.6%).

### Step 2. Preparation of methyl 3-((3S)-7-bromo-2-((2-hydroxy-2,3,3,3-tetradeuteropropyl)amino)-5-(pyridin-2-yl)-3H-benzo[e][1,4]diazepin-3-yl)propanoate (compound 6e)

(*S*)-Methyl 3-(7-bromo-2-oxo-5-(pyridin-2-yl)-2,3-dihydro-1H-benzo[e][1,4]diazepin-3-yl)propanoate (compound 6c, 1 g, 2.49 mmol) was dissolved in dried THF (10 mL). The reaction mixture was cooled to at 0 °C, and NaH (149.2 mg, 3.73 mmol) was added. The mixture was stirred for 30 min. Morpholinyl chlorophosphate (1.27 g, 4.98 mmol) was added. The reaction mixture was stirred for 1 h until LCMS indicated that the reaction was completed. 1-Amino-2,3,3,3-tetradeuteropropan-2-ol (compound 6b, 609 mg, 9.96 mmol) was added, and the mixture was stirred overnight. The reaction mixture was poured into ice water, and extracted with ethyl acetate. The organic layer was washed with water for three times, dried and concentrated. The residue was purified through preparative TLC to obtain methyl 3-((35)-7-bromo-2-((2-hydroxy-2,3,3,3-tetradeuteropropyl)amino)-5-(pyridin-2-yl)-3H-benzo[e][1,4]diazepin-3-yl)propanoate (compound 6e, 784 mg, yield: 68.2%).
MS m/z (ESI): 463 [M+H]⁺.

### Step 3. Preparation of (S)-methyl 3-(8-bromo-1-deuteromethyl-6-(pyridin-2-yl)-4H-benzo[f]imidazo[1,2-a][1,4]diazepin-4-yl)propanoate (compound 6s)

Methyl 3-((3*S*)-7-bromo-2-((2-hydroxy-2,3,3,3-tetradeuteropropyl)amino)-5-(pyridin-2-yl)-3H-benzo[e][1,4]diazepin-3-yl)propanoate (compound 6e, 684 mg, 1.48 mmol) was dissolved in CH₃CN (5 mL), and PDC (1.13 g, 2.96 mmol) and a little silica gel were added. The mixture was heated to 38 °C, and stirred for 1 h until LCMS indicated that the reaction was completed. The reaction mixture was concentrated, and the residue was purified through preparative TLC to obtain (S)-methyl 3-(8-bromo-1-deuteromethyl-6-(pyridin-2-yl)-4H-benzo[f]imidazo[1,2-a][1,4]diazepin-4-yl)propanoate (compound 6s, 25 mg, yield: 3.8%).
MS m/z (ESI): 442 [M+H]⁺.
¹HNMR (400 MHz, DMSO-*d6*) δ: 8.47(d, *J* = 4 Hz,1H), 8.10-8.08 (d, *J =* 4 Hz,1H), 7.96-7.94 (m, 1H), 7.89-7.87(m, 1H), 7.67- 7.61 (m, 2H), 7.51-7.78 (m, 1H), 6.81 (s, 1H), 4.07-4.04 (m, 1H), 3.61 (s, 3H), 2.70-2.50 (m, 4H).

### Example 7. Preparation of methyl 3-(8-chloro-6-(2-fluorophenyl)-4H-benzo[f][1,2,4]triazolo[4,3-a][1,4]diazepin-4-yl)propanoate (compound 7)

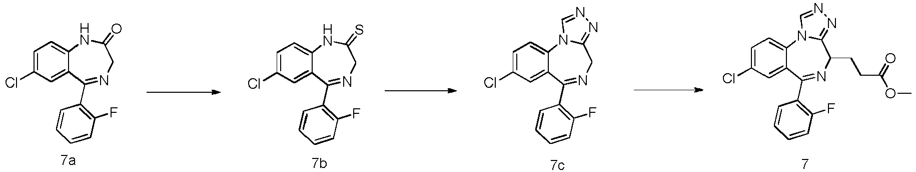

### Step 1. Preparation of 7-chloro-5-(2-fluorophenyl)-1H-benzo[e][1,4]diazepin-2(3H)-thione (compound 7b)

7-Chloro-5-(2-fluorophenyl)-1H-benzo[e][1,4]diazepin-2(3H)-one (compound 7a, 5.0 g, 17.3 mmol) was dissolved in THF (150 mL), and P₂S₅ (5.77 g, 6.55 mmol) was added. The mixture was heated to 78 °C, and allowed to react for 2 h. The reaction mixture was filtered, and washed for three times. Water was added to the filtrate. The mixture was extracted with ethyl acetate for three times. The organic layer was dried and concentrated. The residue was purified through silica gel column chromatography to obtain 7-chloro-5-(2-fluorophenyl)-1H-benzo[e][1,4]diazepin-2(3H)-thione (compound 7b, 4.2 g, yield: 79%).
MS m/z (ESI): 305 [M+H]⁺.

### Step 2. Preparation of 8-chloro-6-(2-fluorophenyl)-4H-benzo[f][1,2,4]triazolo [4,3-a][1,4]diazepine (compound 7c)

7-Chloro-5-(2-fluorophenyl)-1H-benzo[e][1,4]diazepin-2(3H)-thione (compound 7b, 400 mg, 1.31 mmol) was dissolved in dioxane (15 mL), and formic hydrazide (393 mg, 6.55 mmol) and Hg(OAc)₂ (513 mg, 1.97 mmol) were added. The mixture was heated to 100 °C, and allowed to react for 5 h. After cooling, the reaction mixture was poured into ice water, and extracted with ethyl acetate. The organic layer was washed with water for five times, dried and concentrated to obtain 8-chloro-6-(2-fluorophenyl)-4H-benzo[f][1,2,4]triazolo [4,3-a][1,4]diazepine (compound 7c, 280 mg, yield: 68.5%).
MS m/z (ESI): 313 [M+H]⁺.

### Step 3. Preparation of methyl 3-(8-chloro-6-(2-fluorophenyl)-4H-benzo[f][1,2,4]triazolo[4,3-a][1,4]diazepin-4-yl)propanoate (compound 7)

8-Chloro-6-(2-fluorophenyl)-4H-benzo[f][1,2,4]triazolo[4,3-a][1,4]diazepine (compound 7c, 187 mg, 0.60 mmol) and methyl acrylate (771 mg, 9 mmol) were dissolved in dried THF (2 mL). The mixture was cooled to -15 °C, and potassium *tert*-butoxide (134 mg, 1.2 mmol) was added portionwise. The reaction mixture was stirred for 1 h until LCMS indicated that the reaction was completed. The reaction mixture was concentrated, and the residue was purified through preparative TLC to obtain methyl 3-(8-chloro-6-(2-fluorophenyl)-4H-benzo[f][1,2,4]triazolo[4,3-a][1,4]diazepin-4-yl)propanoate (compound 7, 25 mg, yield: 10.5%).
MS m/z (ESI): 399 [M+H]⁺.
¹HNMR (400 MHz, CDCl₃) δ: 9.22(s, 1H), 7.96-7.89 (m, 2H), 7.63-7.54 (m, 2H), 7.36-7.36(m, 2H), 7.25-7.20 (m, 1H), 4.33 (s, 1H), 3.61(s, 3H), 2.76-2.50 (m, 4H).

### Example 8. Preparation of (S)-methyl 3-(8-bromo-1-cyclopropyl-6-(pyridin-2-yl)-4H-benzo[f][1,2,4]triazolo[4,3-a][1,4]diazepin-4-yl)propanoate (compound 8s)

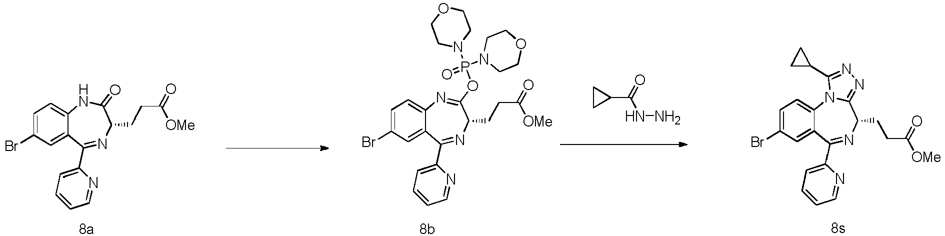

### Step 1. Preparation of (S)-methyl 3-(7-bromo-2-((dimorpholinophosphoryl)oxy)-5-(pyridin-2-yl)-3H-benzo[e][1,4]diazepin-3-yl)propanoate (compound 8b)

(*S*)-Methyl 3-(7-bromo-2-oxo-5-(pyridin-2-yl)-2,3-dihydro-1H-benzo[e][1,4]diazepin-3-yl)propanoate (compound 8a, 1 g, 2.49 mmol) was dissolved in dried THF (10 mL). The mixture was cooled to 0 °C, and NaH (149.2 mg, 3.73 mmol) was added. The mixture was stirred for 30 min. Morpholinyl chlorophosphate (1.27 g, 4.98 mmol) was added, and the reaction mixture was stirred for 1 h until LCMS indicated that the reaction was completed. Water was added, and the mixture was extracted with ethyl acetate for three times. The organic layer was dried and concentrated to obtain crude product. The crude product was used directly in the next step without further purification.

### Step 2. Preparation of (S)-methyl 3-(8-bromo-1-cyclopropyl-6-(pyridin-2-yl)-4H-benzo[f][1,2,4]triazolo[4,3-a][1,4]diazepin-4-yl)propanoate (compound 8s)

(*S*)-methyl 3-(7-bromo-2-((dimorpholinophosphoryl)oxy)-5-(pyridin-2-yl)-3H-benzo[e][1,4]diazepin-3-yl)propanoate (compound 8b) was dissolved in dioxane, and cyclopropanecarbohydrazide (996 mg, 9.96 mmol) was added. The mixture was heated to 100 °C, and stirred for 5 h. The reaction mixture was poured into ice water, and extracted with ethyl acetate. The organic layer was washed with water for three times, dried and concentrated. The residue was purified through preparative TLC to obtain (S)-methyl 3-(8-bromo-1-cyclopropyl-6-(pyridin-2-yl)-4H-benzo[f][1,2,4]triazolo[4,3-a][1,4]diazepin-4-yl)propanoate (compound 8s, 100 mg, yield: 8.7%).
MS m/z (ESI): 446 [M+H]⁺.
¹HNMR (400 MHz, DMSO-*d6*) δ: 8.55(d, *J* = 5.2 Hz, 1H), 8.09 (d, *J* = 7.6, 1H), 7.99-7.94 (m, 2H), 7.85 (d, *J* = 8.8 Hz, 1H), 7.65 (d, *J* = 2 Hz, 1H), 7.52-7.49 (m, 1H), 4.30-4.27 (m, 1H), 3.62 (s, 3H), 2.75-2.54 (m, 4H), 1.26-1.22 (m, 1H), 1.18-1.15 (m, 1H), 1.09-1.05 (m, 1H), 1.00-0.96 (m, 2H).

### Example 9. Preparation of (S)-methyl 3-(8-bromo-6-(pyridin-2-yl)-4H-benzo[f][1,2,4]triazolo[4,3-a][1,4]diazepin-4-yl)propanoate (compound 9s)

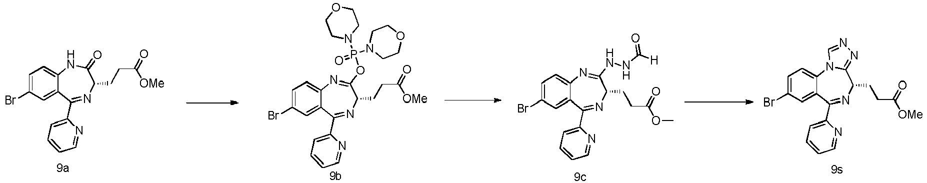

### Step 1. Preparation of (S)-methyl 3-(7-bromo-2-((dimorpholinophosphoryl)oxy)-5-(pyridin-2-yl)-3H-benzo[e][1,4]diazepin-3-yl)propanoate (compound 9b)

(*S*)-Methyl 3-(7-bromo-2-oxo-5-(pyridin-2-yl)-2,3-dihydro-1H-benzo[e][1,4]diazepin-3-yl)propanoate (compound 9a, 1 g, 2.49 mmol) was dissolved in dried THF (10 mL). The mixture was cooled to 0 °C, and NaH (149.2 mg, 3.73 mmol) was added. The mixture was stirred for 30 min. Morpholinyl chlorophosphate (1.27 g, 4.98 mmol) was added, and the reaction mixture was stirred for 1 h until LCMS indicated that the reaction was completed. Water was added, and the mixture was extracted with ethyl acetate for three times. The organic layer was dried and concentrated to obtain a crude product. The crude product was used in the next step directly without further purification.

### Step 2. Preparation of (S)-methyl 3-(8-bromo-6-(pyridin-2-yl)-4H-benzo[f][1,2,4]triazolo[4,3-a][1,4]diazepin-4-yl)propanoate (compound 9s)

(*S*)-Methyl 3-(7-bromo-2-((dimorpholinophosphoryl)oxy)-5-(pyridin-2-yl)-3H-benzo[e][1,4]diazepin-3-yl)propanoate (compound 9b) was dissolved in dioxane, and formhydrazide (597.6 mg, 9.96 mmol) was added. The mixture was heated to 100 °C, and stirred for 5 h. The reaction mixture was poured into ice water, and extracted with ethyl acetate. The organic layer was washed with water, dried and concentrated. The residue was purified through preparative TLC to obtain (S)-methyl 3-(8-bromo-6-(pyridin-2-yl)-4H-benzo[f][1,2,4]triazolo[4,3-a][1,4]diazepin-4-yl)propanoate (compound 9s, 70 mg, yield: 6.6%).
MS m/z (ESI): 426 [M+H]⁺.
¹HNMR (400 MHz, DMSO-*d6*) δ: 9.233 (s, 1H), 8.51(d, *J* = 4.0 Hz, 1H), 8.08 (d, *J* = 7.6 Hz, 1H), 8.01-7.94 (m, 2H), 7.81(d, *J* = 8.8 Hz, 1H), 7.63 (s, 1H), 7.52-7.49 (m, 1H), 4.42-4.39 (m, 1H), 3.63 (s, 3H), 2.82-2.50 (m, 4H).

### Example 10. Preparation of (S)-methyl 3-(8-bromo-1-(methoxymethyl)-6-(pyridin-2-yl)-4H-benzo[f]imidazo[1,2-a][1,4]diazepin-4-yl)propanoate (compound 10s)

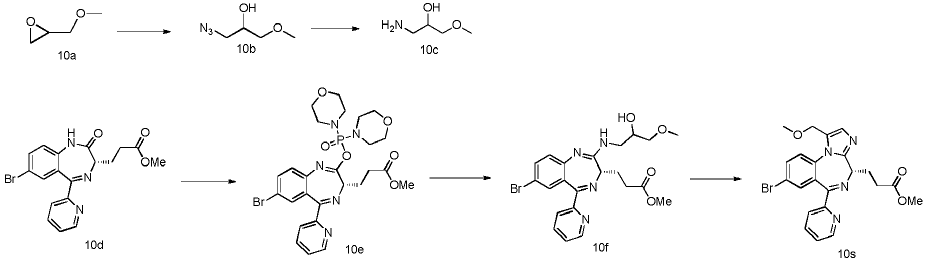

### Step 1. Preparation of 1-azido-3-methoxypropan-2-ol (compound 10b)

2-(Methoxymethyl)oxirane (compound 10a, 2.5 g, 28.4 mmol) was dissolved in DMF, and NaN₃ (5.54 g, 85.1 mmol), NH₄Cl (6.07 g, 113.6 mmol), and H₂O (0.8 mL) were added. The mixture was heated to 80 °C, and stirred for 3 h. The reaction mixture was poured into ice water and extracted with ethyl acetate for three times. The organic layer was washed with water for three times, dried and concentrated. The obtained product 1-azido-3-methoxypropan-2-ol (compound 10b) was used in the next step directly without further purification.

### Step 2. Preparation of 1-amino-3-methoxypropan-2-ol (compound 10c)

1-Azido-3-methoxypropan-2-ol (compound 10b) was dissolved in MeOH. 10% Pd/C (200 mg) was added, and hydrogen was introduced. The resulting mixture was stirred at room temperature for 5 h. The reaction mixture was filtered and concentrated to obtain 1-amino-3-methoxypropan-2-ol (compound 10c, 1.5 g, yield: 50%).

### Step 3. Preparation of methyl 3-((3S)-7-bromo-2-((2-hydroxy-3- methoxypropyl) amino)-5-(pyridin-2-yl)-3H-benzo[e][1,4]diazepin-3-yl)propanoate (compound 10f)

(*S*)-Methyl 3-(7-bromo-2-oxo-5-(pyridin-2-yl)-2,3-dihydro-1H-benzo[e] [1,4]diazepin-3-yl)propanoate (compound 10d, 804 mg, 2 mmol) was dissolved in dried THF (10 mL). The mixture was cooled to 0 °C, and NaH (120 mg, 3 mmol) was added. The mixture was stirred for 30 min. Morpholinyl chlorophosphate (1.024 g, 4 mmol) was added, and the reaction mixture was stirred for 3 h until LCMS indicated that the reaction was completed. 1-Amino-3-methoxypropan-2-ol (compound 10c, 630.66 mg, 6 mmol) was added, and the mixture was stirred overnight. The reaction mixture was poured into ice water, and extracted with ethyl acetate. The organic layer was washed with water for three times, dried and concentrated. The residue was purified through preparative TLC to obtain methyl 3-((3S)-7-bromo-2-((2-hydroxy-3-methoxypropyl)amino)-5-(pyridin-2-yl)-3H-benzo[e] [1,4]diazepin-3-yl)propanoate (compound 10f, 410 mg, yield: 42%).
MS m/z (ESI):489 [M+H]⁺.

### Step 4. Preparation of (S)-methyl 3-(8-bromo-1-(methoxymethyl)-6-(pyridin-2-yl)-4H-benzo[f]imidazo[1,2-a][1,4]diazepin-4-yl)propanoate (compound 10s)

Methyl 3-((3S)-7-bromo-2-((2-hydroxy-3-methoxypropyl)amino)-5-(pyridin-2-yl)-3H-benzo[e][1,4]diazepin-3-yl)propanoate (compound 10f, 410 mg, 0.84 mmol) was dissolved in butanone (15 mL), and Dess-Martin periodinane (713 mg, 1.68 mmol) was added. The mixture was heated to 78 °C and stirred for 1 h until LCMS indicated that the reaction was completed. The reaction mixture was filtered and concentrated, and the residue was purified through preparative TLC to obtain (S)-methyl 3-(8-bromo-1-(methoxymethyl)-6-(pyridin-2-yl)-4H-benzo[f]imidazo[1,2-a][1,4]diazepin-4-yl)propanoate (compound 10s, 100 mg, yield: 25%).
MS m/z (ESI): 470 [M+H]⁺
¹HNMR (400 MHz, DMSO-*d6*) δ: 8.53(t, *J* = 4Hz, 1H), 8.08 (d, *J=* 8 Hz, 1H), 7.96-7.92 (m, 2H), 7.86(d, *J* = 8.8 Hz, 1H), 7.51-7.47 (m, 2H), 7.14 (m, 1H), 4.53-4.50 (m, 1H), 4.30-4.26 (m, 1H), 4.14-4.10 (m, 1H), 3.61 (s, 3H), 3.25 (s, 3H), 2.75-2.50 (m, 4H).

### Example 11. Preparation of (S)-methyl 3-(8-bromo-1-deuteromethyl-6-(pyridin-2-yl)-4H-benzo[f][1,2,4]triazolo[4,3-a][1,4]diazepin-4-yl)propanoate (compound 11s)

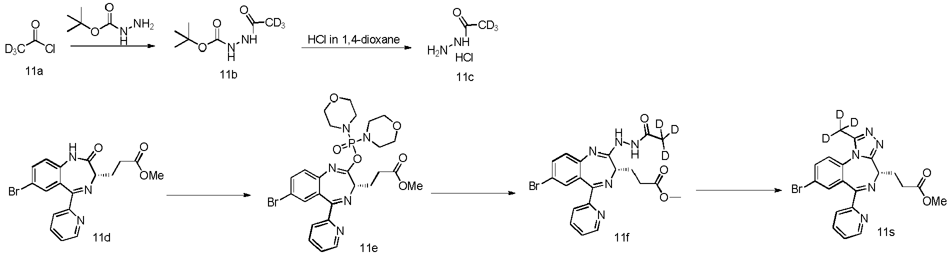

### Step 1. Preparation of tert-butyl (2-deuteroacethydrazide)carbonate (compound 11b)

*Tert*-butyl hydrazinocarboxylate (compound 11a, 1.5 g, 11.3 mmol) was dissolved in THF (15 mL), and Et₃N (1.73 g, 17.0 mmol) was added. *d₃*-Acetyl chloride (0.89 mL, 12.43 mmol) was added dropwise in an ice bath. The mixture was stirred for 8 h. The reaction mixture was poured into water, and extracted with ethyl acetate. The organic layer was washed with water, dried and concentrated to obtain *tert-butyl* (2-deuteroacethydrazide)carbonate (compound 11b), which was used in the next step directly without further purification.

### Step 2. Preparation of d₃-acethydrazide hydrochloride (compound 11c)

*Tert*-butyl (2-deuteroacethydrazide)carbonate (compound 11b) obtained in Step 1 was dissolved in an HCl solution of dioxane (20 mL). The mixture was stirred at room temperature for 5 h, and filtered. The precipitate was washed with ethyl acetate to obtain *d₃*-acethydrazide hydrochloride (compound 11c, 1.0 g, yield 78%).

### Step 3. Preparation of (S)-methyl 3-(7-bromo-2-((dimorpholinophosphoryl)oxy)-5-(pyridin-2-yl)-3H-benzo[e][1,4]diazepin-3-yl)propanoate (compound 11e)

(*S*)-Methyl 3-(7-bromo-2-oxo-5-(pyridin-2-yl) -2,3-dihydro-1H-benzo[e][1,4]diazepin-3-yl)propanoate (compound 11d, 804 mg, 2.0 mmol) was dissolved in dried THF (10 mL). The mixture was cooled to 0 °C, and NaH (120 mg, 3 mmol) was added. The mixture was stirred for 30 min. Morpholinyl chlorophosphate (1.024 g, 4 mmol) was added, and the mixture was stirred for 3 h until LCMS indicated that the reaction was completed. Water was added and the mixture was extracted with ethyl acetate. The organic layer was dried and concentrated to obtain a crude product. The crude product was used in the next step directly without further purification.

### Step 4. Preparation of (S)-methyl 3-(8-bromo-1-deuteromethyl-6-(pyridin-2-yl)-4H-benzo[f][1,2,4]triazolo[4,3-a][1,4]diazepin-4-yl)propanoate (compound 11s)

(*S*)-Methyl 3-(7-bromo-2-((dimorpholinophosphoryl)oxy)-5-(pyridin-2-yl)-3H-benzo[e][1,4]diazepin-3-yl)propanoate (compound 11e) was dissolved in dioxane, and *d₃*-acethydrazide hydrochloride (compound 11c, 565 mg, 5 mmol) was added. The mixture was heated to 100 °C and stirred for 5 h. The reaction mixture was poured into ice water, and extracted with ethyl acetate. The organic layer was washed with water for three times, dried and concentrated. The residue was purified through preparative TLC to obtain (*S*)-methyl 3-(8-bromo-1-deuteromethyl-6-(pyridin-2-yl)-4H-benzo[f][1,2,4]triazolo[4,3-a][1,4]diazepin-4-yl)propanoate (compound 11s, 100 mg, yield: 11.3%).
MS m/z (ESI): 443 [M+H]⁺.
¹HNMR (400 MHz, DMSO-*d6*) δ: 8.55(d, *J* = 4.0 Hz, 1H), 8.09 (d, *J* = 8.0 Hz, 1H), 7.98-7.93 (m, 2 H), 7.78(d, *J* = 8.8 Hz, 1H), 7.65 (d, *J* = 2 Hz, 1H), 7.53-7.49 (m, 1H), 4.31-4.28 (m, 1H), 3.62 (s, 3H), 2.78-2.50 (m, 4H).

### Example 12. Preparation of methyl 3-(8-chloro-1-cyclopropyl-6-(2-fluorophenyl)-4H-benzo[f][1,2,4]triazolo[4,3-a][1,4]diazepin-4-yl)propanoate (compound 12)

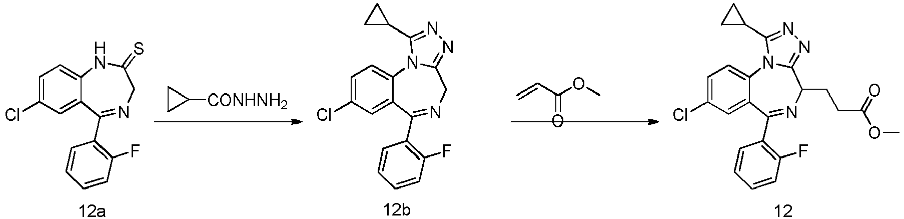

### Step 1. Preparation of 8-chloro-1-cyclopropyl-6-(2-fluorophenyl)-4H-benzo[f][1,2,4]triazolo[4,3-a][1,4]diazepine (compound 12b)

7-Chloro-5-(2-fluorophenyl)-1H-benzo[e][1,4]diazepin-2(3H)-thione (compound 12a, 400 mg, 1.31 mmol, ref: Example 7, Step 1 for systhesis) was dissolved in dioxane (15 mL), and cyclopropanecarbohydrazide (394 mg, 3.93 mmol) and Hg(OAc)₂(513 mg, 1.97 mmol) were added. The mixture was heated to 100°C and allowed to react for 5 h. After cooling, the mixture was poured into ice water, and extracted with ethyl acetate. The organic layer was washed with water for five times, dried and concentrated. The residue was purified through preparative TLC to obtain 8-chloro-1-cyclopropyl-6-(2-fluorophenyl)-4H-benzo[f][1,2,4]triazolo[4,3-a][1,4]diazepine (compound 12b, 280 mg, yield: 68.5%).
MS m/z (ESI): 353 [M+H]⁺.

### Step 2. Preparation of methyl 3-(8-chloro-1-cyclopropyl-6-(2-fluorophenyl)-4H-benzo[f][1,2,4]triazolo[4,3-a][1,4]diazepin-4-yl)propanoate (compound 12)

8-Chloro-1-cyclopropyl-6-(2-fluorophenyl)-4H-benzo[f][1,2,4]triazolo[4,3-a][1,4]diazep ine (compound 12b, 150 mg, 0.43 mmol) and methyl acrylate (366 mg, 4.3 mmol) were dissolved in dried THF (2 mL). The mixture was cooled to -15 °C, and potassium *tert*-butoxide (95 mg, 0.86 mmol) was added portionwise. The mixture was stirred for 1 h until LCMS indicated that the reaction was completed. The reaction mixture was concentrated. The residue was purified through reverse HPLC to obtain methyl 3-(8-chloro-1-cyclopropyl-6-(2-fluorophenyl)-4H-benzo[f][1,2,4]triazolo[4,3-a][1,4]diazepin -4-yl)propanoate (compound 12, 31 mg, yield: 16.6%).
MS m/z (ESI): 439 [M+H]⁺.
¹HNMR (400 MHz, CDCl₃) δ: 7.99 (d, *J* = 8.8 Hz, 1H), 7.87 (dd, *J* = 8.8, 6.4 Hz, 1H),7.66-7.54 (m, 2H), 7.38-7.32 (m, 2H), 7.25-7.20 (m,1H), 4.25-4.22 (m, 1H), 3.61 (s, 3H), 2.76-2.53 (m, 4H), 2.12-2.08 (m, 1H), 1.17-1.14 (m, 1H), 1.03-0.90 (m, 3H).

### Example 13. Preparation of (S)-methyl 3-(8-bromo-1-ethenyl-6-(pyridin-2-yl)-4H-benzo[f]imidazo[1,2-a][1,4]diazepin-4-yl)propanoate (compound 13s)

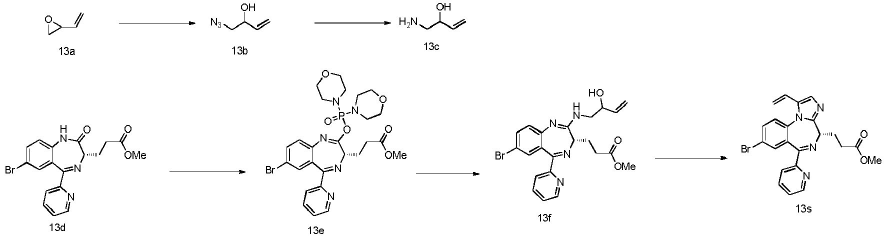

### Step 1. Preparation of 1-azido-but-3-en-2-ol (compound 13b)

2-Vinyloxirane (compound 13a, 1 g, 14.3 mmol) was dissolved in DMF, and NaN₃ (2.8 g, 42.8 mmol), NH₄Cl (3.05 g, 57.2 mmol) and H₂O (0.8 mL) were added. The mixture was heated to 80 °C and stirred for 3 h. The reaction mixture was poured into ice water, and extracted with ethyl acetate for three times. The organic layer was washed with water for three times, dried and concentrated to obtain 1-azido-but-3-en-2-ol (compound 13b). The crude product was used in the next step directly without further purification.

### Step 2. Preparation of 1-amino-but-3-en-2-ol (compound 13c)

1-Azido-but-3-en-2-ol (compound 13b) obtained in Step 1 was dissolved in THF, and Ph₃P (7.5 g, 28.6 mmol) and H₂O (4 mL) were added. The mixture was stirred at room temperature for 18 h, filtered and concentrated. The residue was dissolved in diethyl ether to remove insoluble substances. The diethyl ether layer was concentrated to obtain 1-amino-but-3-en-2-ol (compound 13c, 200 mg, yield 17%).

### Step 3. Preparation of methyl 3-((3S)-7-bromo-2-((2-hydroxybut-3-enyl)amino)-5-(pyridin-2-yl)-3H-benzo[e][1,4]diazepin-3-yl)propanoate (compound 13f)

(*S*)-Methyl 3-(7-bromo-2-oxo-5-(pyridin-2-yl)-2,3-dihydro-1H-benzo[e][1,4]diazepin-3-yl)propanoate (compound 13d, 804 mg, 2 mmol) was in dried THF (10 mL). The mixture was cooled to 0 °C, and NaH (120 mg, 3 mmol) was added. The mixture was stirred for 30 min. Morpholinyl chlorophosphate (1.024 g, 4 mmol) was added, and the mixture was stirred for 3 h until LCMS indicated that the reaction was completed. 1-Amino-but-3-en-2-ol (compound 13c, 200 mg, 2.3 mmol) was added, and the mixture was stirred overnight. The reaction mixture was poured into ice water, and extracted with ethyl acetate. The organic layer was washed with water for three times, dried and concentrated. The residue was purified through preparative TLC to obtain methyl 3-((3*S*)-7-bromo-2-((2-hydroxybut-3-enyl)amino)-5-(pyridin-2-yl)-3H-benzo[e][1,4]diazepin-3-yl)propanoate (compound 13f, 200 mg, yield. 21%).
MS m/z (ESI): 471 [M+H]⁺.

### Step 4. Preparation of (S)-methyl 3-(8-bromo-1-ethenyl-6-(pyridin-2-yl)-4H-benzo[f]imidazo[1,2-a][1,4]diazepin-4-yl)propanoate (compound 13s)

Methyl 3-((3*S*)-7-Bromo-2-((2-hydroxybut-3-enyl)amino)-5-(pyridin-2-yl)-3H-benzo [e][1,4]diazepin-3-yl)propanoate (compound 13f, 200 mg, 0.43mmol) was dissolved in butanone (10 mL), and was added Dess-Martin reagent (234 mg, 0.55 mmol) was added. The mixture was heated to 43 °C, and stirred for 3 h until LCMS indicated that the reaction was completed. The reaction mixture was filtered and concentrated, and the residue was purified through preparative TLC to obtain (*S*)-methyl 3-(8-bromo-1-ethenyl-6-(pyridin-2-yl)-4H-benzo[f]imidazo[1,2-a][1,4]diazepin-4-yl) propanoate (compound 13s, 20 mg, yield: 10%).
MS m/z (ESI): 451 [M+H]⁺.
¹HNMR (400 MHz, DMSO-*d6*) δ: 8.50(s, 1H), 8.07 (d, *J=* 7.6 Hz, 1H), 7.96-7.90 (m, 2H), 7.86(s, 1H), 7.67 (d, *J* = 8.4 Hz, 1H), 7.58 (s, 1H), 7.49-7.47 (m, 1H), 6.62-6.55 (m, 1H), 5.77 (d, *J* = 17.2Hz,1H), 4.15-4.14 (m, 1H), 3.61 (s, 3H), 2.76-2.62 (m, 4H).

### Example 14. Preparation of (S)-methyl 3-(8-chloro-1-cyclopropyl-6-(pyridin-2-yl)-4H-benzo[f][1,2,4]triazolo[4,3-a][1,4]diazepin-4-yl)propanoate (compound 14s)

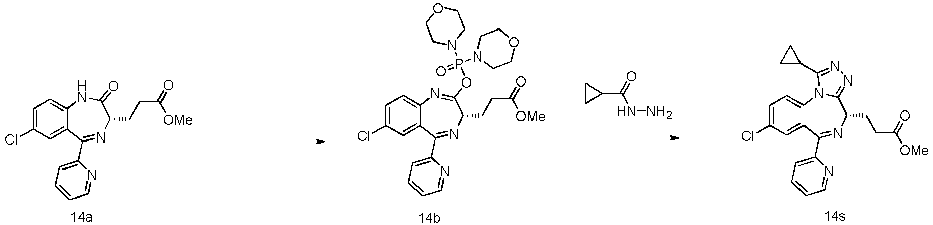

### Step 1. Preparation of (S)-methyl 3-(7-chloro-2-((dimorpholinophosphoryl)oxy)-5-(pyridin-2-yl)-3H-benzo[e][1,4]diazepin-3-yl)propanoate (compound 14b)

(*S*)-Methyl 3-(7-chloro-2-oxo-5-(pyridin-2-yl)-2,3-dihydro-1H-benzo[e][1,4]diazepin-3-yl)propanoate (compound 14a, 1 g, 2.79 mmol) was dissolved in dried THF (10 mL). The mixture was cooled to 0 °C, and NaH (167 mg, 4.19 mmol) was added. The mixture was stirred for 30 min. Morpholinyl chlorophosphate (1.42 g, 5.58 mmol) was added, and the mixture was stirred for 1 h until LCMS indicated that the reaction was completed. Water was added, and the mixture was extracted with ethyl acetate for three times. The organic layer was dried and concentrated to obtain a crude product. The crude product was used in the next step directly without further purification.
MS m/z (ESI): 576 [M+H]⁺.

### Step 2. Preparation of (S)-methyl 3-(8-chloro-1-cyclopropyl-6-(pyridin-2-yl)-4H-benzo[f][1,2,4]triazolo[4,3-a][1,4]diazepin-4-yl)propanoate (compound 14s)

(*S*)-Methyl 3-(7-chloro-2-((dimorpholinophosphoryl)oxy)-5-(pyridin-2-yl)-3H-benzo[e][1,4]diazepin-3-yl)propanoate (compound 14b) obtained in Step 1 was dissolved in dioxane, and cyclopropanecarbohydrazide (996 mg, 9.96 mmol) was added. The mixture was heated to 100 °C and stirred for 5 h. The reaction mixture was poured into ice water, and extracted with ethyl acetate. The organic layer was washed with water for three times, dried and concentrated. The residue was purified with preparative TLC to obtain (S)-methyl 3-(8-chloro-1-cyclopropyl-6-(pyridin-2-yl)-4H-benzo[f][1,2,4]triazolo[4,3-a][1,4]diazepin-4-yl)propanoate (compound 14s, 102 mg, yield. 8.7%).
MS m/z (ESI):422 [M+H]⁺.
¹HNMR (400 MHz, DMSO-*d6*) δ: 8.56-8.55 (d, *J* = 4.0 Hz, 1H), 8.11-8.10 (d, *J* = 4.0 Hz, 1H), 7.98-7.85 (m, 3H), 7.55-7.50 (m, 2H), 4.31-4.28 (m, 1H), 3.62 (s, 3H), 2.76-2.50 (m, 4H), 2.08-2.04 (m, 1H), 1.19-0.96 (m, 4H).

### Example 15. Preparation of (S)-methyl 3-(8-bromo-1-ethyl-6-(pyridin-2-yl)-4H-benzo[f]imidazo[1,2-a][1,4]diazepin-4-yl)propanoate (compound 15s)

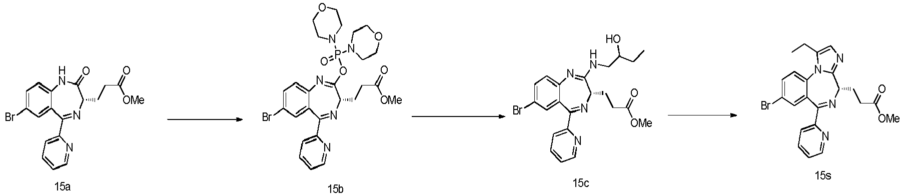

### Step 1. Preparation of methyl 3-((3S)-7-bromo-2-((2-hydroxybutyl)amino)-5-(pyridin-2-yl)-3H-benzo[e][1,4]diazepin-3-yl)propanoate (compound 15c)

(*S*)-Methyl 3-(7-bromo-2-oxo-5-(pyridin-2-yl)-2,3-dihydro-1H-benzo [e][1,4]diazepin-3-yl)propanoate (compound 15a, 1 g, 2.5 mmol) was dissolved in dried THF (10 mL). The mixture was cooled to 0 °C, and NaH (149 mg, 3.75 mmol) was added. The mixture was stirred for 30 min. Morpholinyl chlorophosphate (1.27 g, 5 mmol) was added, and the mixture was stirred for 3 h until LCMS indicated that the reaction was completed. 1-Aminobutan-2-ol (0.88 g, 10 mmol) was added, and the mixture was stirred overnight. The reaction mixture was poured into ice water, and extracted with ethyl acetate. The organic layer was washed with water for three times, dried and concentrated. The residue was purified through preparative TLC to obtain methyl 3-((3*S*)-7-bromo-2-((2-hydroxybutyl)amino)-5-(pyridin-2-yl)-3H-benzo[e][1,4]diazepin-3-yl)propanoate (compound 15c, 500 mg, yield 42%).
MS m/z (ESI):473 [M+H]⁺.

### Step 2. Preparation of (S)-methyl 3-(8-bromo-1-ethyl-6-(pyridin-2-yl)-4H-benzo[f]imidazo[1,2-a][1,4]diazepin-4-yl)propanoate (compound 15s)

Methyl 3-((3*S*)-7-bromo-2-((2-hydroxybutyl)amino)-5-(pyridin-2-yl)-3H-benzo[e][1,4]diazepin-3-yl)propanoate (compound 15c, 500 mg, 1.06 mmol) was dissolved in butanone (15 mL), and Dess-Martin periodinane (672 mg, 1.59 mmol) was added. The reaction mixture was heated to 43 °C, and was stirred for 1 h until LCMS indicated that the reaction was completed. The reaction mixture was filtered and concentrated. The residue was purified through preparative TLC to obtain (S)-methyl 3-(8-bromo-1-ethyl-6-(pyridin-2-yl)-4H-benzo[f]imidazo[1,2-a][1,4]diazepin-4-yl)propanoate (compound 15s, 100 mg, yield. 21%).
MS m/z (ESI): 453 [M+H]⁺.
¹HNMR (400 MHz, DMSO-*d6*) δ: 8.54(d, *J* = 4 Hz, 1H), 8.08 (d, *J* = 8 Hz, 1H), 7.95-7.94 (m, 1H), 7.86(d, *J* = 8.8 Hz, 1H), 7.65 (d, *J* = 8.8 Hz, 1H), 7.60 (s, 1H), 7.50-7.49 (m, 1H), 6.83 (s, 1H), 4.53-4.50 (m, 1H), 3.60 (s, 3H), 2.94-2.88 (m, 1H), 2.71-2.42 (m, 5H), 1.10-1.05 (t, 3H).

### Example 16. Preparation of (S)-methyl 3-(8-bromo-1-(methoxymethyl)-6-(pyridin-2-yl)-4H-benzo[f][1,2,4]triazolo[4,3-a][1,4]diazepin-4-yl)propanoate (compound 16s)

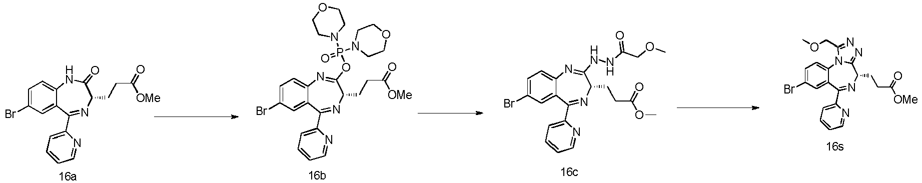

### Step 1. Preparation of (S)-methyl 3-(7-bromo-2-((dimorpholinophosphoryl)oxy)-5-(pyridin-2-yl)-3H-benzo[e][1,4]diazepin-3-yl)propanoate (compound 16b)

(*S*)-Methyl 3-(7-bromo-2-oxo-5-(pyridin-2-yl)-2,3-dihydro-1H-benzo[e][1,4]diazepin-3-yl)propanoate (compound 16a, 1 g, 2.49 mmol) was dissolved in dried THF(10 mL). The mixture was cooled to 0 °C, and NaH (149.2 mg, 3.73 mmol) was added. The mixture was stirred for 30 min. Morpholinyl chlorophosphate (1.27 g, 4.98 mmol) was added, and the reaction mixture was stirred for 1 h until LCMS indicated that the reaction was completed. Water was added, and the mixture was extracted with ethyl acetate. The organic layer was dried and concentrated to obtain a crude product. The crude product was used directly in the next step without further purification.

### Step 2. Preparation of (S)-methyl 3-(8-bromo-1-(methoxymethyl)-6-(pyridin-2-yl)-4H-benzo[f][1,2,4]triazolo[4,3-a][1,4]diazepin-4-yl)propanoate (compound 16s)

(*S*)-Methyl 3-(7-bromo-2-((dimorpholinophosphoryl)oxy)-5-(pyridin-2-yl)-3H-benzo[e] [1,4]diazepin-3-yl)propanoate (compound 16b) was dissolved in dioxane, and methoxyacethydrazide (1.3 g, 12.45 mmol) was added. The mixture was heated to 100 °C and stirred for 20 h. The reaction mixture was poured into ice water and extracted with ethyl acetate. The organic layer was washed with water for three times, dried and concentrated. The residue was purified through preparative TLC to obtain (*S*)-methyl 3-(8-bromo-1-(methoxymethyl)-6-(pyridin-2-yl)-4H-benzo[f][1,2,4]triazolo[4,3-a][1,4]diazep in-4-yl)propanoate (compound 16s, 100 mg, yield: 6.1%).
MS m/z (ESI): 469 [M+H]⁺.
¹HNMR (400 MHz, DMSO-*d6*) δ: 8.53 (d, *J* = 4.0 Hz, 1H), 8.09 (d, *J* = 7.6 Hz, 1H), 8.01-7.94 (m, 2H), 7.87 (d, *J=* 9.2 Hz, 1H), 7.65 (d, *J=* 2.4 Hz, 1H), 7.51-7.48 (m, 1H), 4.76 (d, *J* = 13.2 Hz, 1H), 4.53 (d, *J* = 12.4 Hz, 1H), 4.34-4.33 (m, 1H), 3.62 (s, 3H), 3.26 (s, 3H), 2.72-2.66 (m, 4H).

### Example 17. Preparation of (S)-methyl 3-(8-chloro-6-(2-fluorophenyl)-4H-benzo[f][1,2,4]triazolo[4,3-a][1,4]diazepm-4-yl)propanoate (compound 7s)

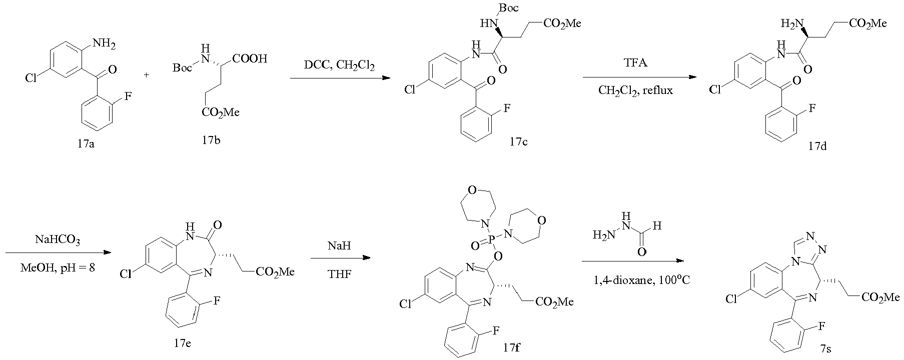

### Step 1. Preparation of (S)-methyl 5-((2-fluorobenzoyl-4-chlorophenyl)amino)-4-((tert-butoxycarbonyl)amino)-5-oxopentanoate (compound 17c)

(2-Amino-5-chlorophenyl)(2'-fluorophenyl)methanone (compound 17a, 20 g, 0.083 mol) and 5-methyl N-*tert*-butoxycarbonyl-L-glutamate (compound 17b, 23 g, 0.088 mol) were dissolved in DCM (300 mL). The mixture was cooled to 0 °C, and DCC (18.2 g, 0.088 mmol) was added. The mixture was stirred for 24 h until LCMS indicated that the reaction was completed. The reaction mixture was poured into ice water, and extracted with ethyl acetate. The organic layer was washed with water for three times, dried and concentrated to obtain crude (S)-methyl 5-((2-fluorobenzoyl-4-chlorophenyl)amino)-4-(*tert*-butoxycarbonyl)amino)-5-oxopentanoate (compound 17c, 50 g), which was used directly in the next step without further purification.

### Step 2. Preparation of (S)-methyl 4-amino-5-((2-fluorobenzoyl-4-chlorophenyl)amino)-5-oxopentanoate (compound 17d)

(*S*)-Methyl 5-((2-fluorobenzoyl-4-chlorophenyl)amino)-4-((*tert*-butoxycarbonyl)amino)-5-oxopentanoate (compound 17c, 50 g) was dissolved in DCM (200 mL), and TFA (100 mL) was added. The mixture was heated to 40 °C and stirred for 2 h until LCMS indicated that the reaction was completed. The reaction mixture was concentrated to obtain crude (S)-methyl 4-amino-5-((2-fluorobenzoyl-4-chlorophenyl)amino)-5-oxopentanoate (compound 17d, 40 g), which was used in the next step directly without further purification.

### Step 3. Preparation of (S)-methyl 3-(7-chloro-2-oxo-5-(2-fluorophenyl)-2,3-dihydro-1H-benzo[e][1,4]diazepin-3-yl)propanoate (compound 17e)

(*S*)-Methyl 4-amino-5-((2-fluorobenzoyl-4-chlorophenyl)amino)-5-oxopentanoate (compound 17d, 40 g) was dissolved in MeOH (500 mL). NaHCO₃ was added to adjust pH to about 10, and the mixture was stirred for 24 h until LCMS indicated that the reaction was completed. The reaction mixture was filtered, and the filtrate was poured into ice water. The resulting mixture was extracted with ethyl acetate. The organic layer was washed with water for three times, dried and concentrated. The residue was purified through silica gel column chromatography to obtain (*S*)-methyl 3-(7-chloro-2-oxo-5-(2-fluorophenyl)-2,3-dihydro-1H-benzo[e][1,4]diazepin-3-yl)propanoate (compound 17e, 22 g, yield 73%).
MS m/z (ESI): 374 [M+H]⁺.

### Step 4. Preparation of (S)-methyl 3-(7-chloro-2-((dimorpholinophosphoryl)oxy)-5-(2-fluorophenyl)-3H-benzo[e][1,4]diazepin-3-yl)propanoate (compound 17f)

(*S*)-Methyl 3-(7-chloro-2-oxo-5-(2-fluorophenyl)-2,3-dihydro-1H-benzo [e][1,4]diazepin-3-yl)propanoate (compound 17e, 6 g, 0.016 mol) was dissolved in dried THF (100 mL). The mixture was cooled to 0 °C, and NaH (963 mg, 0.024 mol) was added. The mixture was stirred for 30 min. Morpholinyl chlorophosphate (8.21 g, 0.032 mmol) was added, and the mixture was stirred for 1 h until LCMS indicated that the reaction was completed. The reaction mixture was poured into ice water and extracted with ethyl acetate. The organic layer was washed with water for three times, dried and concentrated to obtain crude (*S*)-methyl 3-(7-chloro-2-((dimorpholinophosphoryl)oxy)-5-(2-fluorophenyl)-3H-benzo[e][1,4]diazepin-3-yl)propanoate (compound 17f, 12 g), which was used directly in the next step without further purification.

### Step 5. Preparation of (S)-methyl 3-(8-chloro-6-(2-fluorophenyl)-4H-benzo[f][1,2,4]triazolo[4,3-a][1,4]diazepin-4-yl)propanoate (compound 7s)

(*S*)-Methyl 3-(7-chloro-2-((dimorpholinophosphoryl)oxy)-5-(2-fluorophenyl)-3H-benzo[e][1,4]diazepin-3-yl)propanoate (compound 17f, 12.0 g) was dissolved in 1,4-dioxane (150 mL), and formhydrazide (2.89 g, 0.048 mol) was added. The mixture was heated to 100 °C and stirred for 14 h until LCMS indicated that the reaction was completed. The reaction mixture was concentrated, and the residue was purified through preparative HPLC to obtain (*S*)-methyl 3-(8-chloro-6-(2-fluorophenyl)-4H-benzo[f][1,2,4]triazolo[4,3-a][1,4]diazepin-4-yl)propanoa te (compound 7s, 3.0 g, yield: 37.5%).
MS m/z (ESI): 399 [M+H]⁺.
¹HNMR (400 MHz, CDCl₃) δ: 9.29 (s, 1H), 7.95-7.89 (m, 2H), 7.63-7.54 (m, 2H), 7.37-7.32 (m, 2H), 7.25-7.20 (m, 1H), 4.34 (m, 1H), 3.61 (s, 3H), 2.77-2.56 (m, 4H).

### Example 18. Preparation of (S)-methyl 3-(8-chloro-1-cyclopropyl-6-(2-fluorophenyl)-4H-benzo[f][1,2,4]triazolo[4,3-a][1,4]diazepin-4-yl)propanoate (compound 12s)

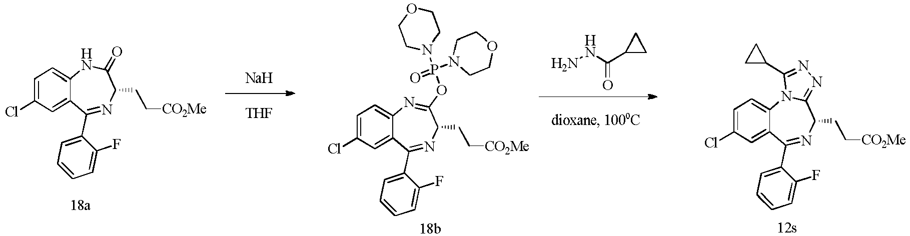

### Step 1. Preparation of (S)-methyl 3-(7-chloro-2-((dimorpholinophosphoryl)oxy)-5-(2-fluorophenyl)-3H-benzo[e][1,4]diazepin-3-yl)propanoate (compound 18b)

(*S*)-Methyl 3-(7-chloro-2-oxo-5-(2-fluorophenyl)-2,3-dihydro-1H-benzo [e][1,4]diazepin-3-yl)propanoate (compound 18a, 6 g, 0.016 mol) was dissolved in dried THF (100 mL). The mixture was cooled to 0 °C, and NaH (963 mg, 0.024 mol) was added. The mixture was stirred for 30 min. Morpholinyl chlorophosphate (8.21 g, 0.032 mmol) was added, and the mixture was stirred for 1 h until LCMS indicated that the reaction was completed. The reaction mixture was poured into ice water and extracted with ethyl acetate. The organic layer was washed with water for three times, dried and concentrated to obtain crude (*S*)-methyl 3-(7-chloro-2-((dimorpholinophosphoryl)oxy)-5-(2-fluorophenyl)-3H-benzo[e][1,4]diazepin-3-yl)propanoate (compound 18b, 12 g), which was used directly in the next step without further purification.

### Step 2. Preparation of (S)-methyl 3-(8-chloro-1-cyclopropyl-6-(2-fluorophenyl)-4H-benzo[f][1,2,4]triazolo[4,3-a][1,4]diazepin-4-yl)propanoate (compound 12s)

(*S*)-Methyl 3-(7-chloro-2-((dimorpholinophosphoryl)oxy)-5-(2-fluorophenyl)-3H-benzo[e][1,4]diazepin-3-yl)propanoate (compound 18b, 13.0 g) was dissolved in 1,4-dioxane (150 mL), and cyclopropanecarbohydrazide (4.81 g, 0.048 mol) was added. The mixture was was heated to 100 °C and stirred for 14 h until LCMS indicated that the reaction was completed. The reaction mixture was concentrated, and the residue was purified through preparative HPLC to obtain (*S*)-methyl 3-(8-chloro-1-cyclopropyl-6-(2-fluorophenyl)-4H-benzo[f][1,2,4]triazolo[4,3-a][1,4]diazepin-4-yl)propanoate (compound 12s, 2.8 g, yield 30%).
MS m/z (ESI): 439 [M+H]⁺.
¹HNMR (400 MHz, CDCl₃) δ: 7.99 (d, *J* = 8.8 Hz, 1H), 7.87 (dd, *J* = 8.8, 6.4 Hz, 1H), 7.66-7.54 (m, 2H), 7.38-7.32 (m, 2H), 7.25-7.20 (m,1H), 4.25-4.22 (m, 1H), 3.61 (s, 3H), 2.76-2.53 (m, 4H), 2.12-2.08 (m, 1H), 1.17-1.14 (m, 1H), 1.03-0.90 (m, 3H).

### Example 19. Preparation of (S)-methyl 3-(8-bromo-1-isopropyl-6-(pyridin-2-yl)-4H-benzo[f]imidazo[1,2-a][1,4]diazepin-4-yl)propanoate (compound 17s)

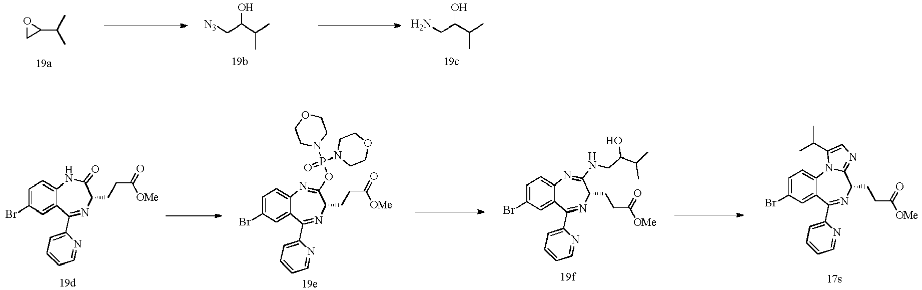

### Step 1. Preparation of 1-azido-3-methylbutan-2-ol (compound 19b)

1,2-Epoxy-3-methylbutane (compound 19a, 2.0 g, 0.023 mol) was dissolved in DMF, and NaN₃(4.53 g, 0.069 mol), NH₄Cl (4.97 g, 0.092 mol) and H₂O (0.8 mL) were added. The mixture was heated to 80°C and stirred for 3 h. The reaction mixture was poured into water, and extracted with ethyl acetate for three times. The organic layer was washed with water for three times, dried and concentrated to obtain crude 1-azido-3-methylbutan-2-ol (compound 19b), which was used in the next step directly without further purification.

### Step 2. Preparation of 1-amino-3-methylbutan-2-ol (compound 19c)

1-Azido-3-methylbutan-2-ol (compound 19b) was dissolved in MeOH, and 10% Pd/C (500 mg) was added. Hydrogen was introduced, and the reaction mixture was was stirred at room temperature for 5 h. The reaction mixture was filtered and concentrated to obtain crude 1-amino-3-methylbutan-2-ol (compound 19c, 618 mg, yield. 26%). The crude product was used in the next step directly without further purifcaition.

### Step 3. Preparation of methyl 3-((3S)-7-bromo-2-((2-hydroxy-3-methylbutyl)amino)-5-(pyridin-2-yl)-3H-benzo[e][1,4]diazepin-3-yl)propanoate (compound 19f)

(*S*)-Methyl 3-(7-bromo-2-oxo-5-(pyridin-2-yl)-2,3-dihydro-1H-benzo[e][1,4]diazepin-3-yl)propanoate (compound 19d, 804 mg, 2 mmol) was dissolved in dried THF(10 mL). The mixture was cooled to 0 °C, and NaH (120 mg, 3 mmol) was added. The mixture was stirred for 30 min. Morpholinyl chlorophosphate (1.024 g, 4 mmol) was added, and the mixture was stirred for 3 h until LCMS indicated that the reaction was completed. 1-Amino-3-methylbutan-2-ol (compound 19c, 618 mg, 6 mmol) was added, and the mixture was stirred overnight. The reaction mixture was poured into ice water and extracted with ethyl acetate. The organic layer was washed with water for three times, dried and concentrated. The residue was purified through preparative TLC to obtain methyl 3-((3*S*)-7-bromo-2-((2-hydroxy-3-methylbutyl)amino)-5-(pyridin-2-yl)-3H-benzo[e][1,4]diaz epin-3-yl)propanoate (compound 19f, 500 mg, yield 51%).
MS m/z(ESI):487 [M+H]⁺.

### Step 4. Preparation of (S)-methyl 3-(8-bromo-1-isopropyl-6-(pyridin-2-yl)-4H-benzo[f]imidazo[1,2-a][1,4]diazepin-4-yl)propanoate (compound 17s)

Methyl 3-((3S)-7-bromo-2-((2-hydroxy-3-methylbutyl)amino)-5-(pyridin-2-yl)-3H-benzo[e][1,4]diazepin-3-yl)propanoate (compound 19f, 500 mg, 1.03 mmol) was dissolved in butanone (15 mL), and Dess-Martin periodinane (654 mg, 1.54 mmol) was added. The mixture was heated to 43 °C, and stirred for 1 h until LCMS indicated that the reaction was completed. The reaction mixture was filtered and concentrated. The residue was purified through preparative TLC to obtain (*S*)-methyl 3-(8-bromo-1-isopropyl-6-(pyridin-2-yl)-4H-benzo[f]imidazo[1,2-a][1,4]diazepin-4-yl)propanoate (compound 17s, 88 mg, yield 18%).
MS m/z (ESI): 467 [M+H]⁺.
¹HNMR (400 MHz, DMSO-*d6*) δ: 8.57-8.55(m, 1H), 8.07 (d, *J* = 8 Hz, 1H), 7.96-7.92 (m, 1H), 7.88-7.85(m, 1H),7.69 (d, *J* = 8 Hz, 1H), 7.61 (d, *J* = 6 Hz, 1H), 7.50-7.47 (m, 1H), 6.82 (m, 1H),4.06-4.03 (m, 1H), 3.61 (s, 3H), 3.30-3.23 (m, 1H), 2.71-2.53 (m, 4H), 1.33 (d, *J* = 6.4 Hz, 3H), 0.74 (d, *J* = 6.8 Hz, 3H).

### Example 20. Preparation of (S)-methyl 3-(8-chloro-6-(pyridin-2-yl)-4H-benzo[f][1,2,4]triazolo[4,3-a][1,4]diazepin-4-yl)propanoate (compound 18s)

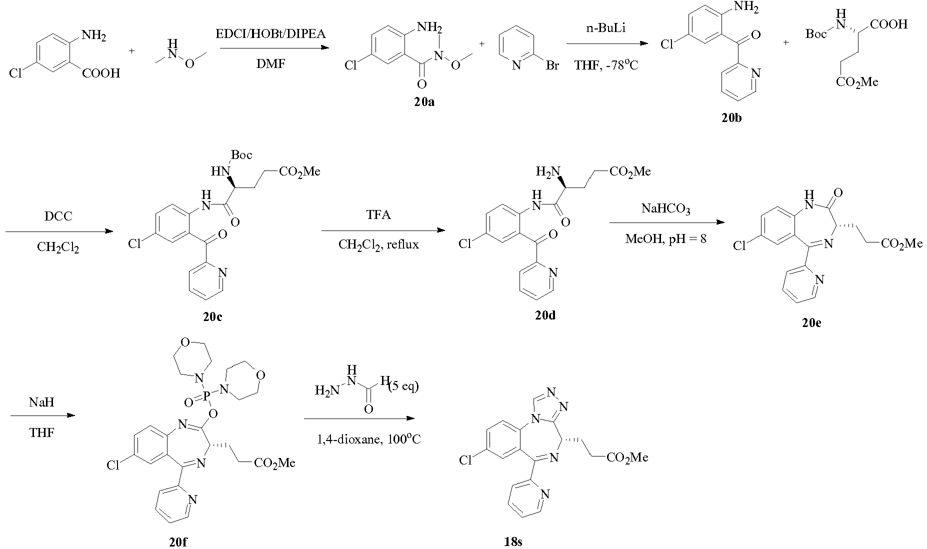

### Step 1. Preparation of 2-amino-5-chloro-N-methoxy-N-methylbenzamide (compound 20a)

2-Amino-5-chlorobenzoic acid (10 g, 58.1 mmol), N,O-dimethylhydroxylamine hydrochloride (10 g, 103 mmol), EDCI (14 g, 71.5 mmol) and 1-hydroxybenzotriazole (9.2 g, 68 mmol) were dissolved in DMF (150 mL), and DIPEA (23.6 g, 183 mmol) was added. The mixture was stirred at room temperature for 7 h until TLC indicated that the reaction was completed. The reaction mixture was diluted with 1 N aq. NaOH solution and extracted with DCM. The organic layer was washed with water for three times, dried and concentrated. The residue was purified through silica gel column chromatography to obtain 2-amino-5-chloro-N-methoxy-N-methylbenzamide (compound 20a, 7.2 g, yield 72%).

### Step 2. Preparation of (2-amino-5-chlorophenyl)(pyridin-2-yl)methanone (compound 20b)

2-Amino-5-chloro-N-methoxy-N-methylbenzamide (compound 20a, 5 g, 23.3 mmol) and 2-bromopyridine (3.7 g, 23.3 mmol) were dissolved in THF (50 mL). The mixture was cooled to -78 °C, and *n*-butyllithium (23 mL, 58.3 mmol) was added. The reaction mixture was stirred for 2 h until TLC indicated that the reaction was completed. IN HCl solution was added to quench the reaction. The water phase was pH-adjusted to basic, and then extracted with ethyl acetate. The organic layer was washed with saturated saline once, dried and concentrated. The residue was purified through silica gel column chromatography to obtain (2-amino-5-chlorophenyl)(pyridin-2-yl)methanone (compound 20b, 4 g, yield 80%).

### Step 3. Preparation of (S)-methyl 5-((4-chloro-2-picolinoylphenyl)amino)-4-((tert-butoxycarbonyl)amino)-5-oxopentanoate (compound 20c)

(2-Amino-5-chlorophenyl)(pyridin-2-yl)methanone (compound 20b, 4 g, 17.2 mmol) and 5-methyl N-*tert*-butoxycarbonyl-L-glutamate (6.7 g, 25.8 mmol) were dissolved in DCM (50 mL). The mixture was cooled to 0 °C, and DCC (7.1 g, 34.4 mmol was added). The mixture was stirred for 24 h until LCMS indicated that the reaction was completed. The reaction mixture was poured into ice water, and extracted with ethyl acetate. The organic layer was washed with water for three times, dried and concentrated. The residue was purified through slilica gel column chromatography to obtain (*S*)-methyl 5-((4-chloro-2-picolinoylphenyl)amino)-4-((*tert*-butoxycarbonyl)amino)-5-oxopentanoate (compound 20c, 5.5 g, yield 67.3%).

### Step 4. Preparation of (S)-methyl 5-((4-chloro-2-picolinoylphenyl)amino)-4-amino-5-oxopentanoate (compound 20d)

(*S*)-Methyl 5-((4-chloro-2-picolinoylphenyl)amino)-4-((*tert*-butoxycarbonyl)amino)-5-oxopentanoate (compound 20c, 5.5 g, 11.6 mmol) was dissolved in DCM (70 mL), and TFA (70 mL) was added. The mixture was heated to 40 °C and stirred for 2 h until LCMS indicated that the reaction was completed. SThe reaction mixture was concentrated to obtain crude (*S*)-methyl 5-((4-chloro-2-picolinoylphenyl)amino)-4-amino-5-oxopentanoate (compound 20d, 6 g), which was used in the next step directly without further purification.

### Step 5. Preparation of (S)-methyl 3-(7-chloro-2-oxo-5-(pyridin-2-yl)-2,3-dihydro-1H-benzo[e][1,4]diazepin-3-yl)propanoate (compound 20e)

(*S*)-Methyl 5-((4-chloro-2-picolinoylphenyl)amino)-4-amino-5-oxopentanoate (compound 20d, 6 g) was dissolved in MeOH (250 mL). NaHCO₃ was added to adjust pH to about 8, and then the mixture was stirred for 24 h until LCMS indicated that the reaction was completed. The reaction mixture was filtered, and the filtrate was poured into ice water. The resulting mixture was extracted with ethyl acetate, and the organic layer was washed with water for three times, dried and concentrated. The residue was purified through silica gel column chromatography to obtain (*S*)-methyl 3-(7-chloro-2-oxo-5-(pyridin-2-yl)-2,3-dihydro-1H-benzo[e][1,4]diazepin-3-yl)propanoate (compound 20e, 2.4 g, yield 57.7%).
MS m/z (ESI): 358 [M+H]⁺.

### Step 6. Preparation of (S)-methyl 3-(7-chloro-2-((dimorpholinophosphoryl)oxy)-5-(pyridin-2-yl)-3H-benzo[e][1,4]diazepin-3-yl)propanoate (compound 20f)

(*S*)-Methyl 3-(7-chloro-2-oxo-5-(pyridin-2-yl)-2,3-dihydro-1H-benzo[e][1,4]diazepin-3-yl)propanoate (compound 20e, 1 g, 2.79 mmol) was dissolved in dried THF(10 mL). The mixture was cooled to 0 °C, and NaH (167 mg, 4.19 mmol) was added. The mixture was stirred for 30 min. Morpholinyl chlorophosphate (1.4 g, 5.58 mmol) was added, and the reaction mixture was stirred for 1 h until LCMS indicated that the reaction was completed. The reaction mixture was poured into ice water and extracted with ethyl acetate. The organic layer was washed with water for three times, dried and concentrated. The residue was purified through TLC to obtain (*S*)-methyl 3-(7-chloro-2-((dimorpholinophosphoryl)oxy)-5-(pyridin-2-yl)-3H-benzo[e][1,4]diazepin-3-yl)propanoate (compound 20f, 300 mg, yield. 20%).

### Step 7. (S)-methyl 3-(8-chloro-6-(pyridin-2-yl)-4H-benzo[f][1,2,4]triazolo [4,3-a][1,4]diazepin-4-yl)propanoate (compound 18s)

(*S*)-Methyl 3-(7-chloro-2-((dimorpholinophosphoryl)oxy)-5-(pyridin-2-yl)-3H-benzo[e][1,4]diazepin-3-yl)propanoate (compound 20f, 300 mg) was dissolved in 1,4-dioxane (2 mL), and formhydrazide (159 mg, 2.65 mmol) was added. The mixture was heated to 100 °C and stirred for 14 h until LCMS indicated that the reaction was completed. The reaction mixture was concentrated, and the residue was purified through Prep-HPLC to obtain (*S*)-methyl 3-(8-chloro-6-(pyridin-2-yl)-4H-benzo[f][1,2,4]triazolo[4,3-a][1,4]diazepin-4-yl)propanoate (compound 18s, 60 mg, yield 31%).
MS m/z (ESI): 382 [M+H]⁺.
¹HNMR (400 MHz, DMSO-*d*₆) δ: 9.23 (s, 1H), 8.52-8.50 (m, 1H), 8.09-8.07 (d, *J* = 8.0 Hz, 1H), 7.98-7.94 (m, 1H), 7.89-7.86 (m, 2H), 7.52-7.49 (m, 2H), 4.42-4.39 (m, 1H), 3.63 (s, 3H), 2.79-2.58 (m, 4H).

### Example 21. Preparation of (S)-methyl 3-(8-chloro-1-cyclopropyl-6-(pyridin-2-yl)-4H-benzo[f]imidazo[1,2-a][1,4]diazepin-4-yl)propanoate (compound 19s)

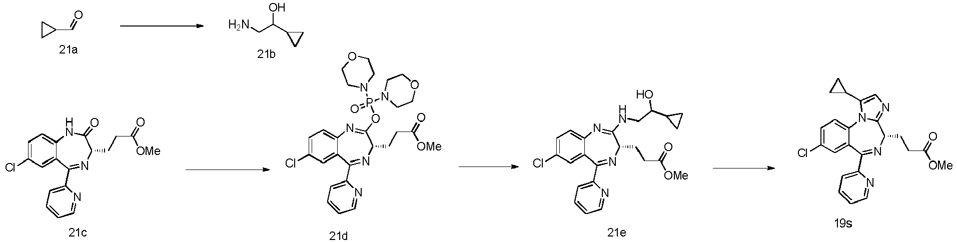

### Step 1. Preparation of 2-amino-1-cyclopropylethanol (compound 21b)

To a 500 m L three-necked flask, TMSCN (25.8 g, 0.26 moL), DCM (300 mL) and ZnI₂ (10 mg) in were added. Th mixture was cooled to 0 °C, and cyclopropylformaldehyde (compound 21a, 15.2 g, 0.217 mol) was added dropwise. The resulting mixture was stirred at room temperature for 3 h, and then was concentrated to dryness. The residue was dissolved in THF (300 mL), and then LAH (9.88 g, 0.26 mol) was added portionwise in an ice bath. The mixture was allowed to react at room temperatue for 3 h. Na₂SO₄·10H₂O (30 g) was added portionwise in an ice bath, and the mixture was stirred overnight. The reaction mixture was filtered and concentrated to obtain 2-amino-1-cyclopropylethanol (compound 21b, 6.5g, yield 30%).

### Step 2. Preparation of methyl 3-((3S)-7-chloro-2-((2-hydroxy-2-cyclopropylethyl)amino) -5-(pyridin-2-yl)-3H-benzo[e][1,4]diazepin-3-yl)propanoate (compound 21e)

(*S*)-Methyl 3-(7-chloro-2-oxo-5-(pyridin-2-yl)-2,3-dihydro-1H-benzo[e][1,4]diazepin-3-yl)propanoate (compound 21c, 1 g, 2.79 mmol) was dissolved in dried THF(10 mL). The mixture was cooled to 0 °C, and NaH (167 mg, 4.19 mmol) was added. The mixture was stirred for 30 min. Morpholinyl chlorophosphate (1.42 g, 5.58 mmol) was added, and the reaction mixture was stirred for 1 h until LCMS indicated that the reaction was completed. 2-Amino-1-cyclopropylethanol (compound 21b, 1.13 g, 11.2 mmol) was added, and the mixture was stirred overnight. The reaction mixture was poured into ice water and extracted with ethyl acetate. The organic layer was washed with water for three times, dried and concentrated. The residue was purified through preparative TLC to obtain methyl 3-((3*S*)-7-chloro-2-((2-hydroxy-2-cyclopropylethyl)amino)-5-(pyridin-2-yl)-3H-benzo[e][1,4] diazepin-3-yl)propanoate (compound 21e, 409 mg, yield 33.3%).
MS m/z (ESI): 441 [M+H]⁺

### Step 3. Preparation of (S)-methyl 3-(8-chloro-1-cyclopropyl-6-(pyridin-2-yl)-4H-benzo[f]imidazo[1,2-a][1,4]diazepin-4-yl)propanoate (compound 19s)

Methyl 3-((3*S*)-7-Chloro-2-((2-hydroxy-2-cyclopropylethyl)amino)-5-(pyridin-2-yl)-3H-benzo[e][1,4]diazepin-3-yl)propanoate (compound 21e, 409 mg, 0.93 mmol) was dissolved in CH₃CN (5 mL), and PDC (699.7 mg, 1.86 mmol) and a little silica gel were added. The mixture was heated to 38 °C and stirred for 1 h until LCMS indicated that the reaction was completed. The reaction mixture was concentrated. The residue was purified through preparative TLC to obtain (*S*)-methyl 3-(8-chloro-1-cyclopropyl-6-(pyridin-2-yl)-4H-benzo[f]imidazo[1,2-a][1,4]diazepin-4-yl)propanoate (compound 19s, 51 mg, yield 13.1%).
MS m/z (ESI): 421 [M+H]+.
¹HNMR (400 MHz, DMSO-*d*₆) δ: 8.55-8.54(d, *J* = 4 Hz, 1H), 8.09-8.07 (d, *J* = 8.0 Hz, 1H), 7.97-7.92 (m, 2H), 7.80-7.78 (m, 1H), 7.51-7.48 (m, 1H), 6.76 (s, 1H), 4.08-4.05 (m, 1H), 3.61 (s, 3H), 2.72-2.50 (m, 4 H), 1.84-1.80 (m, 1H), 1.02-0.99 (m, 1H), 0.82-0.78(m, 1H), 0.72-0.69 (m, 2H), 0.55-0.52 (m, 1H).

### Example 22. Preparation of (S)-methyl 3-(8-bromo-1-cyclobutyl-6-(pyridin-2-yl)-4H-benzo[f]imidazo[1,2-a][1,4]diazepin-4-yl)propanoate (compound 20s)

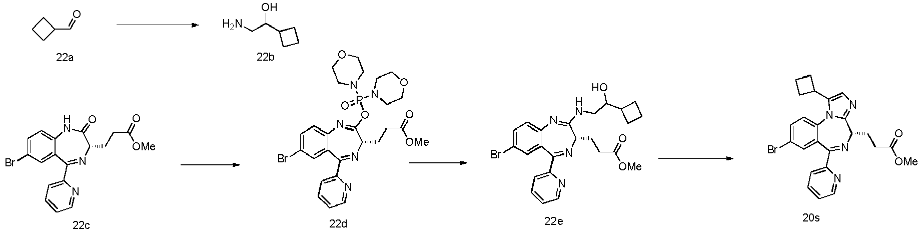

### Step 1. Preparation of 2-amino-1-cyclobutylethanol (compound 22b)

To a 500 mL three-necked flask, TMSCN (25.8 g, 0.26 moL), DCM (300 mL) and ZnI₂ (10 mg) were added. The mixture was cooled to 0 °C understirring, and cyclobutanecarbaldehyde (compound 22a, 18.2 g, 0.217 mol) was added dropwise. The resulting mixture was stirred at room temperature for 3 h, and then was concentrated to dryness. The residue was dissolved in THF (300 mL), and then LAH (9.88 g, 0.26 mol) was added portionwise in an ice bath. The mixture was allowed to react at room temperatue for 3 h. Na₂SO₄·10H₂O (30 g) was added portionwise in an ice bath, and the mixture was stirred overnight. The reaction mixture was filtered and concentrated to obtain 2-amino-1-cyclobutylethanol (compound 22b, 7.5g, yield 30%). The crude product was used in the next step directly without further purification.

### Step 2. Preparation of methyl 3-((3S)-7-bromo-2-((2-hydroxy-2-cyclobutylethyl)amino)-5-(pyridin-2-yl)-3H-benzo[e][1,4]diazepin-3-yl)propanoate (compound 22e)

(*S*)-Methyl 3-(7-bromo-2-oxo-5-(pyridin-2-yl)-2,3-dihydro-1H-benzo[e][1,4]diazepin-3-yl)propanoate (compound 22c, 1 g, 2.49 mmol) was dissolved in dried THF (10 mL). The mixture was cooled to 0 °C, and NaH (149.2 mg, 3.73 mmol) was added. The mixture was stirred for 30 min. Morpholinyl chlorophosphate (1.27 g, 4.98 mmol) was added, and the mixture was stirred for 1 h until LCMS indicated that the reaction was completed. 2-Amino-1-cyclobutylethanol (compound 22b, 1.15 g, 9.96 mmol) was added and the mixture was stirred overnight. The reaction mixture was poured into ice water,and was extracted with ethyl acetate. The organic layer was washed with water for three times, dried and concentrated. The residue was purified through preparative TLC to obtain methyl 3-((3*S*)-7-bromo-2-((2-hydroxy-2-cyclobutylethyl)amino)-5-(pyridin-2-yl)-3H-benzo[e][1,4] diazepin-3-yl)propanoate (compound 22e, 414 mg, yield 33.3%).
MS m/z (ESI): 499 [M+H]⁺

### Step 3. Preparation of (S)-methyl 3-(8-bromo-1-cyclobutyl-6-(pyridin-2-yl)-4H-benzo[f]imidazo[1,2-a][1,4]diazepin-4-yl)propanoate (compound 20s)

Methyl 3-((3*S*)-7-bromo-2-((2-hydroxy-2-cyclobutylethyl)amino)-5-(pyridin-2-yl)-3H-benzo[e][1,4]diazepin-3-yl)propanoate (compound 22e, 414 mg, 0.83 mmol) was dissolved in CH₃CN (5 mL), and PDC (624 mg, 1.66mmol) and a little silica gel were added. The mixture was heated to 38 °C and stirred for 1 h until LCMS indicated that the reaction was completed. The reaction mixture was concentrated. The residue was purified through preparative TLC to obtain (*S*)-methyl 3-(8-bromo-1-cyclobutyl-6-(pyridin-2-yl)-4H-benzo[f]imidazo [1,2-a][1,4]diazepin-4-yl)propanoate (compound 20s, 52 mg, yield 13.1%).
MS m/z (ESI): 479 [M+H]+.
¹HNMR (400 MHz, DMSO-*d*₆) δ: 8.56-8.54(m, 1H), 8.09-8.07 (d, *J* = 8.0 Hz, 1H), 7.96-7.87 (m, 2H), 7.59 (m, 1H), 7.50-7.42 (m, 2H), 6.96 (s, 1H), 4.06-4.03 (m, 1H), 3.61-3.54 (m, 4H), 2.72-2.48 (m, 5 H), 2.20-1.78 (m, 5H).

### Example 23. Preparation of methyl 3-(8-bromo-1-methyl-6-(pyridin-2-yl)-4H-benzo[f]imidazo[1,2-a][1,4]diazepin-4-yl)butyrate (compound 21)

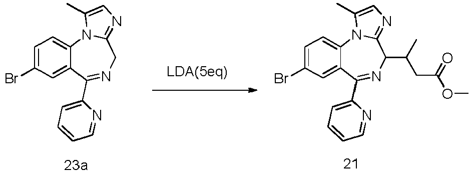

8-Bromo-1-methyl-6-(pyridin-2-yl)-4H-benzo[f]imidazo[1,2-a][1,4]diazepine (compound 23a, 300 mg, 0.85 mmol) was dissolved in dried THF (2 mL). The mixture was cooled to -65 °C, and LDA (2.13 mL, 4.25 mmol) was added dropwise. The mixture was stirred for 30 min. Methyl crotonate (170.2 mg, 1.7 mmol) was added, and the mixture was stirred for 1 h until LCMS indicated that the reaction was completed. The reaction mixture was concentrated, and the residue was purified through preparative TLC to obtain methyl 3-(8-bromo-1-methyl-6-(pyridin-2-yl)-4H-benzo[f]imidazo[1,2-a][1,4]diazepin-4-yl)butyrate (compound 21, 30 mg, yield 7.8%).
MS m/z (ESI): 453[M+H]⁺.
¹HNMR (400 MHz, DMSO-*d6*) δ: 8.54-8.53(m, 1H), 8.11-8.09 (m, 1H), 7.95-7.87 (m, 2H), 7.68-7.64 (m, 2H), 7.49 (m, 1H), 6.68 (s, 1H), 3.88-3.86 (m, 1H), 3.58 (s, 3H), 3.05-2.91 (m, 3 H), 2.25 (s, 3H), 1.25 (s, 3H).

### Example 24. Preparation of methyl 3-(8-bromo-1-methyl-6-(pyridin-2-yl)-4H-benzo[f]imidazo[1,2-a][1,4]diazepin-4-yl)-2-methylpropanoate (compound 22)

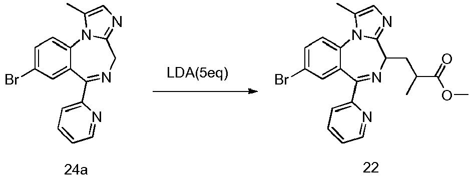

8-Bromo-1-methyl-6-(pyridin-2-yl)-4H-benzo[f]imidazo[1,2-a][1,4]diazepine (compound 24a, 300 mg, 0.85 mmol) was dissolved in dried THF (2 mL). The mixture was cooled to -65 °C, and LDA (2.13 mL, 4.25 mmol) was added dropwise. The mixture was stirred for 30 min. Methyl methacrylate (170.2 mg, 1.7 mmol) was added, and the reaction mixture was stirred for 1 h until LCMS indicated that the reaction was completed. The reaction mixture was concentrated, and the residue was purified through preparative TLC to obtain methyl 3-(8-bromo-1-methyl-6-(pyridin-2-yl)-4H-benzo[f]imidazo [1,2-a][1,4]diazepin-4-yl)-2-methylpropanoate (compound 22, 25 mg, yield 6.5%).
MS m/z (ESI): 453[M+H]⁺.
¹HNMR (400 MHz, DMSO-*d6*) δ: 8.54-8.53(m, 1H), 8.11-8.09 (m, 1H), 7.95-7.87 (m, 2H), 7.68-7.64 (m, 2H), 7.49 (m, 1H), 6.68 (s, 1H), 3.88-3.86 (m, 1H), 3.58 (s, 3H), 2.91-2.82 (m, 2 H), 2.75-2.72 (m, 1H), 2.25 (s, 3H), 1.51 (s, 3H).

### Example 25. Preparation of (S)-methyl 3-(8-bromo-1-cyclopropyl-2-methyl-6-(pyridin-2-yl)-4H-benzo[f]imidazo[1,2-a][1,4]diazepin-4-yl)propanoate (compound 23s)

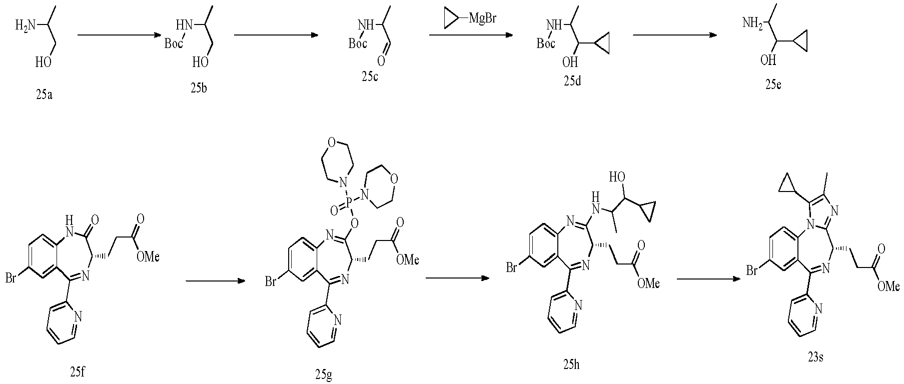

### Step 1. Preparation of 2-(N-tert-butoxycarbonyl-DL-amino)propanol (compound 25b)

2-(DL-Amino)propanol (compound 25a, 4.0g, 0.053 mol) was dissolved in MeOH, and Boc₂O (12.84 g, 0.059 mol) was added. The mixture was stirred at room temperature for 12 h. Te reaction mixture was concentrated, and the residue was diluted with DCM. The organic phase was washed with water for three times, dried, and concentrated to obtain 2-(N-*tert*-butoxycarbonyl-DL-amino)propanol (compound 25b).

### Step 2. Preparation of 2-(N-tert-butoxycarbonyl-DL-amino)propionaldehyde (compound 25c)

2-(N-*Tert*-butoxycarbonyl-DL-amino)propanol (compound 25b) was dissolved in DCM, and Dess-Martin periodinane (22.9 g) was added. The mixture was stirred at room temperature about 24 h. The reaction mixture was filtered and concentrated. The residue was purified through silica gel column chromatography to obtain 2-(N-*tert*-butoxycarbonyl-DL-amino)propionaldehyde (compound 25c, 3 g, yield. 33%).

### Step 3. Preparation of 2-(N-tert-butoxycarbonyl-DL-amino)-1-cyclopropylpropanol 1 (compound 25d)

2-(N-*tert*-butoxycarbonyl-DL-amino)propionaldehyde (compound 25c, 0.5 g, 2.89 mmol) was dissolved in dried THF. The mixture was cooled to -78°C, and cyclopropylmagnesium bromide (7.28 mL, 7.28 mmol) was added dropwise. The mixture was stirred at 0 °C for 4 h. Sat. aq. NH₄Cl solution was added to quench the reaction. The reaction mixture was extracted with ethyl acetate twice, and the organic layer was washed with water, dried and concentrated. The residue was purified through silica gel column chromatography to obtain 2-(N-*tert*-butoxycarbonyl-DL-amino)-1-cyclopropylmethnol (compound 25d, 0.1g, yield 16%).

### Step 4. Preparation of 1-cyclopropyl-2-(DL-amino)propanol (compound 25e)

2-(N-*tert*-butoxycarbonyl-DL-amino)-1-cyclopropylmethnol (compound 25d, 0.5 g, 2.33 mmol) was dissolved in DCM. HCl/dioxane (2 mL, 4N) was added, and the mixture was stirred at room temperature for 1 h. The reaction mixture was concentrated, and the residue was used in the next step without further purification.

### Step 5. Preparation of methyl 3-((3S)-7-bromo-2-(1-methyl-(2-hydroxy-2-cyclopropyl)ethylamino)-5-(pyridin-2-yl)-3H-benzo[e][1,4]diazepin-3-yl)propanoate (compound 25h)

(*S*)-Methyl 3-(7-bromo-2-oxo-5-(pyridin-2-yl)-2,3-dihydro-1H-benzo[e][1,4]diazepin-3-yl)propanoate (compound 25f, 804 mg, 2 mmol) was dissolved in dried THF (10 mL). The mixture was cooled to 0 °C, and NaH (120 mg, 3 mmol) was added. The mixture was stirred for 30 min. Morpholinyl chlorophosphate (1.024 g, 4 mmol) was added, and the reaction mixture was stirred for 3 h until LCMS indicated that the reaction was completed. 1-Cyclopropyl-2-(DL-amino)propanol (690 mg, 6 mmol) was added, and the mixture was stirred overnight. The reaction mixture was poured into ice water, and extracted with ethyl acetate. The organic layer was washed with water for three times, dried and concentrated. The residue was purified through preparative TLC to obtain methyl 3-((3*S*)-7-bromo-2-(1-methyl-(2-hydroxy-2-cyclopropyl)ethylamino)-5-(pyridin-2-yl)-3H-benzo[e][1,4]diazepin-3-yl)propanoate (compound 25h, 500 mg, yield 50%).
MS m/z (ESI): 499 [M+H]⁺

### Step 6. Preparation of (S)-methyl 3-(8-bromo-1-cyclopropyl-2-methyl-6-(pyridin-2-yl)-4H-benzo[f]imidazo[1,2-a][1,4]diazepin-4-yl)propanoate (compound 23s)

Methyl 3-((3*S*)-7-bromo-2-(1-methyl-(2-hydroxy-2-cyclopropyl)ethylamino)-5-(pyridin-2-yl)-3H-be nzo[e][1,4]diazepin-3-yl)propanoate (compound 25h, 500 mg, 1.00 mmol) was dissolved in butanone (15 mL), and Dess-Martin periodinane (634 mg, 1.5 mmol) was added. The mixture was heated to 43°C, and stirred for 1 h until LCMS indicated that the reaction was completed. The reaction mixture was filtered and concentrated. The residue was purified through preparative TLC to obtain (*S*)-methyl 3-(8-bromo-1-cyclopropyl-2-methyl-6-(pyridin-2-yl)-4H-benzo[f][1,2,4]triazolo[4,3-a][1,4]diazepin-4-yl)propanoate (compound 23s, 205 mg, yield 42.8%)).
MS m/z (ESI): 479 [M+H]⁺.
¹HNMR (400 MHz, DMSO-*d*₆) δ: 8.53(d, *J* = 4 Hz, 1H), 8.09-8.07 (d*, J* = 8.0 Hz, 1H), 7.97-7.85 (m, 3H), 7.61(s, 1H), 7.51-7.48 (m, 1H), 4.05-4.08 (m, 1H), 3.61 (s, 3H), 2.71-2.49 (m, 4H), 2.25 (s, 3H), 1.02-1.00 (m, 1H), 0.81-0.78(m, 1H), 0.72-0.69 (m, 1H), 0.55-0.53(m, 1H).

### Example 26. Preparation of (S)-methyl 3-(8-bromo-1-(hydroxymethyl)-6-(pyridin-2-yl)-4H-benzo[f]imidazo[1,2-a][1,4]diazepin-4-yl)propanoate (compound 24s)

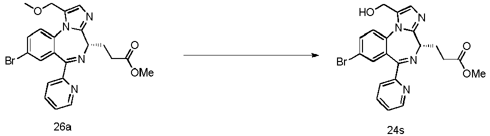

(*S*)-Methyl 3-(8-bromo-1-(methoxymethyl)-6-(pyridin-2-yl)-4H-benzo[f]imidazo [1,2-a][1,4]diazepin-4-yl)propanoate (compound 26a, 1.0 g, 2.0 mmol) was dissolved in DCM. The mixture was cooled to at -78 °C, and BBr₃ (1.9 mL, 20 mmol) was added dropwise. The mixture was warmed to 0 °C and stirred for 3 h. Aq. Na₂CO₃ solution was added to adjust pH to about 8 in an ice bath. The mixture was extracted with DCM, and the organic layer was washed with water for three times, dried and concentrated. The residue was purified through preparative TLC to obtain (*S*)-methyl 3-(8-bromo-1-(hydroxymethyl)-6-(pyridin-2-yl)-4H-benzo[f]imidazo[1,2-a][1,4]diazepin-4-y l)propanoate (compound 24s, 100 mg, yield 10%).
MS m/z (ESI): 455 [M+H]⁺.
¹HNMR (400 MHz, DMSO-*d6*) δ: 8.54-8.53 (m, 1H), 8.08 (d, *J* = 8 Hz, 1H), 8.03-8.01 (m, 1H), 7.96-7.90 (m, 2H), 7.61 (d, *J* = 2.4H, 1H), 7.51-7.47 (m, 1H), 7.01 (s, 1H), 5.44-5.42 (m, 1H), 4.66-4.62 (m, 1H), 4.33-4.29 (m, 1H), 4.12-4.09 (m, 1H), 3.61 (s, 3H), 2.73-2.49 (m, 4H).

### Example 27. Preparation of (S)-methyl 3-(8-chloro-6-(2-fluorophenyl)-1-(methoxymethyl)-4H-benzo[f][1,2,4]triazolo[4,3-a][1,4]diazepin-4-yl)propanoate (compound 25s)

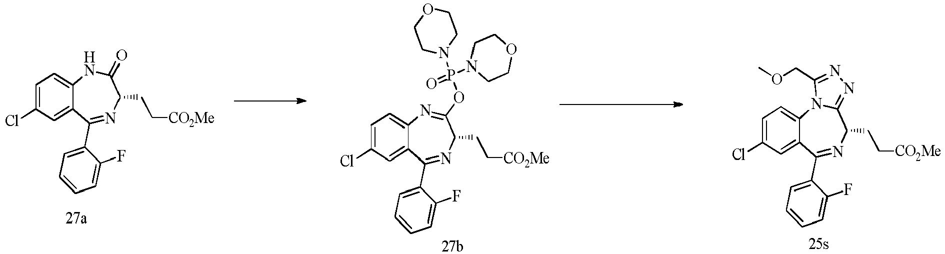

### Step 1. Preparation of (S)-methyl 3-(7-chloro-2-((dimorpholinophosphoryl)oxy)-5-(2-fluorophenyl)-3H-benzo[e][1,4]diazepin-3-yl)propanoate (compound 27b)

(*S*)-Methyl 3-(7-chloro-2-oxo-5-(2-fluorophenyl)-2,3-dihydro-1H-benzo [e][1,4]diazepin-3-yl)propanoate (compound 27a, 748 mg, 0.002 mol) was dissolved in dried THF (15 mL). The mixture was cooled to 0 °C, and NaH (120 mg, 0.003 mol) was added. The mixture was stirred for 30 min. Morpholinyl chlorophosphate (1.024 g, 0.004 mol) was added, and the mixture was stirred for 1 h until LCMS indicated that the reaction was completed. the reaction mixture was poured into ice water and extracted with ethyl acetate. The organic layer was washed with water for three times, dried and concentrated to obtain crude (*S*)-methyl 3-(7-chloro-2-((dimorpholinophosphoryl)oxy)-5-(2-fluorophenyl)-3H-benzo[e][1,4]diazepin-3-yl)propanoate (compound 27b, 1.4 g), which was used in the next step directly without further purification.

### Step 2. Preparation of (S)-methyl 3-(8-chloro-6-(2-fluorophenyl)-1-(methoxymethyl)-4H-benzo[f][1,2,4]triazolo[4,3-a][1,4]diazepin-4-yl)propanoate (compound 25s)

(*S*)-Methyl 3-(7-chloro-2-((dimorpholinophosphoryl)oxy)-5-(2-fluorophenyl)-3H-benzo[e][1,4]diazepin-3-yl)propanoate (compound 27b, 1.4 g) was dissolved in 1,4-dioxane (15 mL), and methoxyacethydrazide (624 mg, 0.006 mol) was added. The mixture was heated to 100 °C and stirred for 48 h until LCMS indicated that the reaction was completed. The reaction mixture was concentrated, and the residue was purified through preparative HPLC to obtain (*S*)-methyl 3-(8-chloro-6-(2-fluorophenyl)-1-(methoxymethyl)-4H-benzo[f][1,2,4]triazolo[4,3-a][1,4]diazepin-4-yl)propanoate (compound 25s, 115mg, yield 14%).
MS m/z (ESI): 443 [M+H]⁺.
¹HNMR (400 MHz, CDCl₃) δ: 8.01-7.99 (m, 1H), 7.91-7.88 (m, 1H), 7.62-7.54 (m, 2H), 7.36-7.31 (m, 2H), 7.25-7.20 (m, 1H), 4.93-4.90 (m, 1H), 4.57-4.54 (m, 1H), 4.30-4.27 (m, 1H), 3.61 (s, 3H), 3.22 (s, 3H), 2.75-2.50 (m, 4H).

### Example 28. Preparation of (S)-methyl 3-(8-chloro-6-(2-fluorophenyl)-1-(methoxymethyl)-4H-benzo[f]imidazo[1,2-a][1,4]diazepin-4-yl)propanoate (compound 26s)

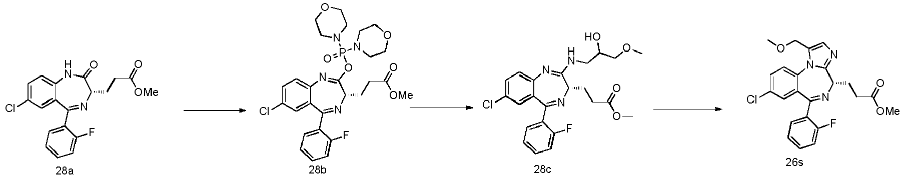

### Step 1. Preparation of methyl 3-((3S)-7-chloro-5-(2-fluorophenyl)-2-((2-hydroxy-3 -methoxypropyl)amino)-3H-benzo [e] [1,4] diazepin-3 -yl)propanoate (compound 28c)

(*S*)-Methyl 3-(7-chloro-2-oxo-5-(2-fluorophenyl)-2,3-dihydro-1H-benzo [e][1,4]diazepin-3-yl)propanoate (compound 28a, 748 mg, 2 mmol) was dissolved in dried THF (15 mL). The mixture was cooled to 0 °C, and NaH (120 mg, 3 mmol) was added. The mixture was stirred for 30 min. Morpholinyl chlorophosphate (1.024 g, 4 mmol) was added, and the mixture was tirred for 1 h until LCMS indicated that the reaction was completed. The reaction mixture was poured into ice water, and extracted with ethyl acetate. The organic layer was washed with water for three times, dried and concentrated to obtain a crude product (1.4 g), which was used in the next step directly without further purification. 1-Amino-3-methoxypropan-2-ol (630.66 mg, 6 mmol) was added, and the mixture was stirred overnight. The reaction mixture was poured into ice water, and extracted with ethyl acetate. The organic layer was washed with water for three times, dried and concentrated. The residue was purified through preparative TLC to obtain methyl 3-((3*S*)-7-chloro-5-(2- fluorophenyl)-2-((2-hydroxy-3 -methoxypropyl)amino)-3H-benzo [e] [1,4] diazepin-3 -yl)propanoate (compound 28c, 250 mg, yield 29%).
MS m/z (ESI): 462[M+H]⁺.

### Step 2. Preparation of (S)-methyl 3-(8-chloro-6-(2-fluorophenyl)-1-(methoxymethyl)-4H-benzo[f]imidazo[1,2-a][1,4]diazepin-4-yl)propanoate (compound 26s)

Methyl 3-((3*S*)-7-chloro-5-(2-fluorophenyl)-2-((2-hydroxy-3-methoxypropyl)amino)-3H-benzo[e][1,4]diazepin-3-yl)propanoate (compound 28c, 250 mg, 0.54 mmol) was dissolved in butanone (10 mL), and Dess-Martin periodinane (573 mg, 1.35 mmol) was added. The mixture was heated to 78°C and stirred for 1 h until LCMS indicated that the reaction was completed. The reaction mixture was filtered and concentrated. The residue was purified through preparative TLC to obtain (*S*)-methyl 3-(8-chloro-1-methoxymethyl-6-(2-fluorophenyl)-4H-benzo[f][1,2,4]triazolo[4,3-a][1,4]diazepin-4-yl)propanoate (compound 26s, 22 mg, yield 9%).
MS m/z (ESI): 442 [M+H]⁺.
¹HNMR (400 MHz, CDCl₃) δ: 7.97-7.95 (m, 1H), 7.85-7.82 (m, 1H), 7.62-7.54 (m, 2H), 7.34-7.30 (m, 2H), 7.23-7.16 (m, 2H), 4.63-4.60 (m, 1H), 4.31-4.28 (m, 1H), 4.07-4.03 (m, 1H), 3.61 (s, 3H), 3.22 (s, 3H), 2.71-2.50 (m, 4H).

### Example 29. Preparation of (S)-methyl 3-(8-chloro-1-cyclobutyl-6-(pyridin-2-yl)-4H-benzo[f]imidazo[1,2-a][1,4]diazepin-4-yl)propanoate (compound 27s)

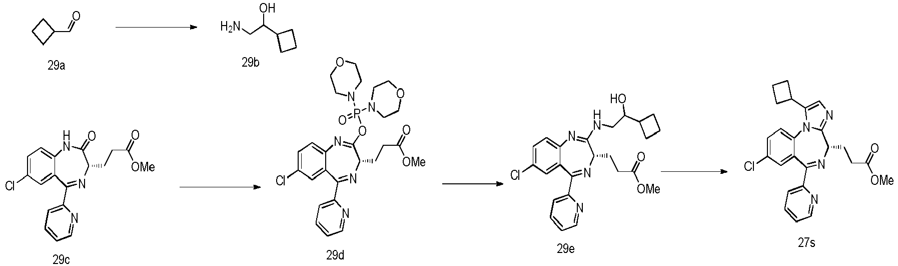

### Step 1. Preparation of 2-amino-1-cyclobutylethanol (compound 29b)

To a 500 mL three-necked flask, TMSCN (25.8 g, 0.26 moL), DCM (300 mL) and ZnI₂ (10 mg) were added. The mixture was cooled to 0 °C under stirring, and cyclobutanecarbaldehyde (compound 29a, 18.2 g, 0.217 mol) was added dropwise. The resulting mixture was stirred at room temperature for 3 h, and then concentrated. The residue was dissolved in THF (300 mL), and then LAH (9.88 g, 0.26 mol) was added portionwise at 0 °C. The mixture was allowed to react at room temperatue for 3 h. Na₂SO₄·10H₂O (30 g) was added portionwise in an ice bath, and the mixture was stirred overnight. The reaction mixture was filtered concentrated to obtain 2-amino-1-cyclobutylethanol (compound 29b, 7.5 g, yield 30%).

### Step 2. Preparation of methyl 3-((3S)-7-chloro-2-((2-hydroxy-2-cyclobutyl)ethylamino)-5-(pyridin-2-yl)-3H-benzo[e][1,4]diazepin-3-yl)propanoate (compound 29e)

(*S*)-Methyl 3-(7-chloro-2-oxo-5-(pyridin-2-yl)-2,3-dihydro-1H-benzo [e][1,4]diazepin-3-yl)propanoate (compound 29c, 1 g, 2.79 mmol) was dissolved in dried THF (10 mL). The mixture was cooled to 0 °C, and NaH (167 mg, 3.73 mmol) was added. The mixture was stirred for 30 min. Morpholinyl chlorophosphate (1.42 g, 5.58 mmol) was added, and the mixture was stirred for 1 h until LCMS indicated that the reaction was completed. 2-Amino-1-cyclobutylethanol (1.29 g, 11.2 mmol) was added, and the mixture was stirred overnight. The reaction mixture was poured into ice water, and extracted with ethyl acetate. The organic layer was washed with water for three times, dried and concentrated. The residue was purified through preparative TLC to obtain methyl 3-((3*S*)-7-chloro-2-((2-hydroxy-2-cyclobutyl)ethylamino)-5-(pyridin-2-yl)-3H-benzo[e][1,4] diazepin-3-yl)propanoate (compound 29e, 423 mg, yield 33.3%).
MS m/z (ESI): 455 [M+H]⁺.

### Step 3. Preparation of (S)-methyl 3-(8-chloro-1-cyclobutyl-6-(pyridin-2-yl)-4H-benzo[f]imidazo[1,2-a][1,4]diazepin-4-yl)propanoate (compound 27s)

Methyl 3-((3S)-7-chloro-2-((2-hydroxy-2-cyclobutyl)ethylamino)-5-(pyridin-2-yl)-3H-benzo[e][1,4]diazepin-3-yl)propanoate (compound 29e, 423 mg, 0.93 mmol) was dissolved in CH₃CN (5 mL), and PDC (699.7 mg, 1.86 mmol) and a little silica gel were added. The mixture was heated to 38 °C and stirred for 1 h until LCMS indicated that the reaction was completed. The reaction mixture was concentrated. The residue was purified through preparative TLC to obtain (*S*)-methyl 3-(8-chloro-1-cyclobutyl-6-(pyridin-2-yl)-4H-benzo[f]imidazo[1,2-a][1,4]diazepin-4-yl) propanoate (compound 27s, 53 mg, yield 13.1%).
MS m/z (ESI): 435 [M+H]+.
¹HNMR (400 MHz, DMSO-*d6*) δ: 8.55-8.54(m, 1H), 8.09-8.07 (m, 1H), 7.96-7.92 (m, 1H), 7.78-7.75 (m, 1H), 7.52-7.47 (m, 3H), 6.96 (s, 1H), 4.06-4.03 (m, 1H), 3.61-3.55 (m, 4H), 2.72-2.50 (m, 6 H), 2.20-2.16 (m, 1H), 2.03-1.78 (m, 4H).

### Example 30. Preparation of (S)-methyl 3-(8-chloro-1-ethyl-6-(pyridin-2-yl)-4H-benzo[f][1,2,4]triazolo[4,3-a][1,4]diazepin-4-yl)propanoate (compound 28s)

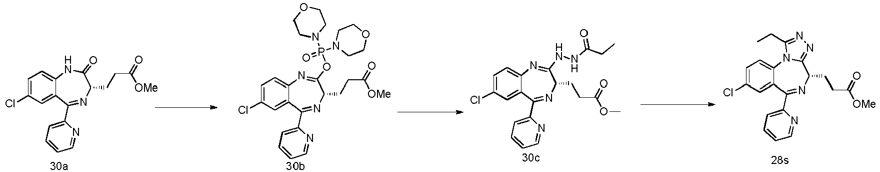

### Step 1. Preparation of (S)-methyl 3-(7-chloro-2-((dimorpholinophosphoryl)oxy)-5-(pyridin-2-yl)-3H-benzo[e][1,4]diazepin-3-yl)propanoate (compound 30b)

(*S*)-Methyl 3-(7-chloro-2-oxo-5-(pyridin-2-yl)-2,3-dihydro-1H-benzo[e][1,4]diazepin-3-yl) propanoate (compound 30a, 1 g, 2.79 mmol) was dissolved in dried THF (10 mL). The mixture was cooled to 0 °C, and NaH (167 mg, 4.19 mmol) was added. The mixture was stirred for 30 min. Morpholinyl chlorophosphate (1.42 g, 5.58 mmol) was added, and the mixture was stirred for 1 h until LCMS indicated that the reaction was completed. Water was added, and the mixture was extracted with ethyl acetate for three times. The organic layer was dried and concentrated to obtain a crude product. The crude product was used in the next step directly without further purification.
MS m/z (ESI): 576 [M+H]⁺

### Step 2. Preparation of (S)-methyl 3-(8-chloro-1-ethyl-6-(pyridin-2-yl)-4H-benzo[f][1,2,4]triazolo[4,3-a][1,4]diazepin-4-yl)propanoate (compound 28s)

(*S*)-Methyl 3-(7-chloro-2-((dimorpholinophosphoryl)oxy)-5-(pyridin-2-yl)-3H-benzo[e][1,4]diazepin-3-yl)propanoate (compound 30b) obtained in Step 1 was dissolved in dioxane, and propionohydrazide (737 mg, 8.37 mmol) was added. The mixture was heated to 100 °C and stirred for 20 h. The reaction mixture was poured into ice water and extracted with ethyl acetate. The organic layer was washed with water for three times, dried and concentrated. The residue was purified through preparative TLC to obtain (*S*)-methyl 3-(8-chloro-1-ethyl-6-(pyridin-2-yl)-4H-benzo[f][1,2,4]triazolo[4,3-a][1,4]diazepin-4-yl) propanoate (compound 28s, 110 mg, yield 8.7%).
MS m/z (ESI): 455 [M+H]⁺.
¹HNMR (400 MHz, DMSO-*d6*) δ: 8.56-8.54(m, 1H), 8.11-8.09 (m, 1H), 7.98-7.93 (m, 1H), 7.86-7.81 (m, 2H), 7.53-7.49 (m, 2H), 4.31-4.28 (m, 1H), 3.63 (s, 3H), 2.79-2.50 (m, 6H), 1.22-1.18 (m, 3H).

### Example 31. Preparation of (S)-methyl 3-(8-chloro-1-isopropyl-6-(pyridin-2-yl)-4H-benzo[f][1,2,4]triazolo[4,3-a][1,4]diazepin-4-yl)propanoate (compound 29s)

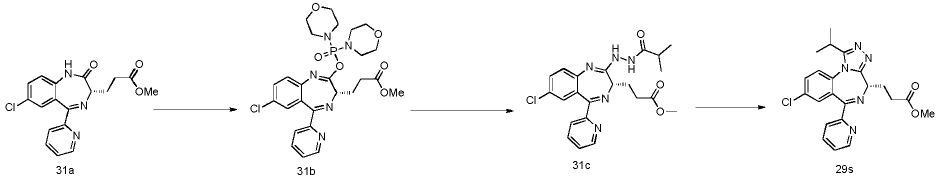

### Step 1. Preparation of (S)-methyl 3-(7-chloro-2-((dimorpholinophosphoryl)oxy)-5-(pyridin-2-yl)-3H-benzo[e][1,4]diazepin-3-yl)propanoate (compound 31b)

(*S*)-Methyl 3-(7-chloro-2-oxo-5-(pyridin-2-yl)-2,3-dihydro-1H-benzo [e][1,4]diazepin-3-yl)propanoate (compound 31a, 1 g, 2.79 mmol) was dissolved in dried THF(10 mL). The mixture was cooled to 0 °C, and NaH (167 mg, 4.19 mmol) was added. The mixture was stirred for 30 min. Morpholinyl chlorophosphate (1.42 g, 5.58 mmol) was added, and the mixture was stirred for 1 h until LCMS indicated that the reaction was completed. Water was added, and the mixture was extracted with ethyl acetate for three times. The organic layer was dried and concentrated to obtain a crude product. The crude product was used in the next step directly without further purification.
MS m/z (ESI): 576 [M+H]⁺.

### Step 2. Preparation of (S)-methyl 3-(8-chloro-1-isopropyl-6-(pyridin-2-yl)-4H-benzo[f][1,2,4]triazolo[4,3-a][1,4]diazepin-4-yl)propanoate (compound 29s)

(*S*)-Methyl 3-(7-chloro-2-((dimorpholinophosphoryl)oxy)-5-(pyridin-2-yl)-3H-benzo[e][1,4]diazepin-3-yl)propanoate (compound 31b) was dissolved in dioxane, and isobutyrohydrazide (854 mg, 8.37 mmol) was added. The mixture was heated to 100 °C and stirred for 20 h. The mixture was poured into ice water and extracted with ethyl acetate. The organic layer was washed with water for three times, dried and concentrated. The residue was purified through preparative TLC to obtain (*S*)-methyl 3-(8-chloro-1-isopropyl-6-(pyridin-2-yl)-4H-benzo[f][1,2,4]triazolo[4,3-a][1,4]diazepin-4-yl) propanoate (compound 29s, 114 mg, yield 8.7%).
MS m/z (ESI): 468 [M+H]⁺.
¹HNMR (400 MHz, DMSO-*d6*) δ: 8.56-8.54(m, 1H), 8.10-8.08 (m, 1H), 7.98-7.93 (m, 1H), 7.88-7.81 (m, 2H), 7.53-7.49 (m, 2H), 4.30-4.26 (m, 1H), 3.63 (s, 3H), 3.45-3.38 (m, 1H), 2.78-2.50 (m, 4H), 1.47-1.45 (m, 3H), 0.91-0.89 (m, 3H).

### Example 32. Preparation of (S)-ethyl 3-(8-chloro-1-methyl-6-(pyridin-2-yl)-4H-benzo[f]imidazo[1,2-a] [1,4]diazepin-4-yl)propanoate (compound 30s)

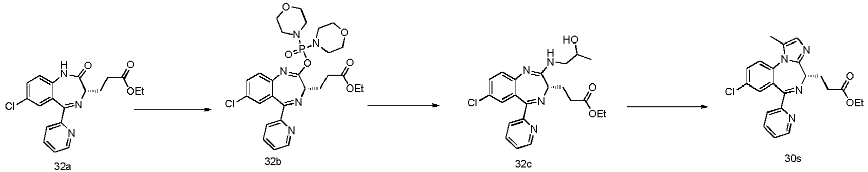

### Step 1. Preparation of ethyl 3-((3S)-7-chloro-2-((2-hydroxypropyl)amino)-5-(pyridin-2-yl)-3H-benzo[e][1,4]diazepin-3-yl)propanoate (compound 32c)

(*S*)-Ethyl 3-(7-chloro-2-oxo-5-(pyridin-2-yl)-2,3-dihydro-1H-benzo [e][1,4]diazepin-3-yl)propanoate (compound 32a, 1 g, 2.69 mmol) (prepared according to the synthesis of compound 20e in Example 20, except that 5-ethyl N-*tert*-butoxycarbonyl-L-glutamate was used instead of 5-methyl N-*tert*-butoxycarbonyl-L-glutamate) in was dissolved dried THF (10 mL). The mixture was cooled to 0 °C, and NaH (162 mg, 4.04 mmol) was added. The mixture was stirred for 30 min. Morpholinyl chlorophosphate (1.37 g, 5.38 mmol) was added, and the mixture was stirred for 1 h until LCMS indicated that the reaction was completed. 1-Amino-2-propanol (810 mg, 10.8 mmol) was added, and the mixture was stirred overnight. The reaction mixture was poured into ice water, and extracted with ethyl acetate. The organic layer was washed with water for three times, dried and concentrated. The residue was purified through preparative TLC to obtain ethyl 3-((3*S*)-7-chloro-2-((2-hydroxypropyl)amino)-5-(pyridin-2-yl)-3H-benzo [e][1,4]diazepin-3-yl)propanoate (compound 32c, 384 mg, yield 33.3%).
MS m/z (ESI): 429 [M+H]⁺.

### Step 2. Preparation of (S)-ethyl 3-(8-chloro-1-methyl-6-(pyridin-2-yl)-4H-benzo[f]imidazo[1,2-a][1,4]diazepin-4-yl)propanoate(compound 30s)

Ethyl 3-((3S)-7-chloro-2-((2-hydroxypropyl) amino)-5-(pyridin-2-yl)-3H-benzo [e][1,4]diazepin-3-yl)propanoate (compound 32c, 384 mg, 0.90 mmol) was dissolved in CH₃CN (5 mL), and PDC (676.8 mg, 1.80 mmol) and a little silica gel were added. The mixture was heated to 38 °C and stirred for 1 h until LCMS indicated that the reaction was completed. The reaction mixture was concentrated, and the residue was purified through preparative TLC to obtain (*S*)-ethyl 3-(8-chloro-1-methyl-6-(pyridin-2-yl)-4H-benzo[f]imidazo[1,2-a][1,4]diazepin-4-yl)propano ate (compound 30s, 48 mg, yield 13.1%).
MS m/z (ESI): 409 [M+H]+.
¹HNMR (400 MHz, DMSO-*d*₆) δ: 8.55-8.53(m, 1H), 8.10-8.08 (m, 1H), 7.97-7.92 (m, 1H), 7.88-7.82 (m, 2H), 7.51-7.48 (m, 2H), 6.82 (s, 1H), 4.08-4.04 (m, 1H), 3.97-4.01 (m, 2H), 3.61 (s, 3H), 2.72-2.50 (m, 4H), 1.03-1.07 (m, 3H).

### Example 33. Preparation of (S)-methyl 3-(8-chloro-1-cyclopropyl-2-methyl-6-(pyridin-2-yl)-4H-benzo[f]imidazo[1,2-a][1,4]diazepin-4-yl)propanoate (compound 31s)

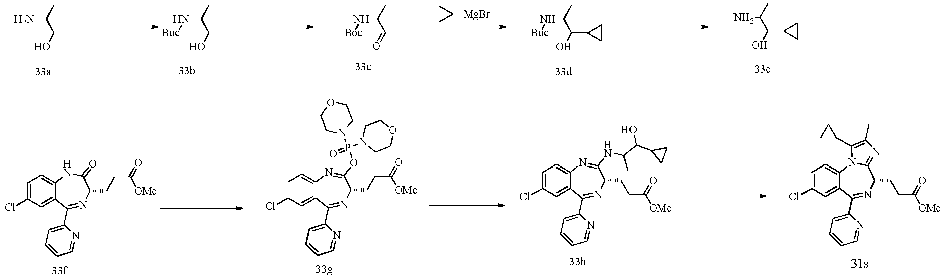

### Step 1. Preparation of 2-(N-tert-butoxycarbonyl-DL-amino)propanol (compound 33b)

2-(DL-Amino)propanol (compound 33a, 4.0 g, 0.053 mol) was dissolved in MeOH, and Boc₂O (12.84 g, 0.059 mol) was added. The mixture was stirred at room temperature for 12 h. The reaction mixture was concentrated, and the residue was diluted with DCM. The organic layer was washed with water for three times, dried and concentrated to obtain 2-(N-*tert*-butoxycarbonyl-DL-amino)propanol (compound 33b) was used in the next step directly without further purification.

### Step 2. Preparation of 2-(N-tert-butoxycarbonyl-DL-amino)propionaldehyde (compound 33c)

2-(N-*tert*-butoxycarbonyl-DL-amino)propanol (compound 33b) obtained in Step 1 was dissolved in DCM, and Dess-Martin periodinane (22.9 g) was added. The mixture was stirred at room temperature for 24 h. The reaction mixture was filtered and concentrated. The residue was purified through silica gel column chromatography to obtain 2-(N-*tert*-butoxycarbonyl-DL-amino)propionaldehyde (compound 33c, 3g, yield 33%).

### Step 3. Preparation of 2-(N-tert-butoxycarbonyl-DL-amino)-1-cyclopropylpropanol (compound 33d)

2-(N-*tert*-butoxycarbonyl-DL-amino)propionaldehyde (compound 33c, 0.5 g, 2.89 mmol) was dissolved in dried THF. The mixture was cooled to -78 °C, and cyclopropylmagnesium bromide (7.28 mL, 7.28 mmol) was added dropwise. The mixture was stirred at 0 °C for 4 h. Sat. aq. NH₄Cl solution was added to quench the reaction. The reaction mixture was extracted with ethyl acetate twice, and the organic layer was washed with water, dried and concentrated. The residue was purified through silica gel column chromatography to obtain 2-(N-*tert*-butoxycarbonyl-DL-amino)-1-cyclopropylpropanol (compound 33d, 0.1 g, yield 16%).

### Step 4. Preparation of 1-cyclopropyl-2-DL-aminopropanol (compound 33e)

2-(N-*tert*-butoxycarbonyl-DL-amino)-1-cyclopropylpropanol (compound 33d, 0.5 g, 2.33 mmol) was dissolved in DCM. HCl/dioxane (2 mL, 4 N) was added, and the mixture was stirred at room temperature for 1 h. The reaction mixture was concentrated, and the residue was used in the next step without further purification.

### Step 5. Preparation of methyl 3-((3S)-7-chloro-2-((1-cyclopropyl-1-hydroxypropan-2-yl)amino)-5-(pyridin-2-yl)-3H-benzo[e][1,4]diazepin-3-yl)propanoate (compound 33h)

(*S*)-Methyl 3-(7-chloro-2-oxo-5-(pyridin-2-yl)-2,3-dihydro-1H-benzo[e][1,4]diazepin-3-yl)propanoate (compound 33f, 716 mg, 2 mmol) was dissolved in dried THF (10 mL). The mixture was cooled to 0 °C, and NaH (120 mg, 3 mmol) was added. The mixture was stirred for 30 min. Morpholinyl chlorophosphate (1.024 g, 4 mmol) was added, and the reaction mixture was stirred for 3 h until LCMS indicated that the reaction was completed. 1-Cyclopropyl-2-DL-aminopropanol (690 mg, 6 mmol) was added, and the mixture was stirred overnight. The reaction mixture was poured into ice water, and extracted with ethyl acetate. The organic layer was washed with water for three times, dried and concentrated. The residue was purified through preparative TLC to obtain methyl 3-((3*S*)-7-chloro-2-((1-cyclopropyl-1-hydroxypropan-2-yl)amino)-5-(pyridin-2-yl)-3H-benzo [e][1,4]diazepin-3-yl)propanoate (compound 33h, 455 mg: yield 50%).
MS m/z (ESI): 455 [M+H]⁺

### Step 6. Preparation of (S)-methyl 3-(8-chloro-1-cyclopropyl-2-methyl-6-(pyridin-2-yl)-4H-benzo[f]imidazo[1,2-a][1,4]diazepin-4-yl)propanoate (compound 31s)

Methyl 3-((3*S*)-7-chloro-2-((1-cyclopropyl-1-hydroxypropan-2-yl)amino)-5-(pyridin-2-yl)-3H-benzo[e][1,4]diazepin-3-yl)propanoate (compound 33h, 455 mg, 1.00 mmol) was dissolved in butanone (15 mL), and Dess-Martin periodinane (634 mg, 1.5 mmol) was added. The mixture was heated to 43°C, and stirred for 1 h until LCMS indicated that the reaction was completed. The reaction mixture was filtered and concentrated. The residue was purified through preparative TLC to obtain (*S*)-methyl 3-(8-chloro-1-cyclopropyl-2-methyl-6-(pyridin-2-yl)-4H-benzo[f]imidazo[1,2-a][1,4]diazepin -4-yl)propanoate (compound 31s, 50 mg, yield 11.5%)).
MS m/z (ESI):435 [M+H]⁺.
¹HNMR (400 MHz, DMSO-*d6*) δ: 8.54(d, *J* = 4 Hz, 1H), 8.09-8.07 (d, *J* = 8.0 Hz, 1H), 7.98-7.85 (m, 3H), 7.61(s, 1H), 7.51-7.48 (m, 1H), 4.08-4.04 (m, 1H), 3.61 (s, 3H), 2.71-2.49 (m, 4H), 2.25 (s, 3H), 1.02-1.00 (m, 1H), 0.81-0.78(m, 1H), 0.72-0.69 (m, 1H), 0.55-0.53(m, 1H).

### Example 34. Preparation of (S)-methyl 3-(8-chloro-1-(methoxymethyl)-6-(pyridin-2-yl)-4H-benzo[f][1,2,4]triazolo[4,3-a][1,4]diazepin-4-yl)propanoate (compound 32s)

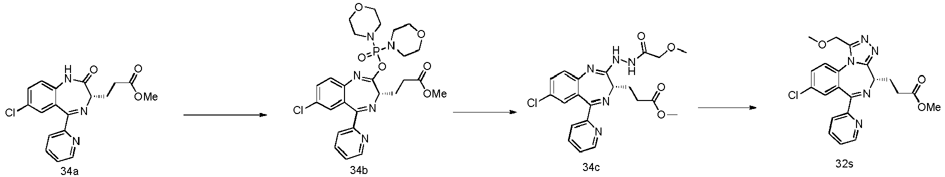

### Step 1. Preparation of (S)-methyl 3-(7-chloro-2-((dimorpholinophosphoryl)oxy)-5-(pyridin-2-yl)-3H-benzo[e][1,4]diazepin-3-yl)propanoate (compound 34b)

(*S*)-Methyl 3-(7-chloro-2-oxo-5-(pyridin-2-yl)-2,3-dihydro-1H-benzo [e][1,4]diazepin-3-yl)propanoate (compound 34a, 1 g, 2.79 mmol) was dissolved in dried THF (10 mL). The mixture was cooled to 0 °C, and NaH (167 mg, 4.19 mmol) was added. The mixture was stirred for 30 min. Morpholinyl chlorophosphate (1.42 g, 5.58 mmol) was added, and the mixture was stirred for 1 h until LCMS indicated that the reaction was completed. Water was added, and the mixture was extracted with ethyl acetate for three times. The organic layer was dried and concentrated to obtain a crude product. The crude product was used in the next step directly without further purification.
MS m/z (ESI): 576 [M+H]⁺.

### Step 2. Preparation of (S)-methyl 3-(8-chloro-1-(methoxymethyl)-6-(pyridin-2-yl)-4H-benzo[f][1,2,4]triazolo[4,3-a][1,4]diazepin-4-yl)propanoate (compound 32s)

(*S*)-Methyl 3-(7-chloro-2-((dimorpholinophosphoryl)oxy)-5-(pyridin-2-yl)-3H-benzo[e][1,4]diazepin-3-yl)propanoate (compound 34b) obtained in Step 1 was dissolved in dioxane, and methoxyacethydrazide (870 mg, 8.37 mmol) was added. The mixture was heated to 100 °C and stirred for 20 h. The mixture was poured into ice water and extracted with ethyl acetate. The organic layer was washed with water for three times, dried and concentrated. The residue was purified through preparative TLC to obtain (*S*)-methyl 3-(8-chloro-1-(methoxymethyl)-6-(pyridin-2-yl)-4H-benzo[f][1,2,4]triazolo[4,3-a][1,4] diazepin-4-yl)propanoate (compound 32s, 103 mg, yield 8.7%).
MS m/z(ESI): 426 [M+H]⁺.
¹HNMR (400 MHz, DMSO-*d6*) δ: 8.55-8.53(m, 1H), 8.11-8.09 (m, 1H), 7.97-7.87 (m, 3H), 7.55-7.49 (m, 2H), 4.79-4.76 (m, 1H), 4.56-4.53 (m, 1H), 4.36-4.33 (m, 1H), 3.63 (s, 3H), 3.27 (s, 3H), 2.80-2.57 (m, 4H).

### Example 35. Preparation of (S)-methyl 3-(8-chloro-1-(methoxymethyl)-6-(pyridin-2-yl)-4H-benzo[f]imidazo[1,2-a][1,4]diazepin-4-yl)propanoate (compound 33s)

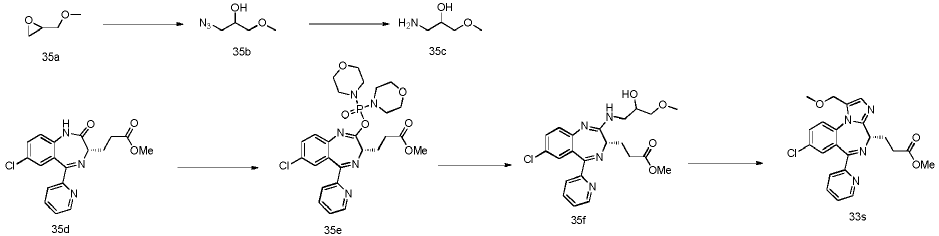

### Step 1. Preparation of 1-azido-3-methoxypropan-2-ol (compound 35b)

2-(Methoxymethyl)oxirane (compound 35a, 2.5 g, 28.4 mmol) was dissolved in DMF, and NaN₃ (5.54 g, 85.1 mmol), NH₄Cl (6.07 g, 113.6 mmol) and H₂O (0.8 mL) were added. The mixture was heated to 80 °C and stirred for 3 h. The mixture was poured into ice water and extracted with ethyl acetate. The organic layer was washed with water for three times, dried and concentrated to obtain 1-azido-3-methoxypropan-2-ol (compound 35b), which was used in the next step directly without further purification.

### Step 2. Preparation of 1-amino-3-methoxypropan-2-ol (compound 35c)

1-Azido-3-methoxypropan-2-ol (compound 35b) obtained in Step 1 was dissolved in MeOH, and 10% Pd/C (200 mg) was added. Hydrogen was introduced, and the mixture was stirred at room temperature for 5 h. The reaction mixture was filtered and concentrated to obtain 1-amino-3-methoxypropan-2-ol (compound 35c, 1.5 g, yield 50%).

### Step 3. Preparation of methyl 3-((3S)-7-chloro-2-((2-hydroxy-3-methoxypropyl) amino)-5-(pyridin-2-yl)-3H-benzo[e][1,4]diazepin-3-yl)propanoate (compound 35f)

(*S*)-Methyl 3-(7-chloro-2-oxo-5-(pyridin-2-yl)-2,3-dihydro-1H-benzo [e][1,4]diazepin-3-yl)propanoate (compound 35d, 716 mg, 2 mmol) was dissolved in dried THF (10 mL). The mixture was cooled to 0 °C, and NaH (120 mg, 3 mmol) was added. The mixture was stirred for 30 min. Morpholinyl chlorophosphate (1.024 g, 4 mmol) was added, and the mixture was stirred for 3 h until LCMS indicated that the reaction was completed. 1-Amino-3-methoxypropan-2-ol (compound 35c, 630.66 mg, 6 mmol) was added, and the mixture was stirred overnight. The reaction mixture was poured into ice water, and extracted with ethyl acetate. The organic layer was washed with water for three times, dried and concentrated. The residue was purified through preparative TLC to obtain methyl 3-((3*S*)-7-chloro-2-((2-hydroxy-3-methoxypropyl)amino)-5-(pyridin-2-yl)-3H-benzo[e][1,4] diazepin-3-yl)propanoate (compound 35f, 374 mg, yield 42%).
MS m/z(ESI):445 [M+H]⁺.

### Step 4. Preparation of (S)-methyl 3-(8-chloro-1-(methoxymethyl)-6-(pyridin-2-yl)-4H-benzo[f]imidazo[1,2-a][1,4]diazepin-4-yl)propanoate (compound 33s)

Methyl 3-((3*S*)-7-chloro-2-((2-hydroxy-3-methoxypropyl)amino)-5-(pyridin-2-yl)-3H-benzo[e][1,4]diazepin-3-yl)propanoate (compound 35f, 374 mg, 0.84 mmol) was dissolved in butanone (15 mL), and Dess-Martin periodinane (713 mg, 1.68 mmol) was added. The mixture was heated to 78 °C and stirred for 1 h until LCMS indicated that the reaction was completed. The reaction mixture was filtered and concentrated. The residue was purified through preparative TLC to obtain (S)-methyl 3-(8-chloro-1-(methoxymethyl)-6-(pyridin-2-yl)-4H-benzo[f][1,2,4]triazolo[4,3-a][1,4]diazepin-4-yl)propanoate.
MS m/z (ESI): 425 [M+H]⁺.
¹HNMR (400 MHz, DMSO-*d6*) δ: 8.54-8.52(m, 1H), 8.10-8.08 (m, 1H), 7.96-7.92 (m, 1H), 7.84-7.81 (m, 2H), 7.51-7.47 (m, 2H), 7.14 (s, 1H), 4.53-4.50 (m, 1H), 4.30-4.27 (m, 1H), 4.14-4.10 (m, 1H), 3.61 (s, 3H), 3.25 (s, 3H), 2.75-2.54 (m, 4H).

### Example 36. Preparation of (S)-methyl 3-(8-chloro-6-(2-fluorophenyl)-1-cyclopropyl-4H-benzo[f]imidazo[1,2-a][1,4]diazepin-4-yl)propanoate (compound 34s)

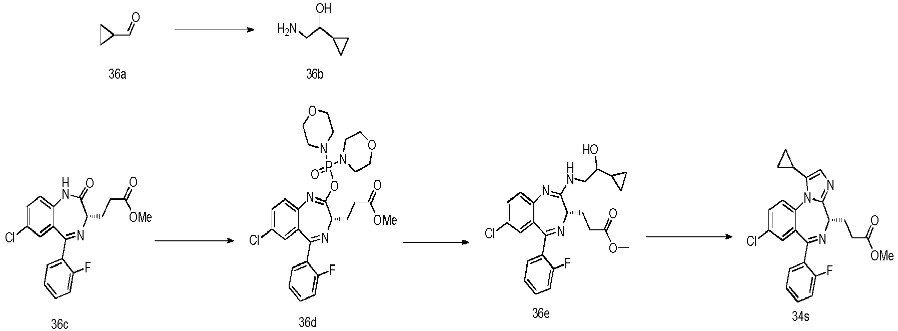

### Step 1. Preparation of 2-amino-1-cyclopropylethanol (compound 36b)

To a 500 Ml three-necked flask, TMSCN (25.8 g, 0.26 moL), DCM (300 mL) and ZnI₂ (10 mg) were added. The mixture was cooled to 0 °C under stirring, and cyclopropylformaldehyde (compound 36a, 15.2 g, 0.217 mol) was added dropwise. The resulting mixture was stirred at room temperature for 3 h, and then concentrated to dryness. The residue was dissolved in THF (300 mL), and then LAH (9.88 g, 0.26 mol) was added portionwise in an ice bath. The mixture was stirred at room temperature for 3 h. Na₂SO₄·10H₂O (30 g) was added portionwise in an ice bath, and the mixture was stirred overnight. The reaction mixture was filtered and concentrated to obtain 2-amino-1-cyclopropylethanol (compound 36b, 6.5 g, yield 30%).

### Step 2. Preparation of methyl 3-((3S)-7-chloro-5-(2-fluorophenyl)-2-((2-cyclopropyl-2-hydroxyethyl)amino)-3H-benzo [e] [1,4] diazepin-3 -yl)propanoate (compound 36e)

(*S*)-Methyl 3-(7-chloro-2-oxo-5-(2-fluorophenyl)-2,3-dihydro-1H-benzo [e][1,4]diazepin-3-yl)propanoate (compound 36c, 748 mg, 2 mmol) was dissolved in dried THF (15 mL). The mixture was cooled to 0 °C, and NaH (120 mg, 3 mmol) was added. The mixture was stirred for 30 min. Morpholinyl chlorophosphate (1.024 g, 4 mmol) was added, and the mixture was stirred for 1 h until LCMS indicated that the reaction was completed. The reaction mixture was poured into ice water, and extracted with ethyl acetate. The organic layer was washed with water for three times, dried and concentrated to obtain a crude product (1.4 g), which was used in the next step directly without further purification. 1-Amino-3-cyclopropyl-2-ethanol(compound 36b, 630.66 mg, 6 mmol) was added, and the mixture was stirred overnight. The reaction mixture was poured into ice water, and extracted with ethyl acetate. The organic layer was washed with water for three times, dried and concentrated. The residue was purified through preparative TLC to obtain methyl 3-((3*S*)-7-chloro-5-(2-fluorophenyl)-2-((2-cyclopropyl-2-hydroxyethyl)amino)-3H-benzo[e][ 1,4]diazepin-3-yl)propanoate (compound 36e, 600 mg, yield 70%).
MS m/z (ESI): 458[M+H]⁺.

### Step 3. Preparation of (S)-methyl 3-(8-chloro-6-(2-fluorophenyl)-1-cyclopropyl-4H-benzo[f]imidazo[1,2-a][1,4]diazepin-4-yl)propanoate (compound 34s)

Methyl 3-((3*S*)-7-chloro-5-(2-fluorophenyl)-2-((2-cyclopropyl-2-hydroxyethyl)amino)-3H-benzo[e][1,4]diazepin-3-yl)propanoate (compound 36e, 600 mg, 1.31 mmol) was dissolved in CH₃CN (20 mL), and PDC (1111 mg, 2.62 mmol) was added. The mixture was heated to 40 °C, and stirred for 1 h until LCMS indicated that the reaction was completed. The reaction mixture was filtered and concentrated, and the residue was purified through preparative TLC to obtain (*S*)-methyl 3-(8-chloro-6-(2-fluorophenyl)-1-cyclopropyl-4H-benzo[f]imidazo[1,2-a][1,4]diazepin-4-yl)propanoate (compound 34s, 100 mg, yield 17%).
MS m/z (ESI): 438 [M+H]⁺.
¹HNMR (400 MHz, CDCl₃) δ: 7.99 (d, *J* = 8.8 Hz, 1H), 7.87 (dd, *J* = 8.8, 6.4 Hz, 1H),7.66-7.54 (m, 2H), 7.38-7.32 (m, 2H), 7.25-7.20 (m,1H), 6.82 (s, 1H), 4.25-4.22 (m, 1H), 3.61 (s, 3H), 2.76-2.53 (m, 4H), 2.12-2.08 (m, 1H), 1.17-1.14 (m, 1H), 1.03-0.90 (m, 3H).

### Example 37. Preparation of (S)-methyl 3-(8-bromo-1-(difluoromethyl)-6-(pyridin-2-yl)-4H-benzo[f]imidazo[1,2-a][1,4]diazepin-4-yl)propanoate (compound 35s)

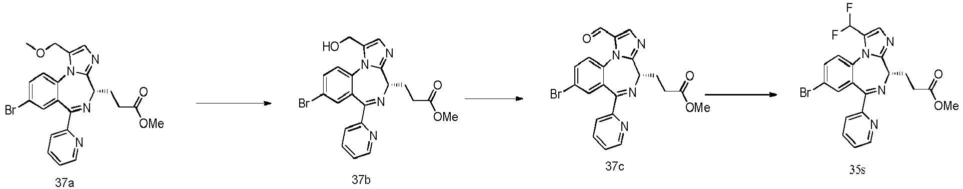

### Step 1: Preparation of (S)-methyl 3-(8-bromo-1-(hydroxymethyl)-6-(pyridin-2-yl)-4H-benzo[f]imidazo[1,2-a][1,4]diazepin-4-yl)propanoate (compound 37b)

(*S*)-Methyl 3-(8-bromo-1-(methoxymethyl)-6-(pyridin-2-yl)-4H-benzo[f]imidazo [1,2-a][1,4]diazepin-4-yl)propanoate (compound 37a, 1.0 g, 2.0 mmol) was dissolved in DCM. The mixture was cooled to -78 °C, and BBr₃ (1.9 mL, 20 mmol) was addeddropwise. The mixture was warmed to 0 °C and stirred for 3 h. Sat. aq. NaHCO₃ solution was added to adjust pH to about 8 in an ice bath, and the mixture was extracted with DCM. The organic layer was washed with water for three times, dried and concentrated. The residue was purified through preparative TLC to obtain (*S*)-methyl 3-(8-bromo-1-(hydroxymethyl)-6-(pyridin-2-yl)-4H-benzo[f]imidazo[1,2-a][1,4]diazepin-4-yl)propanoate (compound 37b, 100 mg, yield 10%).
MS m/z (ESI): 455 [M+H]⁺.

### Step 2: Preparation of (S)-methyl 3-(8-bromo-1-formyl-6-(pyridin-2-yl)-4H-benzo [f]imidazo[1,2-a][1,4]diazepin-4-yl)propanoate (compound 37c)

(*S*)-Methyl 3-(8-bromo-1-(hydroxymethyl)-6-(pyridin-2-yl)-4H-benzo[f]imidazo [1,2-a][1,4]diazepin-4-yl)propanoate (compound 37b, 80 mg, 0.18 mmol) was dissolved in dioxane, and MnO₂(156 mg, 1.8 mmol) was added. The mixture was heated to 80 °C and stirred for 3 h. Water was added, and the mixture was extracted with ethyl acetate. The organic layer was washed with water for three times, dried and concentrated to obtain crude (*S*)-methyl 3-(8-bromo-1-formyl-6-(pyridin-2-yl)-4H-benzo[f]imidazo[1,2-a] [1,4]diazepin-4-yl)propanoate (compound 37c, 78 mg, yield 99%), which was used in the next step directly without further purification.
MS m/z(ESI): 453 [M+H]⁺.

### Step 3: Preparation of (S)-methyl 3-(8-bromo-1-(difluoromethyl)-6-(pyridin-2-yl)-4H-benzo[f]imidazo[1,2-a][1,4]diazepin-4-yl)propanoate (compound 35s)

(*S*)-Methyl 3-(8-bromo-1-formyl-6-(pyridin-2-yl)-4H-benzo[f]imidazo [1,2-a][1,4]diazepin-4-yl)propanoate (compound 37c, 78mg, 0.17 mmol) was dissolved in 1.2-dichloroethane, and DAST (139 mg, 0.85 mmol) was added. The mixture was heated to 65 °C and stirred over night. Sat. aq. NaHCO₃ solution was added to adjust pH to >7, and the mixture was extracted with ethyl acetate. The organic layer was washed with water for threetimes, dried and concentrated. The residue was purified through preparative HPLC to obtain (*S*)-methyl 3-(8-bromo-1-(difluoromethyl)-6-(pyridin-2-yl)-4H-benzo[f]imidazo [1,2-a][1,4]diazepin-4-yl)propanoate (compound 35s, 20 mg, yield 24.4%).
MS m/z (ESI): 475 [M+H]⁺.
¹HNMR (400 MHz, DMSO-*d6*) δ: 8.56-8.55 (m, 1H), 8.08(d, *J* = 8 Hz, 1H), 7.97-7.94 (m, 2H), 7.72 (d, *J* = 8.8 Hz, 1H), 7.66 (d, *J* = 2.0 Hz, 1H), 7.52-7.49 (m, 2H), 7.38-7.24 (m, 1H), 4.19-4.16 (m, 1H), 3.62 (s, 3H), 2.73-2.51 (m, 4H).

All of the compounds listed in the table below were synthesized according to the methods described in the aforementioned examples:

| **Compound** | **Nomenclature** | **Analytical data** |
|---|---|---|
| 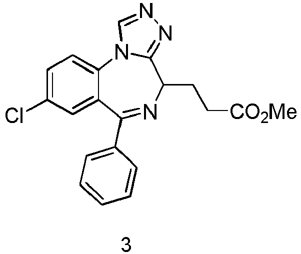 | methyl 3-(8-chloro-6-phenyl-4 H-benzo[f][1,2,4]triazol o[4,3-a][1,4]diazepin-4-yl)propanoate | ¹HNMR (400 MHz, CDCl₃) δ: 8.57-8.54 (s, 1H), 7.67-7.65 (m, 1H), 7.52-7.46 (m, 5H), 7.41-7.37 (m, 2H), 4.27 (m, 1H), 3.68 (s, 3H), 2.92-2.88 (m, 4H). |
| | | MS m/z (ESI): 381 [M+H]+ |
| 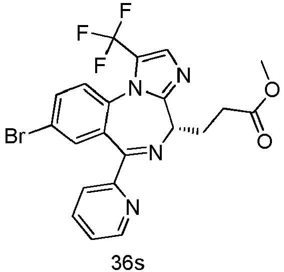 | (*S*)-methyl 3-(8-bromo-6-(pyridin-2-yl)-1-(trifluoromethyl )-4H-benzo[f]imidazo[1 ,2-a][1,4]diazepin-4-yl) propanoate | ¹HNMR (400 MHz, DMSO-d6) δ: 8.61 (d, J = 7.6 Hz, 1H), 7.74-7.59 (m, 4H), 7.58-7.50 (m, 2H), 7.28 (s, 1H), 4.58 (t, J = 9.2 Hz, 1H), 3.64 (s, 3H), 2.76-2.02 (m, 4H) |
| | | MS m/z (ESI): 493 [M+H]+ |
| 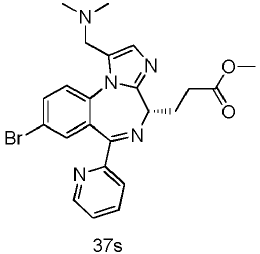 | (*S*)-methyl 3-(8-bromo-1-((N,N-dimethylamino)methyl) -6-(pyridin-2-yl)-4H-be nzo[f]imidazo[1,2-a][1, 4] diazepin-4-yl)propan oate | ¹HNMR (400 MHz, DMSO-d6) δ: 8.59 (d, J = 7.2 Hz, 1H), 7.77-7.45 (m, 6H), 7.00 (s, 1H), 4.69 (t, J = 8.0 Hz, 1H), 4.04 (d, J = 9.6 Hz, 1H), 3.64 (s, 3H), 3.42 (d, J = 9.6 Hz, 1H), 2.78 (s, 3H), 2.72-2.48 (m, 2H), 2.37-2.29 (m, 2H) |
| | | MS m/z (ESI): 482 [M+H]+ |
| 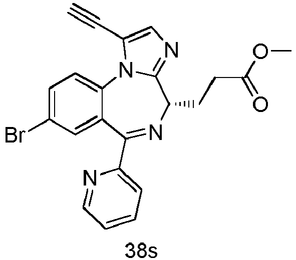 | (*S*)-methyl 3-(8-bromo-1-ethynyl-6 -(pyridin-2-yl)-4H-benz o[f]imidazo[1,2-a][1,4] diazepin-4-yl)propanoat e | ¹HNMR (400 MHz, DMSO-d6) δ: 8.59 (d, J = 7.2 Hz, 1H), 7.77-7.45 (m, 7H), 4.65 (t, J = 8.0 Hz, 1H), 3.64 (s, 3H), 3.30 (s, 1H),2.72-2.48 (m, 2H), 2.37-2.29 (m, 2H) |
| | | MS m/z (ESI): 449[M+H]+ |
| 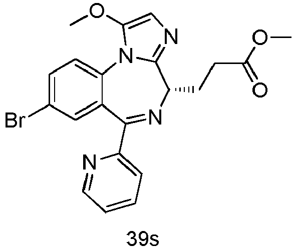 | (*S*)-methyl 3-((8-bromo-1-methoxy -6-(pyridin-2-yl)-4H-be nzo[f]imidazo[1,2-a][1, 4]diazepin-4-yl)propan oate | ¹HNMR (400 MHz, CDCl₃) δ: 8.61-8.58 (m, 1H), 7.71-7.52 (m, 6H), 6.63 (s, 1H), 4.58-4.54 (m, 1H), 4.07 (s, 3H), 3.64 (s, 3H), 2.73-2.66 (m, 1H), 2.45-2.28 (m, 2H), 2.24-2.16 (m, 1H). |
| | | MS m/z (ESI): 455 [M+H]+ |
| 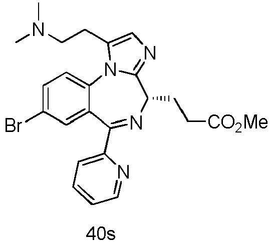 | (*S*)-methyl 3-(8-bromo-1-(2-(N,N-dimethylamino)ethyl)-6 -(pyridin-2-yl)-4H-benz o[f]imidazo[1,2-a][1,4] diazepin-4-yl)propanoat e | ¹HNMR (400 MHz, CDCl₃) δ: 8.61-8.56 (m, 1H), 7.71-7.52 (m, 5H), 7.51-7.47 (m, 1H), 6.99 (s, 1H), 4.61-4.53 (m, 1H), 3.64 (s, 3H), 3.13-3.04 (m, 1H), 3.01-2.94 (m, 1H), 2.85-2.74 (m, 2H), 2.41-2.21 (m, 9H), 2.08-2.03 (m, 1H). |
| | | MS m/z (ESI): 496 [M+H]+ |
| 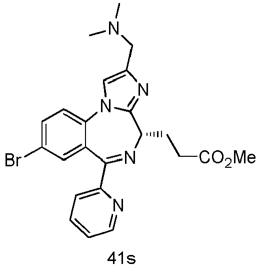 | (*S*)-methyl 3-(8-bromo-2-((N,N-di methylamino)methyl)-6 -(pyridin-2-yl)-4H-benz o[f]imidazo[1,2-a][1,4] diazepin-4-yl)propanoat e | ¹HNMR (400 MHz, CDCl₃) δ: 8.61-8.58 (m, 1H), 7.71-7.61 (m, 3H), 7.59-7.51 (m, 3H), 7.03(m, 1H), 4.82-4.77 (m, 1H), 4.06-4.02 (m, 1H), 3.64 (s, 3H), 3.58-3.64 (m, 1H), 2.93-2.84 (m, 1H), 2.74 (s, 6H), 2.64-2.59 (m, 1H), 2.36-2.30 (m, 2H). |
| | | MS m/z (ESI): 482 [M+H]+ |
| 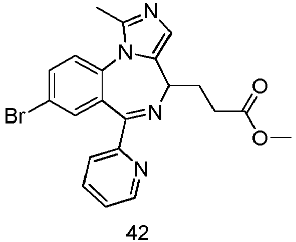 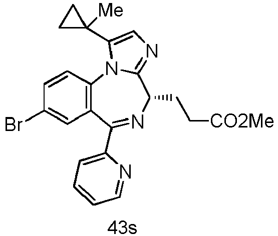 | methyl 3-(8-bromo-1-methyl-6-(pyridin-2-yl)-4H-benz o[f]imidazo[1,5-a][1,4] diazepin-4-yl)propanoat e | ¹HNMR (400 MHz, CDCl₃) δ: 8.61-8.58 (m, 1H), 7.71-7.65 (m, 1H), 7.62-7.52 (m, 5H), 7.10(s, 1H), 4.66-4.61 (m, 1H), 3.64 (s, 3H), 2.87-2.80 (m, 1H), 2.51 (s, 3H), 2.45-2.30 (m, 2H), 2.01-1.94 (m, 1H). |
| | | MS m/z (ESI): 439 [M+H]⁺ |
| | (*S*)-methyl 3-(8-bromo-1-(1-methyl cyclopropyl)-6-(pyridin -2-yl)-4H-benzo[f]imid azo[1,2-a][1,4]diazepin-4-yl)propanoate | ¹HNMR (400 MHz, CDCl₃) δ: 8.62-8.60 (m, 1H), 8.03-8.01 (m, 1H), 7.84-7.80 (m, 1H), 7.75-7.71 (m, 1H), 7.63-7.56 (m, 2H), 7.36-7.34 (m, 1H), 6.80(s, 1H), 4.34-4.30 (m, 1H), 3.64 (s, 3H), 2.62-2.55 (m, 1H), 2.43-2.39 (m, 1H), 2.30-2.26 (m, 1H), 2.11-2.05 (m, 1H), 1.62 (s, 3H), 1.47-1.37 (m, 2H), 0.97-0.88 (m, 2H). |
| | | MS m/z (ESI): 479 [M+H]+ |
| 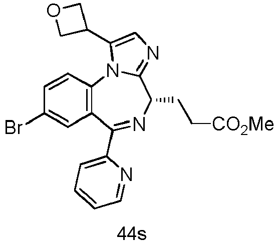 | (*S*)-methyl 3-(8-bromo-1-(oxetan-3 -yl)-6-(pyridin-2-yl)-4H -benzo[f]imidazo[1,2-a] [1,4]diazepin-4-yl)prop anoate | ¹HNMR (400 MHz, CDCl₃) δ: 8.63-8.60 (m, 1H), 8.04-8.01 (m, 1H), 7.84-7.80 (m, 1H), 7.75-7.71 (m, 1H), 7.63-7.56 (m, 2H), 7.36-7.34 (m, 1H), 6.83(s, 1H), 5.22-5.18 (m, 1H), 4.97-4.93 (m, 1H), 4.32 (t, 1H, J = 4 Hz), 4.03-3.97 (m, 1H), 3.64 (s, 3H), 2.43-2.39 (m, 1H), 2.35-2.26 (m, 3H). |
| | | MS m/z (ESI): 481 [M+H]+ |
| 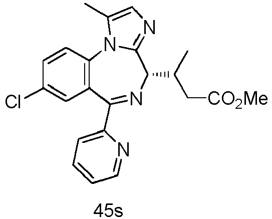 | (*S*)-methyl 3-(8-chloro-1-methyl-6-(pyridin-2-yl)-4H-benz o[f]imidazo[1,2-a][1,4] diazepin-4-yl)butyrate | ¹HNMR (400 MHz, CDCl₃) δ: 8.57-8.53 (m, 1H), 7.69-7.64 (m, 2H), 7.58-7.52 (m, 3H), 7.45-7.41 (m, 1H), 7.00(s, 1H), 4.72 (d, 1H, J = 8 Hz), 3.64 (s, 3H), 2.47-2.39 (m, 4H), 2.26-2.12 (m, 2H), 1.08 (d, 3H, J=8 Hz). |
| | | MS m/z (ESI): 409 [M+H]+ |
| 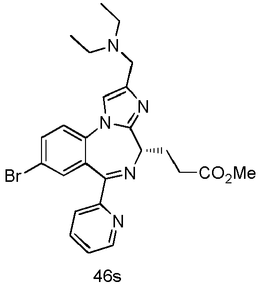 | (*S*)-methyl 3-(8-bromo-2-((N,N-die thylamino)methyl)-6-(p yridin-2-yl)-4H-benzo[f ]imidazo[1,2-a][1,4]dia zepin-4-yl)propionate | ¹HNMR (400 MHz, CDCl₃) δ: 8.61-8.58 (m, 1H), 7.71-7.61 (m, 3H), 7.59-7.51 (m, 3H), 7.03 (m, 1H), 4.82-4.77 (m, 1H), 4.06-4.02 (m, 1H), 3.64 (s, 3H), 3.58-3.64 (m, 1H), 2.93-2.84 (m, 1H), 2.74 (m, 4H), 2.64-2.59 (m, 1H), 2.36-2.30 (m, 2H), 1.03-1.07 (m, 6H). |
| | | MS m/z (ESI): 510 [M+H]+ |
| 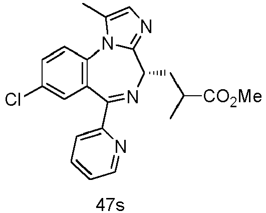 | (*S*)-methyl 3-(8-chloro-1-methyl-6-(pyridin-2-yl)-4H-benz o[f]imidazo[1,2-a][1,4] diazepin-4-yl)-2-methyl propionate | ¹HNMR (400 MHz, DMSO-d6) δ: 8.54-8.53 (m, 1H), 8.11-8.09 (m, 1H), 7.95-7.87 (m, 2H), 7.68-7.64 (m, 2H), 7.49 (m, 1H), 6.68 (s, 1H), 3.88-3.86 (m, 1H), 3.58 (s, 3H), 2.91-2.82 (m, 2 H), 2.75-2.72 (m, 1H), 2.25 (s, 3H), 1.51 (s, 3H). |
| | | MS m/z (ESI): 409[M+H]+ |

### Pharmacological activity tests

In anesthetic activity tests, latency generally refers to the period between the start of administration of a drug to a subject and the moment that the subject becomes unconscious. A short latency is desirable, since it represents fast onset of the drug.

The duration of anesthesia generally refers to the period between the moment that a subject becomes unconscious and the moment that the subject regains consciousness. The durations will be different for different animal models or animal species. An over-long duration of anesthesia may result in adverse suppressive effects on the cardiovascular and respiratory systems, such as side effects on the nervous system, including drowsiness, dizziness, etc.; at the same time, an over-short duration of anesthesia may affect the anesthetic effect, causing dose increase of anesthetic during surgery and other issues.

### Experimental example 1. Drug-induced loss of righting reflex in mice

Kunming mice (male, 18∼25 g) were randomly divided into several groups. After administering the drugs in a single bolus via the tail vein, the latency to loss of righting reflex and the duration of loss of righting reflex were recorded. The test results were shown in Tables 1 and 2.

**Table 1. Effect on the latency to loss of righting reflex in mice**

| Compounds (dose) (60 mg/kg) | Latency (min) |
|---|---|
| Remimazolam (control) | 0.28 |
| Example 2 | 0.13 |
| Example 6 | 0.17 |
| Example 15 | 0.24 |
| Example 18 | 0.19 |
| Example 29 | 0.21 |
| Example 30 | 0.20 |
| Example 31 | 0.16 |

As shown in Table 1, the compounds of the present invention had a shorter latency than remimazolam, suggesting faster onset of action. The compounds of the present invention had similar effects in rats and sheep.

**Table 2. Effect on the duration of loss of righting reflex in mice**

| Compounds (dose) (60 mg/kg) | Duration (min) |
|---|---|
| Example 3 | 11.76 |
| Example 4 | 19.14 |
| Example 5 | 23.33 |
| Example 6 | 23.95 |
| Example 9 | 12.88 |
| Example 10 | 17.27 |
| Example 11 | 12.82 |
| Example 17 | 18.51 |
| Example 18 | 25.61 |
| Example 21 | 23.23 |
| Example 29 | 19.09 |
| Example 30 | 17.74 |
| Example 31 | 17.82 |

As shown in Table 2, the compounds of the present invention exhibited an appropriate duration of LRR in mice, suggesting excellent duration of anesthesia, which is critical to the clinical application of anaesthetics. The compounds of the present invention had similar effects in rats and sheep.

### Experimental example 2. Study of the anesthetic effects in monkeys

Rhesus monkeys (male, 5∼6 kg) were randomly divided into several groups. After administering the drugs intraveneously in a single bolus, the latency to and the duration of loss of consciousness were recorded. The test results were shown in Table 3-5.

**Table 3. Effect on the latency to loss of consciousness in monkeys**

| Compounds (dose) | Latency (min) |
|---|---|
| | |
| Remimazolam (control) (6 mg/kg) | 5.50 |
| Example 2 (6 mg/kg) | 3.92 |
| Example 10 (6 mg/kg) | 4.00 |
| Example 15 (6 mg/kg) | 2.75 |
| Example 18 (6 mg/kg) | 5.09 |
| | |
| Remimazolam (control) (4 mg/kg) | 5.34 |
| Example 15 (4 mg/kg) | 3.75 |
| Example 18 (4 mg/kg) | 5.10 |
| | |
| Example 18 (1 mg/kg) | 3.30 |

As shown in Table 3, the compounds of the present invention had a shorter latency to loss of consciousness in monkey than remimazolam, suggesting better onset of action. In particular, the compound of Example 18 of the present invention was effective even at a much lower dose, suggesting better safety profile and faster onset. In addition, the compounds of the present invention exhibited a short latency even at dose of 8 mg/kg, suggesting that the compounds of the present invention had good safety profile at high doses while having fast onset. The compounds of the present invention had similar effects in rats and sheep.

**Table 4. Effect on the duration of loss of consciousness in monkeys**

| Compounds (dose) | Duration (min) |
|---|---|
| | |
| Example 2 (8 mg/kg) | 55.90 |
| Example 4 (8 mg/kg) | 45.00 |
| Example 9 (8 mg/kg) | 36.47 |
| Example 10 (8 mg/kg) | 26.00 |
| Example 17 (8 mg/kg) | 52.50 |
| Example 2 (6 mg/kg) | 18.10 |
| Example 10 (6 mg/kg) | 26.00 |
| Example 15 (6 mg/kg) | 49.00 |
| | |
| Example 15 (4 mg/kg) | 37.50 |
| | |
| Example 18 (1 mg/kg) | 41.15 |

As shown in Table 4, the compounds of the present invention exhibited an appropriate duration of loss of consciousness in Rhesus monkeys, suggesting excellent duration of anesthesia. The compounds of the present invention had similar effects in rats and sheep.

Experimental example 3. Study of effects of drugs on cellular GABA activation current via whole-cell patch clamp

Test compounds were dissolved in external solution (NaCl 140 mM, KCl 4.7 mM, HEPES 10 mM, CaCl₂ 2 mM, glucose 11 mM, and MgCl₂ 1 mM, pH 7.4) at various concentrations. HEK293T cells were plated on glass cover slips, and incubated in DMEM medium at 37 °C and 5% CO₂ for 24 h.

An HEKA EPC 10 USB patch clamp amplifier was used in the whole-cell recording of GABA Cl⁻ current. 1 µM GABA was used to excite Cl⁻ current. Cells were voltage clamped at a holding potential of -60 mV, and treated with the test compounds of different concentrations together with 1 µM of GABA. The Cl⁻ current induction effect of the test compounds on the same cell, the maximum percentage of current potentiation (Eₘₐₓ), and the concentration of test compounds at half of Eₘₐₓ (EC₅₀) were recorded.

**Table 5-1. Eₘₐₓ of compounds at a concentration of 30 µM**

| Test compounds (concentration: 30 µM) | Eₘₐₓ |
|---|---|
| Example 2 | 358% |
| Example 3 | 325% |
| Example 4 | 472% |
| Example 11 | 319.4% |

**Table 5-2. Eₘₐₓ of compounds at a concentration of 10 µM**

| Test compounds (concentration: 10 µM) | Eₘₐₓ |
|---|---|
| Example 5 | 160% |
| Example 9 | 408% |
| Example 18 | 346.8% |

Health adult has an Eₘₐₓ of 100%. The compounds of the present invention have an Eₘₐₓ higher than 100% at concentrations of 30 µM and 10 µM, even at a lower concentration of 3 µM (for example, the compound of Example 10 of the present invention has an Eₘₐₓ of 196.9%). That is, the compounds of the present invention have Eₘₐₓ higher than 100% at different concentrations, suggesting good anesthetic depth.

**Table 6. EC₅₀ of compounds**

| Compounds | EC₅₀ (µM) |
|---|---|
| Example 3 | 0.95 |
| Example 4 | 0.98 |
| Example 5 | 0.36 |
| Example 9 | 0.72 |
| Example 10 | 0.47 |
| Example 11 | 0.40 |
| Example 18 | 0.47 |

The compounds of the present invention have an EC₅₀ lower than 1 µM, suggesting good anesthetic depth.

As shown in Tables 5-1, 5-2 and 6, the compounds of the present invention possessed appropriate anesthetic depth and good anesthetic effect.

### Experimental example 4. Test of hERG and hNav1.5 via manual patch clamp

Manual whole-cell patch clamp current recording technique was employed to test the inhibitory effect of the compounds of the Examples of the present invention on K⁺ and Na⁺ currents passing through the hERG K⁺ and hNav1.5 Na⁺ channels in half inactivation state (50% inhibitory concentration (IC₅₀)). The test results showed that the compounds of the present invention have IC₅₀ higher than 30 µM on both hERG and hNav1.5, suggesting that the compounds of the present invention had no significant inhibition to hERG and hNav1.5. Therefore, the compounds of the present invention had no hidden danger of prolonging cardic QT intervals.

### Experimental example 5. Safety test in cynomolgus monkeys

Cynomolgus monkeys were randomly divided into several groups. Each group of cynomolgus monkeys were administered with the compounds of the Examples of the present invention at different doses (4 mg/kg, 6 mg/kg, and 8 mg/kg). The general condition, duration of anesthesia, and state of anesthesia were observed. Prior to and after administration, a big-animal non-invasive physiological signal telemetric system (emkaPACK4G) was employed to measure the lead II electrocardiograph; an intelligent non-invasive hemomanometer was employed to measure the arterial pressure (including systolic pressure, diastolic pressure and mean arterial pressure); a TH-212 intelligent digital thermodetector was employed to measure the rectal temperature; and a monitor was employed to monitor the blood oxygen saturation of pulse (SpO2).

The test results showed that the compounds of the present invention have appropriate effects in terms of both latency and duration of anesthesia. No siginificant fluctuation was observed in terms of the parameters including respiratory frequency, body temperature, blood pressure and blood oxygen saturation. No significant change was observed in terms of QTc in most of the periods after administration. This suggested that the compounds of the present invention had no significant side effects to the cardiovascular and respiratory systems of cynomolgus monkeys.

In addition, in both tolerance and toxicity tests, the compounds of the present invention exhibited good safety and tolerance in mice, rats, monkeys and sheep at high single dose.

In addition to those described herein, various modifications to the present invention will be apparent to those skilled in the art. Such modifications are intended to fall within the scope of the appended claims. Each reference cited herein (including all patents, patent applications, journal articles, books and any other publications) are incorporated herein by reference in its entirety.

## Claims

1. A compound of Formula I, 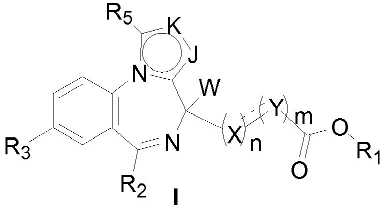 wherein,
R₁ is selected from the group consisting of hydrogen, C₁₋₆ alkyl, C₂₋₆ alkenyl, C₂₋₆ alkynyl, 3-10 membered cycloalkyl, 3-10 membered heterocyclyl, 6-14 membered aryl and 5-14 membered heteroaryl, wherein the C₁₋₆ alkyl, C₂₋₆ alkenyl, C₂₋₆ alkynyl, 3-10 membered cycloalkyl, 3-10 membered heterocyclyl, 6-14 membered aryl and 5-14 membered heteroaryl are each optionally substituted with one or more (e.g., 1, 2, 3 or 4) substituents independently selected from the group consisting of halogen, hydroxy, R₆R₇N-, cyano, nitro, C₁₋₆ alkyl, C₁₋₆ alkoxy, R₈S-, R₈S(O)-, R₈S(O)₂-, 3-10 membered cycloalkyl, 3-10 membered heterocyclyl, 6-14 membered aryl and 5-14 membered heteroaryl, preferably, R₁ is C₁₋₆ alkyl, more preferably, R₁ is methyl or ethyl;
R₂ is selected from the group consisting of 3-10 membered cycloalkyl, 3-10 membered heterocyclyl, 6-14 membered aryl and 5-14 membered heteroaryl, wherein the 3-10 membered cycloalkyl, 3-10 membered heterocyclyl, 6-14 membered aryl and 5-14 membered heteroaryl are each optionally substituted with one or more (e.g., 1, 2, 3 or 4) substituents independently selected from the group consisting of halogen, hydroxy, cyano, nitro, C₁₋₆ alkyl, C₂₋₆ alkenyl, C₂₋₆ alkynyl, R₈C(O)-, R₈S-, R₈S(O)-, R₈S(O)₂-, R₆R₇N-, C₁₋₆ alkoxy, 3-10 membered cycloalkyl, 3-10 membered heterocyclyl, 6-14 membered aryl and 5-14 membered heteroaryl, preferably, R₂ is selected from the group consisting of 6-14 membered aryl and 5-14 membered heteroaryl optionally substituted with one or more (e.g., 1, 2, 3 or 4) halogen, more preferably, R₂ is selected from the group consisting of pyridyl and phenyl optionally substituted with one or more (e.g., 1, 2, 3 or 4) halogen, even more preferably, R₂ is selected from the group consisting of pyridyl and phenyl optionally substituted with one or more (e.g., 1, 2, 3 or 4) fluorines, most preferably, R₂ is selected from the group consisting of 2-pyridyl, 2-fluorophenyl and phenyl;
R₃ is selected from the group consisting of hydrogen, halogen, cyano, nitro, C₁₋₆ alkyl, C₂₋₆ alkenyl and C₂₋₆ alkynyl, wherein the C₁₋₆ alkyl, C₂₋₆ alkenyl, and C₂₋₆ alkynyl are each optionally substituted with one or more (e.g., 1, 2, 3 or 4) substituents independently selected from halogen, preferably, R₃ is halogen, more preferably, R₃ is selected from the group consisting of chlorine and bromine;
K, J are each independently selected from the group consisting of N and CR₄, preferably, K, J are not CR₄ at the same time;
R₄ at each occurance is independently selected from the group consisting of hydrogen, halogen, hydroxy, R₆R₇N-, cyano, carboxy, nitro, C₁₋₆ alkyl, C₁₋₆ alkoxy, C₂₋₆ alkenyl, C₂₋₆ alkynyl, 3-10 membered cycloalkyl, 3-10 membered heterocyclyl, 6-14 membered aryl and 5-14 membered heteroaryl, wherein the C₁₋₆ alkyl, C₁₋₆ alkoxy, C₂₋₆ alkenyl, C₂₋₆ alkynyl, 3-10 membered cycloalkyl, 3-10 membered heterocyclyl, 6-14 membered aryl and 5-14 membered heteroaryl are each optionally substituted with one or more (e.g., 1, 2, 3 or 4) substituents independently selected from the group consisting of halogen, hydroxy, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl, preferably, R₄ at each occurrence is independently selected from the group consisting of hydrogen and C₁₋₆ alkyl optionally substituted with R₆R₇N-, more preferably, R₄ at each occurrence is independently selected from the group consisting of hydrogen, methyl, N,N-dimethylaminomethyl and N,N-diethylaminomethyl;
R₅ is independently selected from the group consisting of hydrogen, halogen, hydroxy, R₆R₇N-, cyano, C₁₋₆ alkyl, C₁₋₆ alkoxy, C₂₋₆ alkenyl, C₂₋₆ alkynyl, 3-10 membered cycloalkyl, 3-10 membered heterocyclyl, 6-14 membered aryl, 5-14 membered heteroaryl, RsC(O)-, R₈S-, R₈S(O)- and R₈S(O)₂-, wherein the C₁₋₆ alkyl, C₁₋₆ alkoxy, C₂₋₆ alkenyl, C₂₋₆ alkynyl, 3-10 membered cycloalkyl, 3-10 membered heterocyclyl, 6-14 membered aryl, 5-14 membered heteroaryl, RsC(O)-, R₈S-, R₈S(O)- and R₈S(O)₂- are each optionally substituted with one or more (e.g., 1, 2, 3 or 4) substituents independently selected from the group consisting of deuterium, halogen, hydroxy, cyano, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl, preferably, R₅ is independently selected from the group consisting of hydrogen, C₁₋₆ alkyl, C₁₋₆ alkoxy, C₂₋₆ alkenyl, C₂₋₆ alkynyl, 3-10 membered cycloalkyl, and 3-10 membered heterocyclyl, wherein the C₁₋₆ alkyl, C₁₋₆ alkoxy, C₂₋₆ alkenyl, C₂₋₆ alkynyl, 3-10 membered cycloalkyl, or 3-10 membered heterocyclyl is each optionally substituted with one or more (e.g., 1, 2, 3 or 4) substituents independently selected from the group consisting of deuterium, halogen, hydroxy, C₁₋₆ alkyl, R₆R₇N-, and C₁₋₆ alkoxy, more preferably, R₅ is independently selected from the group consisting of hydrogen, methyl, ethyl, isopropyl, cyclopropyl, methylcyclopropyl, cyclobutyl, oxetanyl, trifluoromethyl, difluoromethyl, hydroxymethyl, methoxymethyl, N,N-dimethylaminomethyl, N,N-dimethylaminoethyl, N,N-diethylaminomethyl, deuteromethyl, ethenyl, ethynyl and methoxy;
R₆, R₇ at each occurance are each independently selected from the group consisting of hydrogen, C₁₋₆ alkyl, C₂₋₆ alkenyl and C₂₋₆ alkynyl, preferably, R₆, R₇ at each occurance are each independently selected from the group consisting of hydrogen and C₁₋₆ alkyl, more preferably, R₆, R₇ at each occurance are each independently selected from the group consisting of hydrogen, methyl and ethyl;
Rs at each occurance is independently selected from the group consisting of hydroxy, R₆R₇N-, C₁₋₆ alkyl, C₂₋₆ alkenyl, C₂₋₆ alkynyl, 3-10 membered cycloalkyl, 3-10 membered heterocyclyl, 6-14 membered aryl and 5-14 membered heteroaryl;
X, Y are each independently -(R₉R₁₀)C-, and the bond between X and Y is a single bond, a double bond or a triple bond, preferably, the bond between X and Y is a single bond or a double bond;
one or both of R₉ and R₁₀ are absent, or R₉ and R₁₀ at each occurrence are independently selected from the group consisting of hydrogen, halogen, hydroxy, R₆R₇N-, cyano, carboxy, nitro, C₁₋₆ alkoxy, C₁₋₆ alkyl, C₂₋₆ alkenyl, C₂₋₆ alkynyl, 3-10 membered cycloalkyl, 3-10 membered heterocyclyl, 6-14 membered aryl and 5-14 membered heteroaryl, wherein the C₁₋₆ alkoxy, C₁₋₆ alkyl, C₂₋₆ alkenyl, C₂₋₆ alkynyl, 3-10 membered cycloalkyl, 3-10 membered heterocyclyl, 6-14 membered aryl and 5-14 membered heteroaryl are each optionally substituted with one or more (e.g., 1, 2, 3 or 4) substituents independently selected from the group consisting of halogen, hydroxy, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl, preferably, one of R₉ and R₁₀ is absent, or R₉ and R₁₀ at each occurrence are independently selected from the group consisting of hydrogen and C₁₋₆ alkyl, more preferably, one of R₉ and R₁₀ is absent, or R₉ and R₁₀ at each occurrence are independently selected from the group consisting of hydrogen and methyl;
m, n are each independently selected from the group consisting of 0, 1 and 2, and m + n ≥ 1; and
W is selected from the group consisting of hydrogen, halogen, hydroxy, cyano, C₁₋₆ alkoxy, C₁₋₆ alkyl, C₂₋₆ alkenyl and C₂₋₆ alkynyl, wherein the C₁₋₆ alkoxy, C₁₋₆ alkyl, C₂₋₆ alkenyl and C₂₋₆ alkynyl are each optionally substituted with one or more (e.g., 1, 2, 3 or 4) substituents independently selected from the group consisting of halogen, hydroxy, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl group, preferably, W is hydrogen;
wherein the above-mentioned hydrogen and the hydrogen contained in the above-mentioned substituents are independently selected from the group consisting of protium, deuterium and tritium, preferably, selected from the group consisting of protium or deuterium,
or a pharmaceutically acceptable salt, stereoisomer, tautomer, polymorph, solvate, metabolite or prodrug thereof.

2. A compound of claim 1, wherein,
K is N;
J is CR₄; and
R₂ is phenyl or pyridyl, preferably phenyl or 2-pyridyl,
or a pharmaceutically acceptable salt, stereoisomer, tautomer, polymorph, solvate, metabolite or prodrug thereof.

3. A compound of claim 1, wherein,
K is N;
J is N; and
R₂ is phenyl or pyridyl, preferably phenyl or 2-pyridyl,
or a pharmaceutically acceptable salt, stereoisomer, polymorph, solvate, metabolite or prodrug thereof.

4. A compound of claim 1, wherein,
K is N;
J is N;
R₂ is phenyl optionally substituted with one or more (e.g., 1, 2, 3 or 4) halogen, preferably, R₂ is 2-fluorophenyl; and
R₅ is independently selected from the group consisting of hydrogen, 3-10 membered cycloalkyl and C₁₋₆ alkyl, wherein the 3-10 membered cycloalkyl is optionally substituted with one or more (e.g., 1, 2, 3 or 4) substituents independently selected from the group consisting of halogen, hydroxy, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl, and the C₁₋₆ alkyl is substituted with one or more (e.g., 1, 2, 3 or 4) substituents independently selected from the group consisting of halogen, hydroxy, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl, preferably, R₅ is independently selected from the group consisting of hydrogen, 3-10 membered cycloalkyl, and C₁₋₆ alkyl substituted with one or more (e.g., 1, 2, 3 or 4) C₁₋₆ alkoxy, more preferably, R₅ is independently selected from the group consisting of hydrogen, cyclopropyl, and methoxymethyl,
or a pharmaceutically acceptable salt, stereoisomer, tautomer, polymorph, solvate, metabolite or prodrug thereof.

5. A compound of claim 1, wherein,
K is CR₄;
J is N;
R₂ is phenyl optionally substituted with one or more (e.g., 1, 2, 3 or 4) halogen, preferably, R₂ is 2-fluorophenyl; and
R₅ is independently selected from the group consisting of 3-10 membered cycloalkyl and C₁₋₆ alkyl, wherein the 3-10 membered cycloalkyl is optionally substituted with one or more (e.g., 1, 2, 3 or 4) substituents independently selected from the group consisting of halogen, hydroxy, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl, and the C₁-C₆ alkyl is substituted with one or more (e.g., 1, 2, 3 or 4) substituents independently selected from the group consisting of halogen, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl, preferably, R₅ is independently selected from the group consisting of 3-10 membered cycloalkyl, and C₁₋₆ alkyl substituted with one or more (e.g., 1, 2, 3 or 4) C₁₋₆ alkoxy, more preferably, R₅ is independently selected from the group consisting of cyclopropyl and methoxymethyl,
or a pharmaceutically acceptable salt, stereoisomer, tautomer, polymorph, solvate, metabolite or prodrug thereof.

6. A compound of claim 1, wherein,
K is CR₄;
J is N; and
R₂ is phenyl or pyridyl, preferably phenyl or 2-pyridyl,
or a pharmaceutically acceptable salt, stereoisomer, tautomer, polymorph, solvate, metabolite or prodrug thereof;
provided that the compound is not:
methyl (S)-3-(8-chloro-6-(pyridin-2-yl)-4H-benzo[f]imidazo[1,2-a][1,4]diazepin-4-yl)propanoate,
methyl (S)-3-(8-chloro-1-methyl-6-(pyridin-2-yl)-4H-benzo[f]imidazo[1,2-a][1,4]diazepin-4-yl)propanoate,
methyl (S)-3-(8-bromo-1-methyl-6-(pyridin-2-yl)-4H-benzo[f]imidazo[1,2-a][1,4]diazepin-4-yl)propanoate,
methyl (S)-3-(8-bromo-2-methyl-6-(pyridin-2-yl)-4H-benzo[f]imidazo[1,2-a][1,4]diazepin-4-yl)propanoate,
methyl (S)-3-(8-chloro-2-methyl-6-(pyridin-2-yl)-4H-benzo[f]imidazo[1,2-a][1,4]diazepin-4-yl)propanoate,
methyl (S)-3-(8-chloro-2-(hydroxymethyl)-6-(pyridin-2-yl)-4H-benzo[f]imidazo[1,2-a][1,4]diazepin-4-yl)propanoate,
methyl (S)-3-(8-chloro-1-(hydroxymethyl)-6-(pyridin-2-yl)-4H-benzo[f]imidazo[1,2-a][1,4]diazepin-4-yl)propanoate,
methyl (S)-3-(8-chloro-1,2-dimethyl-6-(pyridin-2-yl)-4H-benzo[f]imidazo[1,2-a][1,4]diazepin-4-yl)propanoate, or
methyl (S)-3-(8-chloro-1-methyl-2-(hydroxymethyl)-6-(pyridin-2-yl)-4H-benzo[f]imidazo[1,2-a][1,4]diazepin-4-yl)propanoate.

7. A compound selected from the group consisting of:
methyl 3-(8-bromo-1-methyl-6-(pyridin-2-yl)-4H-benzo[f]imidazo[1,2-a][1,4]diazepin-4-yl)acrylate (compound 1),
methyl 3-(8-bromo-6-(pyridin-2-yl)-4H-benzo[f]imidazo[1,2-a][1,4]diazepin-4-yl)propanoate (compound 2),
methyl 3-(8-chloro-6-phenyl-4H-benzo[f][1,2,4]triazolo[4,3-a][1,4]diazepin-4-yl)propanoate (compound 3),
methyl 3-(8-bromo-1-methyl-6-(pyridin-2-yl)-4H-benzo[f][1,2,4]triazolo[4,3-a][1,4]diazepin-4-yl)propanoate (compound 4),
methyl 3-(8-bromo-1-cyclopropyl-6-(pyridin-2-yl)-4H-benzo[f]imidazo[1,2-a][1,4]diazepin-4-yl)propanoate (compound 5),
methyl 3-(8-bromo-1-deuteromethyl-6-(pyridin-2-yl)-4H-benzo[f]imidazo[1,2-a][1,4]diazepin-4-yl)propanoate (compound 6),
methyl 3-(8-chloro-6-(2-fluorophenyl)-4H-benzo[f][1,2,4]triazolo[4,3-a][1,4]diazepin-4-yl)propanoate (compound 7),
methyl 3-(8-bromo-1-cyclopropyl-6-(pyridin-2-yl)-4H-benzo[f][1,2,4]triazolo[4,3-a][1,4]diazepin-4-yl)propanoate (compound 8),
methyl 3-(8-bromo-6-(pyridin-2-yl)-4H-benzo[f][1,2,4]triazolo[4,3-a][1,4]diazepin-4-yl)propanoate (compound 9),
methyl 3-(8-bromo-1-(methoxymethyl)-6-(pyridin-2-yl)-4H-benzo[f]imidazo[1,2-a][1,4]diazepin-4-yl)propanoate (compound 10),
methyl 3-(8-bromo-1-deuteromethyl-6-(pyridin-2-yl)-4H-benzo[f][1,2,4]triazolo[4,3-a][1,4]diazepin-4-yl)propanoate (compound 11),
methyl 3-(8-chloro-1-cyclopropyl-6-(2-fluorophenyl)-4H-benzo[f][1,2,4]triazolo[4,3-a][1,4]diazepin-4-yl)propanoate (compound 12),
methyl 3-(8-bromo-1-ethenyl-6-(pyridin-2-yl)-4H-benzo[f]imidazo[1,2-a][1,4]diazepin-4-yl)propanoate (compound 13),
methyl 3-(8-chloro-1-cyclopropyl-6-(pyridin-2-yl)-4H-benzo[f][1,2,4]triazolo[4,3-a][1,4]diazepin-4-yl)propanoate (compound 14),
methyl 3-(8-bromo-1-ethyl-6-(pyridin-2-yl)-4H-benzo[f]imidazo[1,2-a][1,4]diazepin-4-yl)propanoate (compound 15),
methyl 3-(8-bromo-1-(methoxymethyl)-6-(pyridin-2-yl)-4H-benzo[f][1,2,4]triazolo[4,3-a][1,4]diazepin-4-yl)propanoate (compound 16),
methyl 3-(8-bromo-1-isopropyl-6-(pyridin-2-yl)-4H-benzo[f]imidazo[1,2-a][1,4]diazepin-4-yl)propanoate (compound 17),
methyl 3-(8-chloro-6-(pyridin-2-yl)-4H-benzo[f][1,2,4]triazolo[4,3-a][1,4]diazepin-4-yl)propanoate (compound 18),
methyl 3-(8-chloro-1-cyclopropyl-6-(pyridin-2-yl)-4H-benzo[f]imidazo[1,2-a][1,4]diazepin-4-yl)propanoate (compound 19),
methyl 3-(8-bromo-1-cyclobutyl-6-(pyridin-2-yl)-4H-benzo[f]imidazo[1,2-a][1,4]diazepin-4-yl)propanoate (compound 20),
methyl 3-(8-bromo-1-methyl-6-(pyridin-2-yl)-4H-benzo[f]imidazo[1,2-a][1,4]diazepin-4-yl)butyrate (compound 21),
methyl 3-(8-bromo-1-methyl-6-(pyridin-2-yl)-4H-benzo[f]imidazo[1,2-a][1,4]diazepin-4-yl)-2-methylpropanoate (compound 22),
methyl 3-(8-bromo-1-cyclopropyl-2-methyl-6-(pyridin-2-yl)-4H-benzo[f]imidazo[1,2-a][1,4]diazepin-4-yl)propanoate (compound 23),
methyl 3-(8-bromo-1-(hydroxymethyl)-6-(pyridin-2-yl)-4H-benzo[f]imidazo[1,2-a][1,4]diazepin-4-yl)propanoate (compound 24),
methyl 3-(8-chloro-6-(2-fluorophenyl)-1-(methoxymethyl)-4H-benzo[f][1,2,4]triazolo[4,3a][1,4]diazepin-4-yl)propanoate (compound 25),
methyl 3-(8-chloro-6-(2-fluorophenyl)-1-(methoxymethyl)-4H-benzo[f]imidazo[1,2-a][1,4]diazepin-4-yl)propanoate (compound 26),
methyl 3-(8-chloro-1-cyclobutyl-6-(pyridin-2-yl)-4H-benzo[f]imidazo[1,2-a][1,4]diazepin-4-yl)propanoate (compound 27),
methyl 3-(8-chloro-1-ethyl-6-(pyridin-2-yl)-4H-benzo[f][1,2,4]triazolo[4,3-a][1,4]diazepin-4-yl)propanoate (compound 28),
methyl 3-(8-chloro-1-isopropyl-6-(pyridin-2-yl)-4H-benzo[f][1,2,4]triazolo[4,3-a][1,4]diazepin-4-yl)propanoate (compound 29),
ethyl 3-(8-chloro-1-methyl-6-(pyridin-2-yl)-4H-benzo[f]imidazo[1,2-a][1,4]diazepin-4-yl)propanoate (compound 30),
methyl 3-(8-chloro-1-cyclopropyl-2-methyl-6-(pyridin-2-yl)-4H-benzo[f]imidazo[1,2-a][1,4]diazepin-4-yl)propanoate (compound 31),
methyl 3-(8-chloro-1-(methoxymethyl)-6-(pyridin-2-yl)-4H-benzo[f][1,2,4]triazolo[4,3-a][1,4]diazepin-4-yl)propanoate (compound 32),
methyl 3-(8-chloro-1-(methoxymethyl)-6-(pyridin-2-yl)-4H-benzo[f]imidazo[1,2-a][1,4]diazepin-4-yl)propanoate (compound 33),
methyl 3-(8-chloro-6-(2-fluorophenyl)-1-cyclopropyl-4H-benzo[f]imidazo[1,2-a][1,4]diazepin-4-yl)propanoate (compound 34),
methyl 3-(8-bromo-1-(difluoromethyl)-6-(pyridin-2-yl)-4H-benzo[f]imidazo[1,2-a][1,4]diazepin-4-yl)propanoate (compound 35),
methyl 3-(8-bromo-6-(pyridin-2-yl)-1-(trifluoromethyl)-4H-benzo[f]imidazo[1,2-a][1,4]diazepin-4-yl)propanoate (compound 36),
methyl 3-(8-bromo-1-((N,N-dimethylamino)methyl)-6-(pyridin-2-yl)-4H-benzo[f]imidazo[1,2-a][1,4]diazepin-4-yl)propanoate (compound 37),
methyl 3-(8-bromo-1-ethynyl-6-(pyridin-2-yl)-4H-benzo[f]imidazo[1,2-a][1,4]diazepin-4-yl)propanoate (compound 38),
methyl 3-((8-bromo-1-methoxy-6-(pyridin-2-yl)-4H-benzo[f]imidazo[1,2-a][1,4]diazepin-4-yl)propanoate (compound 39),
methyl 3-(8-bromo-1-(2-(N,N-dimethylamino)ethyl)-6-(pyridin-2-yl)-4H-benzo[f]imidazo[1,2-a][1,4]diazepin-4-yl)propanoate (compound 40),
methyl 3-(8-bromo-2-((N,N-dimethylamino)methyl)-6-(pyridin-2-yl)-4H-benzo[f]imidazo[1,2-a][1,4]diazepin-4-yl)propanoate (compound 41),
methyl 3-(8-bromo-1-methyl-6-(pyridin-2-yl)-4H-benzo[f]imidazo[1,5-a][1,4]diazepin-4-yl)propanoate (compound 42),
methyl 3-(8-bromo-1-(1-methylcyclopropyl)-6-(pyridin-2-yl)-4H-benzo[f]imidazo[1,2-a][1,4]diazepin-4-yl)propanoate (compound 43),
methyl 3-(8-bromo-1-(oxetan-3-yl)-6-(pyridin-2-yl)-4H-benzo[f]imidazo[1,2-a][1,4]diazepin-4-yl)propanoate (compound 44),
methyl 3-(8-chloro-1-methyl-6-(pyridin-2-yl)-4H-benzo[f]imidazo[1,2-a][1,4]diazepin-4-yl)butyrate (compound 45),
methyl 3-(8-bromo-2-((N,N-diethylamino)methyl)-6-(pyridin-2-yl)-4H-benzo[f]imidazo[1,2-a][1,4]diazepin-4-yl)propionate (compound 46), and
methyl 3-(8-chloro-1-methyl-6-(pyridin-2-yl)-4H-benzo[f]imidazo[1,2-a][1,4]diazepin-4-yl)-2-methylpropionate (compound 47),
or a pharmaceutically acceptable salt, stereoisomer, tautomer, polymorph, solvate, metabolite or prodrug thereof;
preferably, the compound is selected from the group consisting of: 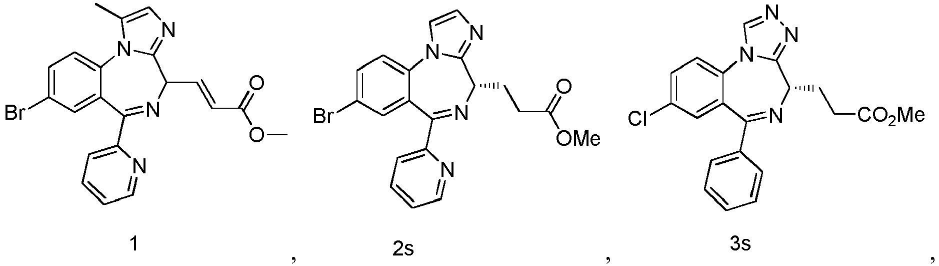 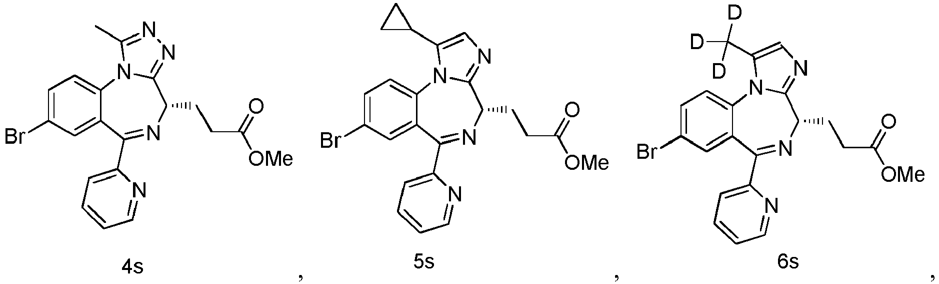 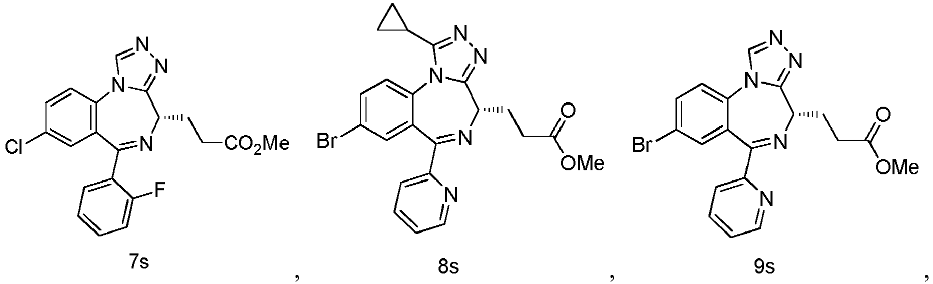 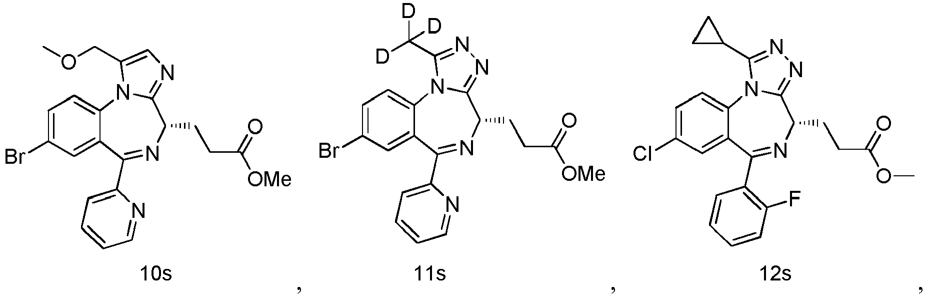 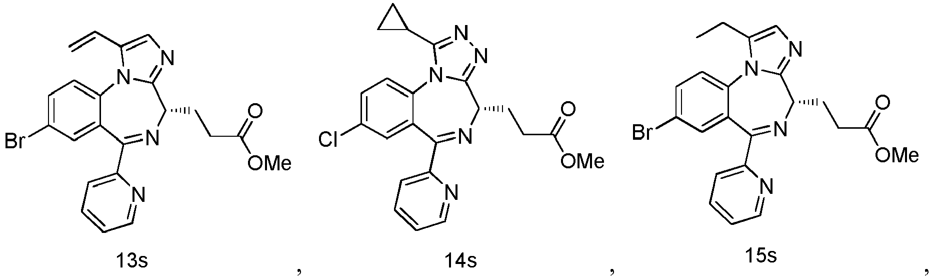 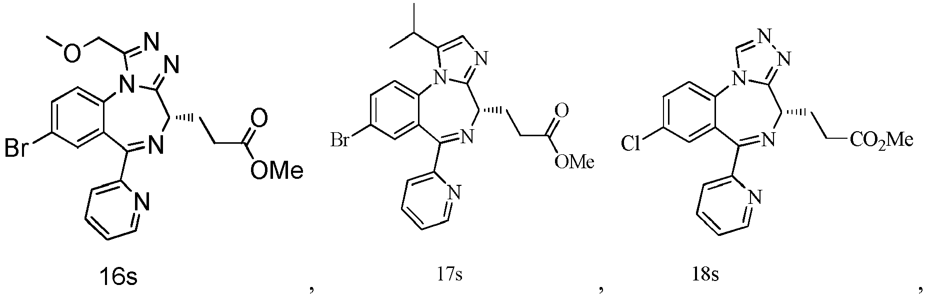 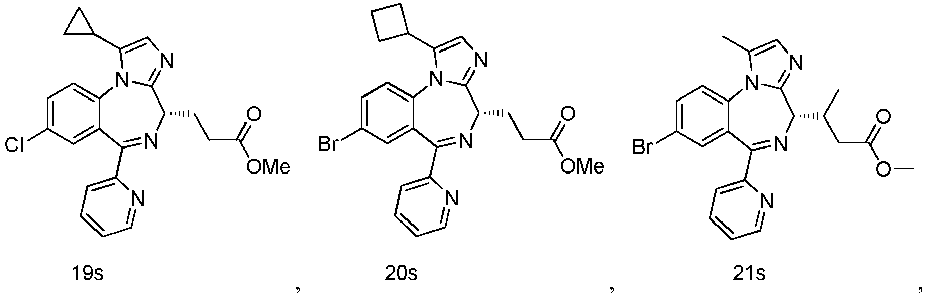 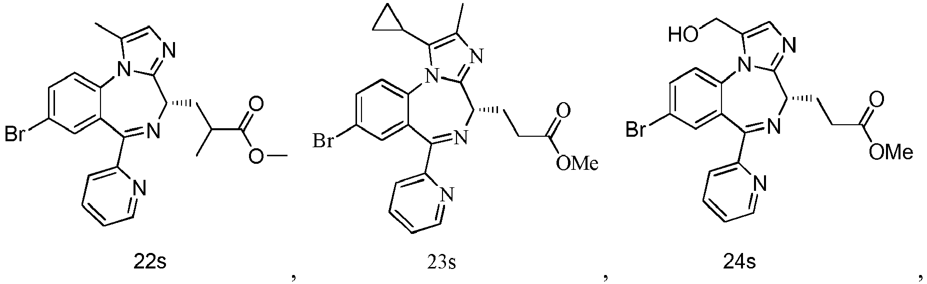 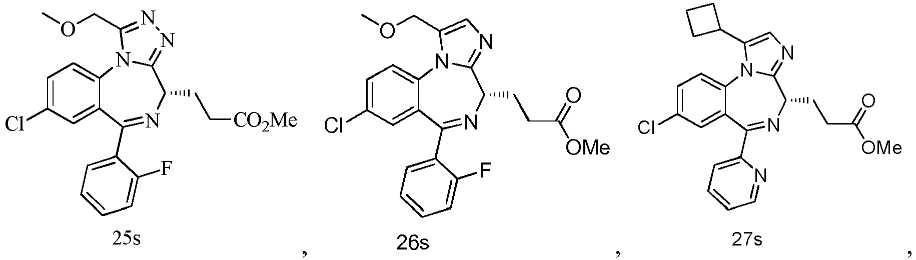 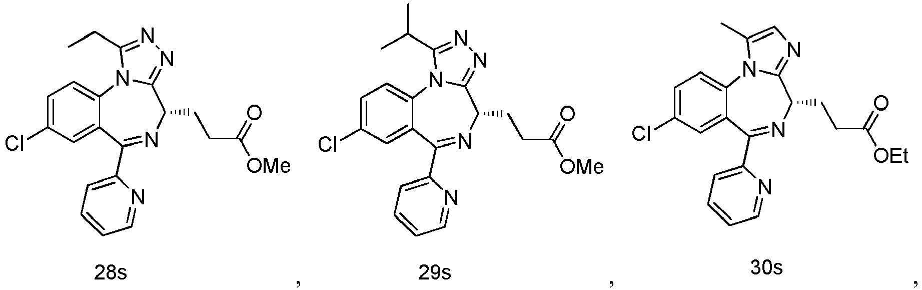 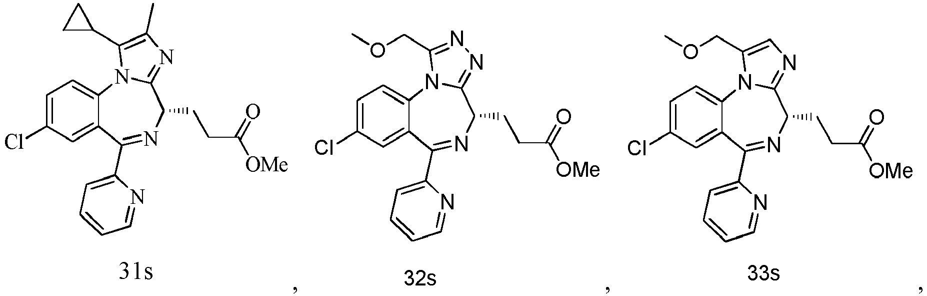 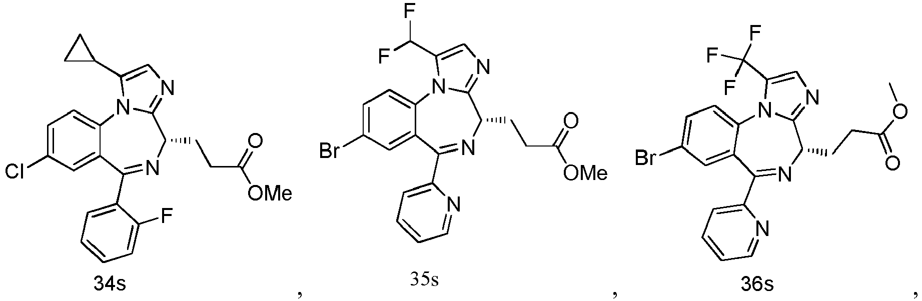 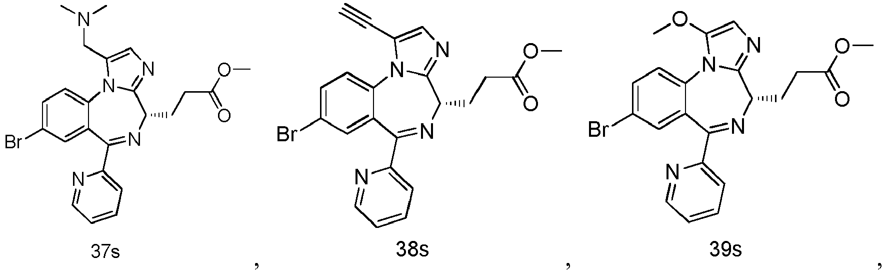 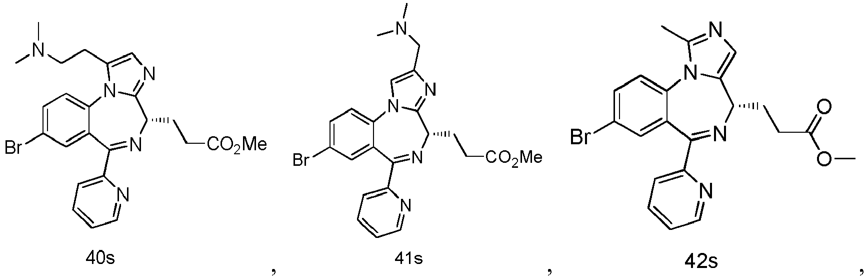 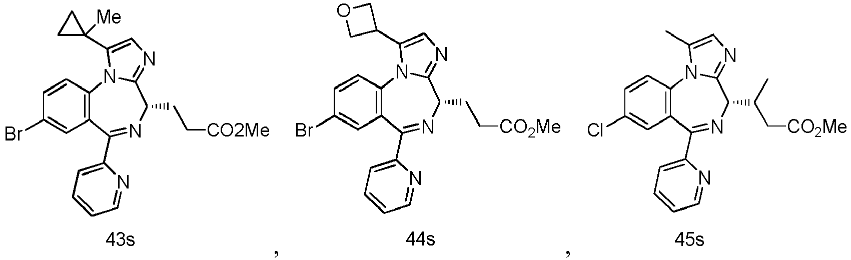 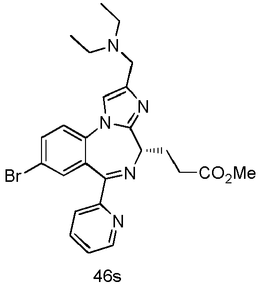 and

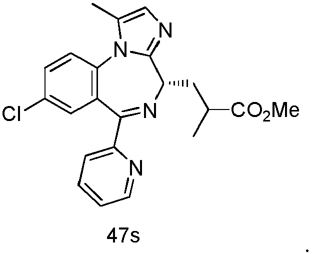

8. A pharmaceutical composition comprising an effective amount of the compound of any one of claims 1-7 and one or more pharmaceutically acceptable carriers, preferably, the effective amount is about 0.01 mg to about 1000 mg, appropriately 0.1-500 mg, preferably 0.5-300 mg, more preferably 1-150 mg, particularly preferably 1-50 mg, e.g., 2 mg, 4 mg, 6 mg, 8 mg, 10 mg, or 25 mg.

9. A kit comprising the compound of any one of claims 1-7 or the pharmaceutical compositions of claim 8.

10. A method for preparing the compound of any one of claims 1-7, wherein
when J is N, the method is carried out according to Scheme I, 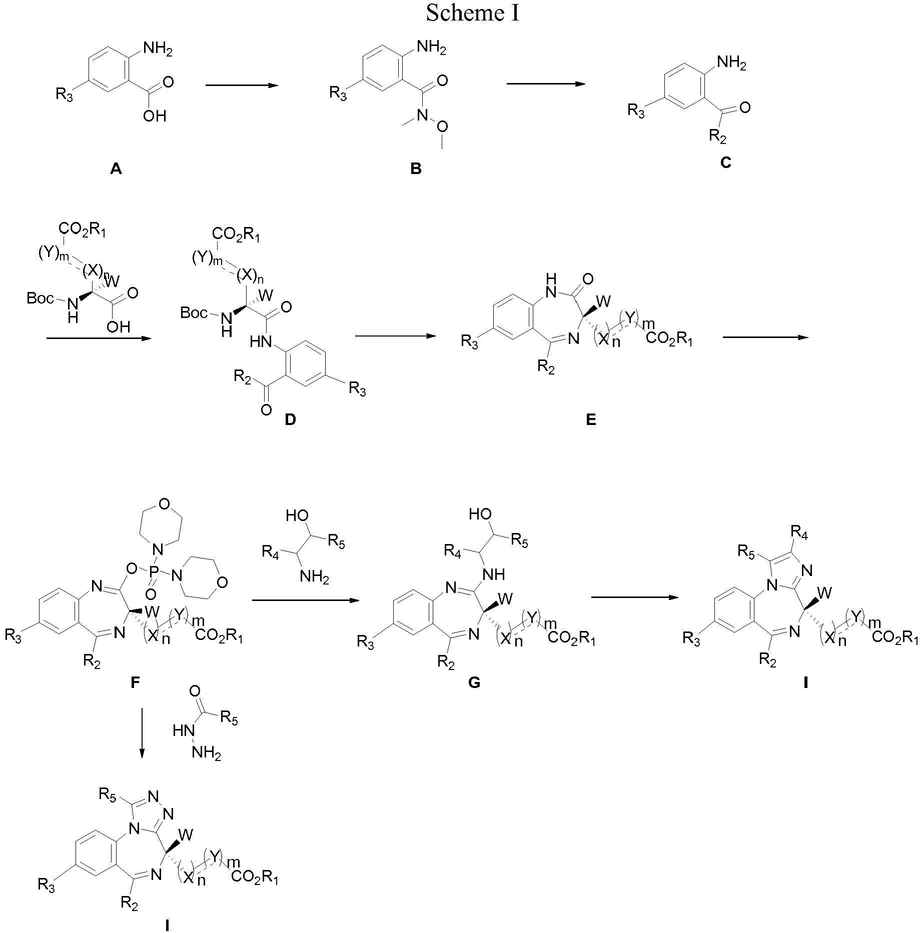 wherein R₁-R₅, n, m, W are as defined in any one of claims 1 and 3-6; and the bond between X and Y is a single bond or a double bond;
the method comprising the following steps:
a. allowing compound A to react with N,O-dimethylhydroxylamine hydrochloride, preferably in the presence of a base such as DIPEA, N-methylmorpholine or TEA and in the presence of a condensing agent such as HATU, DCC, EDCI, PyBOP, BOP-Cl or T3P, preferably in a solvent such as THF, DMF or DCM, to obtain amide intermediate B;
b. allowing the amide intermediate B to react with a lithium reagent such as *n*-butyllithium and a bromo-substituted aryl or heteroaryl compound such as bromobenzene, 1-bromo-2-fluorobenzene or 2-bromopyridine, preferably in a suitable solvent such as THF or diethyl ether, to obtain ketone intermediate C;
c. carrying out a condensation reaction between the ketone intermediate C and an N-protected chiral amino acid or a derivative thereof such as 5-methyl L-glutamate or 5-ethyl L-glutamate, preferably in a ice-water bath or at room temperature, preferably in the presence of a base such as DIPEA, N-methylmorpholine or TEA and in the presence of a condensing agent such as HATU, DCC, EDCI, PyBOP, BOP-Cl or T3P, preferably in a suitable solvent such as THF, DMF or DCM, to obtain intermediate D;
d. deprotecting the intermediate D, preferably under acidic conditions of e.g. trifluoroacetic acid or hydrochloric acid, to obtain benzodiazepine intermediate E;
f. allowing the benzodiazepine intermediate E to react with dimorpholino phosphinic chloride, preferably in the presence of a strong base such as sodium hydride or *t*-BuOK, preferably in a suitable solvent such as THF or diethyl ether, to obtain reactive intermediate F;
g. carrying out a substitution reaction between the reactive intermediate F and an amino alcohol such as 2-amino-1-cyclopropylethanol, 1-amino-3-methoxypropan-2-ol, 2-amino-1-cyclopropylpropan-1-ol, 2-amino-2-cyclopropylethanol or 1-amino-2,3,3,3-tetradeuteropropan-2-ol, to obtain intermediate G; and.
h. oxidizing the intermediate G with an oxidizing agent such as PCC, PDC, TEMPO or Dess-Martin periodinane or through Swern oxidation, preferably in a suitable solvent such as CH₃CN, DMF or DCM, to achieve ring closure and obtain the target product I; or
h'. carrying out a substitution reaction between the reactive intermediate F and a hydrazide such as cyclopropane carbohydrazide, 2-methylpropionyl hydrazide, propionyl hydrazide, 2-methoxyacethydrazide or acethydrazide-D₃, followed by cyclization via condensation, to obtain the target product I;
or
when K is CR₄ and J is N, the method is carried out according to Scheme II: 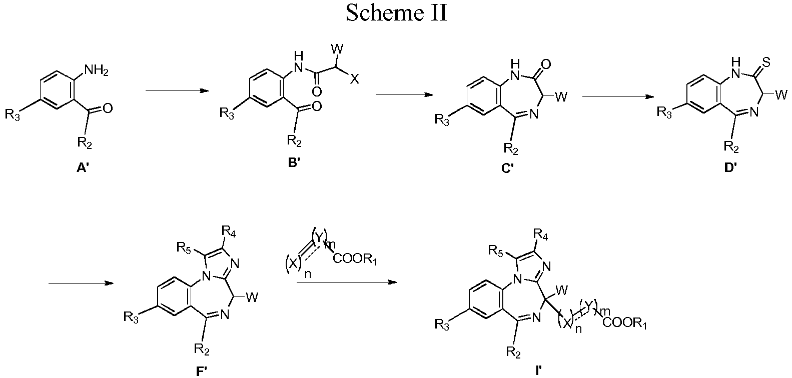 wherein R₁-R₄, W are as defined in any one of claims 1, 5 and 6; the bond between X and Y is a single bond or a double bond; m and n are each 1; and R₅ is C₁₋₆ alkyl optionally substituted with one or more (e.g., 1, 2, 3 or 4) substituents independently selected from the group consisting of deuterium, halogen, hydroxy, cyano, C₁₋₆ alkyl, R₆R₇N-, C₁₋₆ alkoxy, 3-10 membered cycloalkyl, 3-10 membered heterocyclyl, 6-14 membered aryl and 5-14 membered heteroaryl, preferably, R₅ is selected from the group consisting of methyl, ethyl, isopropyl, trifluoromethyl, difluoromethyl, hydroxymethyl, methoxymethyl, N,N-dimethylaminomethyl, N,N-dimethylaminoethyl, N,N-diethylaminomethyl, and deuteromethyl;
the method comprising the following steps:
a. allowing intermediate A' to react with a halogen-substituted acetyl halide such as chloroacetyl chloride or bromoacetyl bromide to obtain intermediate B';
b. cyclizing the intermediate B', preferably in an alcohol (e.g. methanol, ethanol or isopropanol) or ether (e.g. tetrahydrofuran or dioxane) solution of excess ammonia, preferably under heating conditions such as at a temperature of 30 °C to 80 °C, to obtain lactam intermediate C';
c. carrying out a sulfur-oxygen exchange reaction on the lactam intermediate C', preferably under heating conditions such as at a temperature of 80 °C to 150 °C, in the presence of a thiating agent such as phosphorus pentasulfide or Lawesson's reagent, to obtain thiolactam intermediate D';
d. carrying out a substitution reaction between the thiolactam intermediate D' and an amino-containing alkyne such as prop-2-yn-1-amine, but-2-yn-1-amine, 4-methoxybut-2-yn-1-amine, N,N-dimethylpent-2-yn-1,5-diamine or 3-cyclopropylprop-2-yn-1-amine, and then carrying out a cyclization reaction, preferably in the presence of a catalyst such as Hg(OAc)₂ or HgSO₄, to obtain intermediate F'; and
e. carrying out a Michael addition reaction between the intermediate F' and an enoate ester such as methyl acrylate, ethyl methylbut-2-enoate or methyl methacrylate or an alkynoate ester such as methyl propiolate or methyl methylbut-2-ynoate, preferably in the presence of a base such as sodium hydride, LDA or t-BuOK, preferably in a suitable solvent such as THF or 2-methyltetrahydrofuran, to obtain racemic target molecule I', and further carrying out a chiral separation to obtain enantiomerically pure target molecule I';
or
when K is N and J is CR₄, the method is carried out according to Scheme III: 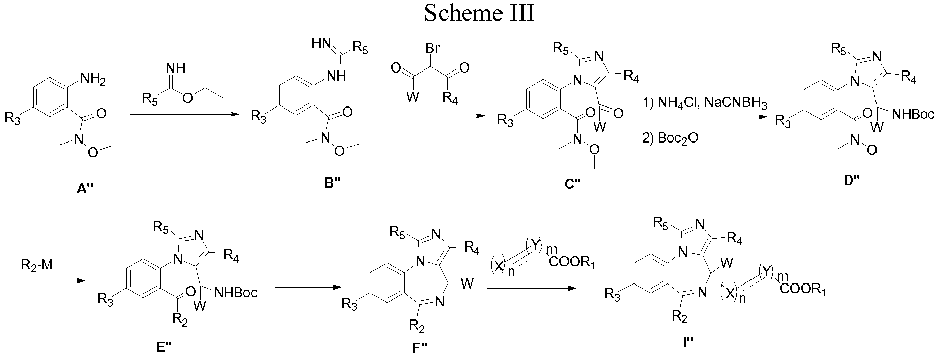 wherein R₁-R₅, W are as defined in claim 1 or 2; the bond between X and Y is a single bond or a double bond; and m and n are each 1;
the method comprising the following steps:
a. allowing intermediate A" to react with substituted ethyl imidate such as ethyl acetimidate, propionimidic acid ethyl ester, 2-methoxy-acetimidic acid ethyl ester or cyclopropanecarboximidic acid ethyl ester to obtain intermediate B";
b. carrying out a condensation reaction between the intermediate B" and 2-bromomalonaldehyde, preferably under heating conditions such as at a temperature of 50 °C to 150 °C, to obtain intermediate C";
c. carrying out a two-step reaction to convert the intermediate C" to intermediate D", wherein the first step converts the aldehyde group in the intermediate C" to aminomethyl using reductive amination, and the second step protects the amino group in the aminomethyl with Boc₂O to obtain a *tert*-butyl carbamate;
d. allowing the amide intermediate D" to react with a lithium reagent such as *n*-butyllithium and a bromo-substituted aryl or heteroaryl compound such as bromobenzene, 1-bromo-2-fluorobenzene or 2-bromopyridine, preferably in a suitable solvent such as THF or diethyl ether, to obtain ketone intermediate E";
e. deprotecting the intermediate E", preferably under acidic conditions of e.g. trifluoroacetic acid or hydrochloric acid, followed by cyclization under the same conditions, to obtain intermediate F"; and
f. carrying out a Michael addition reaction between the intermediate F" and an enoate ester such as methyl acrylate, ethyl methylbut-2-enoate or methyl methacrylate or an alkynoate ester such as methyl propiolate or methyl methylbut-2-ynoate, preferably in the presence of a base such as sodium hydride, LDA or t-BuOK, preferably in a suitable solvent such as THF or 2-methyltetrahydrofuran, to obtain racemic target molecule I", and further carrying out a chiral separation to obtain enantiomerically pure target molecule I".

11. Use of the compound of any one of claims 1-7 or the pharmaceutical composition of claim 8 in the manufacture of an anesthetic medicament, preferably an anesthetic medicament for intravenous administration, wherein the anesthetic medicament is preferably used in the following clinical settings: presurgical sedation, antianxiety and anti-amnesia during surgery; conscious sedation during short-term diagnostic, surgical or endoscopic procedures; induction and maintenance of general anesthesia; and ICU sedation.

12. The compound of any one of claims 1-7 or the pharmaceutical composition of claim 8 for use as an anesthetic medicament.

13. A method of anesthesia, comprising administering, preferably intravenously, an effective amount of the compound of any one of claims 1-7 or the pharmaceutical composition of claim 8.
